# EUROPEAN PATENT APPLICATION

(11) **EP 4 674 950 A1**
(43) Date of publication of application: **07.01.2026**
(21) Application number: 23929301.2
(22) Date of filing: 30.03.2023
(51) Int. Cl.: C12N 1/21, C12N 15/70, C12P 7/42, C12N 9/04, C12R 1/19

(54) **RECOMBINANT MICROORGANISM FOR PRODUCING PANTOIC ACID AND USE THEREOF**

(71) Applicant: Anhui Huaheng Biotechnology Co., Ltd., Hefei, Anhui 231131 (CN); Tianjin Institute of Industrial Biotechnology, Chinese Academy of Sciences, Tianjin 300308 (CN)
(72) Inventor: ZHANG, Xueli, Tianjin 300308 (CN); GUO, Henghua, Hefei, Anhui 231131 (CN); LIU, Pingping, Tianjin 300308 (CN); ZHANG, Dongzhu, Hefei, Anhui 231131 (CN); TANG, Jinlei, Tianjin 300308 (CN); ZHANG, Chao, Hefei, Anhui 231131 (CN)
(74) Representative: Bayramoglu et al.
(86) International application number: PCT/CN2023/085099
(87) International publication number: WO 2024/197704

(57) **Abstract**

The present invention provides a genetically engineered pantoic acid-producing strain having or having an enhanced NADH-dependent acetohydroxy acid reductoisomerase, a method for producing the strain, a method for producing D-pantoic acid using the strain, and use thereof in production of D-pantoic acid.

## Description

### TECHNICAL FIELD

The present invention relates to the technical field of biology, and in particular to a pantoic acid-producing recombinant microorganism, a method for constructing the same, and use of the recombinant microorganism in fermentative preparation of D-pantoic acid.

### BACKGROUND

Calcium pantothenate, also known as vitamin B5, is one of the 13 essential vitamins for the human body. It can only be synthesized in microorganisms and plants, and serves as a crucial precursor for synthesis of coenzyme A as well as an essential vitamin for maintaining the normal physiological functions of organisms. Meanwhile, calcium pantothenate also has extensive applications as a food additive, a pharmaceutical bulk drug, and the like. Currently, calcium pantothenate is mainly obtained by resolving DL-pantolactone through biological or chemical methods to obtain high-purity D-pantolactone, and then reacting the high-purity D-pantolactone with β-alanine. Due to the severe environmental damage caused by the process of preparing pantolactone via petrochemical technology, such as the use of highly toxic, strong acid, or strong alkali reagents, and the production of large amounts of waste water/gas during production, as well as the increased costs incurred by the chiral resolution of DL-pantolactone, enterprises engaged in calcium pantothenate synthesis are frequently subjected to environmental inspections, and the price of calcium pantothenate also varies from tens of thousands to hundreds of thousands of yuan per ton.

The rapid development of synthetic biology and metabolic engineering has made it possible to replace non-renewable petrochemical resources with renewable raw materials, substitute petrochemical processes with green and environmentally friendly fermentation processes, and realize the production of chemicals at low costs instead of high costs through the design and construction of microbial cell factories, and this approach has already achieved great success in the production of many products.

The pantoic acid obtained through microbial fermentation is inherently high-purity D-pantoic acid, which requires no subsequent resolution and can be directly used to react with β-alanine to generate calcium pantothenate. This will significantly reduce the production cost of calcium pantothenate and alleviate environmental pressure. Nevertheless, the synthesis of D-pantoic acid remains a limiting factor for the industrial-scale biological synthesis of calcium pantothenate at present. *Escherichia coli* inherently possesses a pantoic acid synthesis pathway; however, due to the complex metabolic network regulation within the cell itself, wild-type cells can hardly accumulate pantoic acid at a detectable concentration.

Therefore, providing microbial cells that possess genetic stability, require no addition of inducers or antibiotics, and are capable of producing high-purity pantoic acid via microbial fermentation using renewable resources as raw materials will be of great significance for advancing the production of calcium pantothenate, reducing production costs, and alleviating environmental pressure.

### SUMMARY

In one aspect, the present invention provides a genetically engineered pantoic acid-producing strain, having or having an enhanced NADH-dependent acetohydroxy acid reductoisomerase.

In one embodiment, the present invention provides a genetically engineered pantoic acid-producing strain,
having or having enhanced activities of: an acetolactate synthase, a dihydroxy acid dehydratase, a 3-methyl-2-oxobutanoate hydroxymethyltransferase, a 2-dehydropantoate-2-reductase, a serine hydroxymethyltransferase, a glycine cleavage enzyme system (e.g., an aminomethyltransferase and/or a glycine decarboxylase), a phosphoglycerate dehydrogenase, a phosphoserine/phosphohydroxythreonine aminotransferase, a phosphoserine phosphatase, and an NADH-dependent acetohydroxy acid reductoisomerase; and
optionally, having reduced or inactivated activities of: an L-serine deaminase I, a propionate kinase, a formate acetyltransferase, an alcohol dehydrogenase, a pyruvate formate lyase, a fumarate reductase, a lactate dehydrogenase, a methylglyoxal synthase, an acetate kinase, a ribokinase, a valine-pyruvate transaminase, a phosphotransacetylase, and/or a branched-chain amino acid aminotransferase; preferably where the branched-chain amino acid aminotransferase is attenuated.

In one aspect, the present invention provides a method for producing a pantoic acid-producing strain, including conferring or enhancing activity of an NADH-dependent acetohydroxy acid reductoisomerase in a pantoic acid-producing strain.

In one embodiment, the present invention provides a method for producing a genetically engineered pantoic acid-producing strain, including, in a strain, conferring or enhancing activities of: an acetolactate synthase, a dihydroxy acid dehydratase, a 3-methyl-2-oxobutanoate hydroxymethyltransferase, a 2-dehydropantoate-2-reductase, a serine hydroxymethyltransferase, a glycine cleavage enzyme system (e.g., an aminomethyltransferase and/or a glycine decarboxylase), a phosphoglycerate dehydrogenase, a phosphoserine/phosphohydroxythreonine aminotransferase, a phosphoserine phosphatase, and an NADH-dependent acetohydroxy acid reductoisomerase; and
optionally, reducing or inactivating activities of: an L-serine deaminase I, a propionate kinase, a formate acetyltransferase, an alcohol dehydrogenase, a pyruvate formate lyase, a fumarate reductase, a lactate dehydrogenase, a methylglyoxal synthase, an acetate kinase, a ribokinase, a valine-pyruvate transaminase, a phosphotransacetylase, and/or a branched-chain amino acid aminotransferase; and preferably attenuating activity of a branched-chain amino acid aminotransferase.

In one aspect, the present invention provides a method for producing a genetically engineered pantoic acid-producing strain, including: in *Escherichia coli* deposited in the China General Microbiological Culture Collection Center (CGMCC) in Beijing, China, with a deposit number CGMCC No. 21699, replacing an NADPH-dependent acetohydroxy acid reductoisomerase-encoding gene with a gene encoding an NADH-dependent acetohydroxy acid reductoisomerase.

In one aspect, the present invention provides a method for producing D-pantoic acid, including culturing the genetically engineered strain producing pantoic acid of the present invention or a genetically engineered strain producing pantoic acid prepared by the method for producing a genetically engineered strain producing pantoic acid of the present invention under conditions suitable for fermentative production of D-pantoic acid, and optionally including isolating and purifying produced D-pantoic acid.

In one aspect, the present invention provides use of the genetically engineered strain producing pantoic acid of the present invention or a genetically engineered strain producing pantoic acid prepared by the method for producing a genetically engineered strain producing pantoic acid of the present invention in production of D-pantoic acid.

In one aspect, the present invention provides a method for improving D-pantoic acid production of a pantoic acid-producing strain, including: conferring or enhancing activity of an NADH-dependent acetohydroxy acid reductoisomerase in the pantoic acid-producing strain.

### DETAILED DESCRIPTION

Unless otherwise defined, the technical and scientific terms used herein have the meanings commonly understood by those skilled in the art. See, for example, Singleton et al., DICTIONARY OF MICROBIOLOGY AND MOLECULAR BIOLOGY 2nd ed., J. Wiley & Sons (New York, NY 1994); and Sambrook et al., MOLECULAR CLONING, A LABORATORY MANUAL, Cold Springs Harbor Press (Cold Springs Harbor, NY 1989).

As used herein, "genetically engineered" refers to a strain that has been artificially altered by biological means, which has one or more changes compared with the initial strain before modification, for example, gene deletion, amplification, or mutation, thereby having altered biological properties, for example, improved production performance. As used herein, the initial strain may be a natural strain to be subjected to the genetic modification or a strain with other genetic modifications.

As used herein, the terms "strain producing pantoic acid" or "pantoic acid-producing strain" refer to a strain capable of producing D-pantoic acid accumulation reaching a detectable level.

As used herein, the terms "polypeptide," "amino acid sequence," "peptide," and "protein" are used interchangeably herein and refer to a chain of amino acids of any length, which may include engineered amino acids and/or may be interrupted by non-amino acids. This term also encompasses amino acid chains engineered by natural or artificial intervention, for example, disulfide bond formation, glycosylation, lipidation, acetylation, phosphorylation, or any other manipulation or modification, such as conjugation with a labeling component.

As used herein, the expressions "gene," "nucleic acid sequence," "polynucleotide," and "nucleotide sequence" are used interchangeably and refer to a chain of nucleotides, including DNA and RNA. "Expression of a gene" refers to the transcription of a DNA region operably linked to appropriate regulatory regions, particularly a promoter, into biologically active RNA, as well as the ability of the RNA to be translated into a biologically active protein or peptide.

As used herein, a "degenerate sequence" refers to a nucleotide sequence that encodes the same amino acid sequence as a specified sequence but differs in its nucleotide sequence due to the degeneracy of the genetic codon.

As used herein, the terms "homology," "sequence identity," and the like are used interchangeably herein. Sequence identity can be detected by comparing the number of identical nucleotide bases between a polynucleotide and a reference polynucleotide; for example, it can be determined by standard sequence alignment algorithm programs using default gap penalties established by each provider. Whether two nucleic acid molecules have nucleotide sequences that are at least 80%, 85%, 90%, 95%, 96%, 97%, 98%, or 99% "identical" can be determined using known computer algorithms, such as BLASTN, FASTA, DNAStar, and Gap (University of Wisconsin Genetics Computer Group (UWG), Madison, WI, USA). For example, the percentage identity of nucleic acid molecules can be determined, for instance, by comparing sequence information using the GAP computer program (e.g., Needleman et al., J. Mol. Biol. 48: 443 (1970), revised by Smith and Waterman (Adv. Appl. Math. 2: 482 (1981))). In short, the GAP program defines similarity according to the number of symbols (i.e., nucleotides) that are aligned similarly in sequences divided by the total number of symbols in the shorter sequence of the two sequences.

As used herein, acetolactate synthase (EC 2.2.1.6) is responsible for catalyzing the conversion of two molecules of pyruvate to one molecule of acetolactate There are multiple acetolactate synthases from different sources in nature. For example, *Escherichia coli* contains three acetolactate synthases, namely acetolactate synthase I (encoded by the ilvBN gene), acetolactate synthase II (encoded by the ilvGM gene), and acetolactate synthase III (encoded by the ilvIH gene). In contrast, microorganisms such as *Corynebacterium glutamicum* contain only one acetolactate synthase, which is encoded by the ilvBN gene, while in *Bacillus subtilis,* it is encoded by the alsS gene. As used herein, "having or having enhanced acetolactate synthase activity" refers to a strain having or having enhanced acetolactate synthase activity that converts pyruvate to acetolactate. "L-valine feedback-resistant acetolactate synthase III" herein refers to an enzyme whose activity is not inhibited by L-valine, or whose degree of inhibition is reduced compared with that of the wild-type acetolactate synthase III.

As used herein, acetohydroxy acid reductoisomerase (EC 1.1.1.382, EC 1.1.1.383, EC 1.1.1.86) is a key enzyme responsible for catalyzing the conversion of acetolactate to 2,3-dihydroxyisovalerate. Most acetohydroxy acid reductoisomerases commonly used are derived from microorganisms, with common examples including *Escherichia coli, Corynebacterium glutamicum, Bacillus subtilis,* and the like. As used herein, "having or having enhanced acetohydroxy acid reductoisomerase activity" refers to a strain having or having enhanced acetohydroxy acid reductoisomerase activity that converts acetolactate to 2,3-dihydroxyisovalerate.

As used herein, dihydroxy acid dehydratase (EC 4.2.1.9) is a key enzyme responsible for catalyzing the conversion of 2,3-dihydroxyisovalerate to 2-ketoisovalerate. Most dihydroxy acid dehydratases commonly used are derived from microorganisms, with common examples including *Escherichia coli, Corynebacterium glutamicum, Bacillus subtilis,* and the like. As used herein, "having or having enhanced dihydroxy acid dehydratase activity" refers to a strain having or having enhanced dihydroxy acid dehydratase activity that converts 2,3-dihydroxyisovalerate to 2-ketoisovalerate.

As used herein, 3-methyl-2-oxobutanoate hydroxymethyltransferase (EC 2.1.2.11) is responsible for converting 3-methyl-2-oxobutanoate to 2-dehydropantoate in the presence of 5,10-methylenetetrahydrofolate. Most microorganisms and plants contain 3-methyl-2-oxobutanoate hydroxymethyltransferase, and the commonly used genes encoding the 3-methyl-2-oxobutanoate hydroxymethyltransferase are mostly derived from microorganisms such as *Escherichia coli* and *Corynebacterium glutamicum.* As used herein, "having or having enhanced 3-methyl-2-oxobutanoate hydroxymethyltransferase activity" refers to a strain having or having enhanced 3-methyl-2-oxobutanoate hydroxymethyltransferase activity that converts 3-methyl-2-oxobutanoate to 2-dehydropantoate.

As used herein, 2-dehydropantoate 2-reductase (EC 1.1.1.169) is responsible for catalyzing the conversion of 2-dehydropantoate to pantoic acid. Most microorganisms and plants contain 2-dehydropantoate 2-reductase. As used herein, "having or having enhanced 2-dehydropantoate 2-reductase activity" refers to a strain having or having enhanced activity to convert 2-dehydropantoate to pantoic acid.

As used herein, serine hydroxymethyltransferase (EC 2.1.2.1), encoded by the glyA gene, is responsible for catalyzing the conversion of L-serine to glycine while converting tetrahydrofolate to 5,10-methylenetetrahydrofolate. This enzyme is widely present in plants, animals, and microorganisms. The commonly used genes encoding serine hydroxymethyltransferase are mostly derived from microorganisms such as *Escherichia coli, Corynebacterium glutamicum,* yeast, and the like. As used herein, "having or having enhanced serine hydroxymethyltransferase activity" refers to a strain having or having enhanced serine hydroxymethyltransferase activity that converts L-serine to glycine while generating 5,10-methylenetetrahydrofolate.

As used herein, aminomethyltransferase (EC 2.1.2.10) is encoded by the gcvT gene, glycine cleavage system H protein (EC 1.4.1.27) by the gcvH gene, and glycine decarboxylase (EC 1.4.1.27) by the gcvP gene. In *Escherichia coli,* these three enzymes, together with the lipoamide dehydrogenase subunit encoded by the lpdA gene, form the glycine cleavage multienzyme system, which is responsible for catalyzing the cleavage of glycine to produce carbon dioxide, while generating NADH and 5,10-methylenetetrahydrofolate. As used herein, "having or having enhanced glycine cleavage enzyme system activity" refers to a strain having or having enhanced enzyme activity that cleaves glycine to produce carbon dioxide and generate NADH and 5,10-methylenetetrahydrofolate.

As used herein, phosphoglycerate dehydrogenase (EC 1.1.1.95) is encoded by the serA gene and catalyzes the conversion of 3-phosphoglycerate to 3-phosphohydroxypyruvate. "Having or having enhanced phosphoglycerate dehydrogenase activity" herein refers to a strain having or having enhanced enzyme activity that catalyzes the conversion of 3-phosphoglycerate to 3-phosphohydroxypyruvate.

As used herein, phosphoserine phosphatase (EC 3.1.3.3) is encoded by the serB gene and catalyzes the conversion of phospho-L-serine to L-serine. "Having or having enhanced phosphoserine phosphatase activity" herein refers to a strain having or having enhanced enzyme activity that catalyzes the conversion of phospho-L-serine to L-serine.

As used herein, phosphoserine/phosphohydroxythreonine aminotransferase (EC 2.6.1.52) is encoded by the serC gene and catalyzes the conversion of 3-phosphohydroxypyruvate to phospho-L-serine. "Having or having enhanced phosphoserine/phosphohydroxythreonine aminotransferase activity" herein refers to a strain having or having enhanced enzyme activity that catalyzes the conversion of 3-phosphohydroxypyruvate to phospho-L-serine.

As used herein, L-serine deaminase I (EC 4.3.1.17) is encoded by the sdaA gene, and this enzyme catalyzes the deamination of L-serine to generate pyruvate. "L-serine deaminase I with reduced or inactivated activity" herein refers to the reduction or loss of activity of the enzyme in catalyzing the deamination of L-serine to generate pyruvate.

As used herein, acetate kinase (EC 2.7.2.1) is encoded by the ackA gene, and this enzyme catalyzes the conversion of acetyl phosphate to acetate while generating ATP. "Acetate kinase with reduced or inactivated activity" herein refers to the reduction or loss of activity of the enzyme in catalyzing the conversion of acetyl phosphate to acetate.

As used herein, propionate kinase (EC 2.7.2.15) is encoded by the tdcD gene, and this enzyme catalyzes the reversible conversion between propionate and propionyl phosphate. "Propionate kinase with reduced or inactivated activity" herein refers to the reduction or loss of activity of the enzyme in catalyzing the reversible conversion between propionate and propionyl phosphate.

As used herein, formate acetyltransferase (EC 2.3.1.54) is encoded by the tdcE gene, and this enzyme catalyzes the conversion of pyruvate to formate. "Formate acetyltransferase with reduced or inactivated activity" herein refers to the reduction or loss of activity of the enzyme in catalyzing the conversion of pyruvate to formate.

As used herein, phosphotransacetylase (EC 2.3.1.8) is encoded by the pta gene and catalyzes the conversion of acetyl coenzyme A to acetyl phosphate. "Phosphotransacetylase with reduced or inactivated activity" herein refers to the reduction or loss of activity of the enzyme in catalyzing the conversion of acetyl coenzyme A to acetyl phosphate.

As used herein, alcohol dehydrogenase (EC 1.1.1.1) is encoded by the adhE gene and catalyzes the conversion of acetaldehyde to ethanol. "Alcohol dehydrogenase with reduced or inactivated activity" herein refers to the reduction or loss of activity of the enzyme in catalyzing the conversion of acetaldehyde to ethanol.

As used herein, pyruvate formate lyase (EC 2.3.1.54) is encoded by the pflB gene and catalyzes the cleavage of pyruvate to generate formate and acetyl coenzyme A. "Pyruvate formate lyase with reduced or inactivated activity" herein refers to the reduction or loss of activity of the enzyme in catalyzing the cleavage of pyruvate to generate formate and acetyl coenzyme A.

As used herein, fumarate reductase (EC 1.3.5.1) is encoded by frdABCD and catalyzes the conversion of fumarate to succinate. "Fumarate reductase with reduced or inactivated activity" herein refers to the reduction or loss of activity of the enzyme in catalyzing the conversion of fumarate to succinate.

As used herein, lactate dehydrogenase (EC 1.1.1.28) is encoded by ldhA and catalyzes the conversion of pyruvate to D-lactate. "Lactate dehydrogenase with reduced or inactivated activity" herein refers to the reduction or loss of activity of the enzyme in catalyzing the conversion of pyruvate to D-lactate.

As used herein, methylglyoxal synthase (EC 4.2.3.3) is encoded by the mgsA gene and catalyzes the conversion of dihydroxyacetone phosphate to methylglyoxal. "Methylglyoxal synthase with reduced or inactivated activity" herein refers to the reduction or loss of activity of the enzyme in catalyzing the conversion of dihydroxyacetone phosphate to methylglyoxal.

As used herein, ribokinase (EC 2.7.1.16) is encoded by the ara gene and catalyzes the conversion of L-ribulose to ribulose 5-phosphate. "Ribokinase with reduced or inactivated activity" herein refers to the reduction or loss of activity of the enzyme in catalyzing the conversion of L-ribulose to ribulose 5-phosphate.

As used herein, valine-pyruvate transaminase (EC 2.6.1.66) is encoded by the avtA gene and catalyzes the conversion of L-alanine and 3-methyl-2-oxobutanoate to pyruvate and L-valine. "Valine-pyruvate transaminase with reduced or inactivated activity" herein refers to the reduction or loss of activity of the enzyme in catalyzing the conversion of L-alanine and 3-methyl-2-oxobutanoate to pyruvate and L-valine.

As used herein, branched-chain amino acid transaminase (EC 2.6.1.42) is encoded by the ilvE gene and catalyzes the conversion of corresponding keto acids to three branched-chain amino acids, including L-valine, L-leucine, and L-isoleucine. "Branched-chain amino acid transaminase with reduced or inactivated activity" herein refers to the reduction or loss of activity of the enzyme in catalyzing the conversion of the corresponding keto acids to the three branched-chain amino acids.

As used herein, "having... activity" refers to having detectable activity compared with a reference without such activity (e.g., the initial strain or wild-type strain).

As used herein, "having enhanced... activity" refers to an increase in activity of at least 5%, at least 10%, at least 20%, at least 30%, at least 40%, at least 50%, at least 60%, at least 70%, at least 80%, at least 90%, at least 100%, at least 150%, at least 200%, at least 250%, at least 300% or more, compared with a reference having such activity (e.g., the initial strain or wild-type strain).

As used herein, "with reduced or inactivated activity" refers to a reduction in activity of at least 5%, at least 10%, at least 20%, at least 30%, at least 40%, at least 50%, at least 60%, at least 70%, at least 80%, at least 90%, at least 95%, at least 99% or more, compared with the activity of a reference (e.g., corresponding activity in the initial strain or wild-type strain).

As used herein, "inactivated" refers to having no detectable activity compared with the activity of a reference (e.g., corresponding activity in the initial strain or wild-type strain).

In this article, the reference may be a wild-type microorganism or a microorganism before the desired genetic manipulation (e.g., an initial microorganism used for genetic manipulation to increase gene activity). A "parental microorganism" and an "initial microorganism" are used interchangeably herein, referring to the microorganism on which the desired genetic manipulation (e.g., enhancing or attenuating gene or protein activity) is performed.

The activity of a protein (e.g., an enzyme) can be generated or enhanced by any suitable means known in the art, including but not limited to expressing or overexpressing the corresponding gene encoding the protein in the strain (e.g., via a vector such as a plasmid), introducing mutations that result in increased activity of the protein, and the like.

The activity of a protein (e.g., an enzyme) can be reduced or inactivated by any suitable means known in the art, including but not limited to using an attenuated or inactivated corresponding gene encoding the protein, introducing mutations that result in reduced or inactivated activity of the protein, using antagonists or inhibitors of the protein (e.g., antibodies, ligands, etc.).

As used herein, an "attenuated or inactivated gene" refers to a gene whose activity, e.g., expression level (when acting as a protein-coding gene) or regulatory performance (when acting as a regulatory element), is reduced by at least 5%, at least 10%, at least 20%, at least 30%, at least 40%, at least 50%, at least 60%, at least 70%, at least 80%, at least 90%, at least 95%, at least 99% or more, or even undetectable, compared with a reference (e.g., a corresponding gene in the initial strain or wild-type strain). In the cases where a gene encodes a protein such as an enzyme, "attenuated or inactivated gene" also encompasses that the activity level of the protein expressed by the gene is reduced compared with the activity level of the corresponding protein in the initial strain or wild-type strain, for example, reduced by at least 5%, at least 10%, at least 20%, at least 30%, at least 40%, at least 50%, at least 60%, at least 70%, at least 80%, at least 90%, at least 95%, at least 99% or more, or even 100%.

As used herein, "conferring... activity" refers to generating, in a genetically engineered strain producing 2-hydroxyisovalerate, an activity that is absent in the initial strain prior to genetic modification.

As used herein, "enhancing... activity" refers to increasing the activity by, for example, at least 5%, at least 10%, at least 20%, at least 30%, at least 40%, at least 50%, at least 60%, at least 70%, at least 80%, at least 90%, at least 95%, at least 100%, at least 150%, at least 200%, at least 250%, at least 300% or more.

Various methods known in the art can be used to confer or enhance the activity of a desired protein, including but not limited to expression or overexpression of protein-coding genes, and mutations or other modifications that increase protein activity.

As used herein, "overexpression" refers to an increase in the expression level of a gene relative to the level before genetic manipulation, for example, an increase of at least 5%, at least 10%, at least 20%, at least 30%, at least 40%, at least 50%, at least 60%, at least 70%, at least 80%, at least 90%, at least 100%, at least 150%, at least 200%, at least 250%, at least 300% or more. Methods for overexpression of genes are well known in the art, including but not limited to using strong promoters, increasing gene copy numbers, enhancers, and the like. Increasing gene copy number can be achieved, for example, but not limited to, by introducing one or more copies of an exogenous gene or an endogenous gene, e.g., via an expression vector or integration into the genome.

As used herein, an "exogenous gene" refers to a gene derived from another cell or organism, for example, a gene from the same species or a different species.

As used herein, an "endogenous gene" refers to a gene that is native to the cell or organism itself.

As used herein, "reducing or inactivating the activity of a protein such as an enzyme" refers to reducing the activity of the protein by at least 5%, at least 10%, at least 20%, at least 30%, at least 40%, at least 50%, at least 60%, at least 70%, at least 80%, at least 90%, at least 95%, at least 99% or more, or even rendering it undetectable. Various means for reduction or inactivation are known in the art, including, for example, inhibiting gene expression such as knockdown (e.g., using small interfering RNA), using weak promoters (when the gene is a polypeptide-encoding gene), and the like; gene knockout, deletion of part or all of the gene or polypeptide sequence; mutating certain sites in the gene or polypeptide such as coding sequences or active domains to reduce gene expression, regulatory activity, or the activity of expression products; and using antagonists or inhibitors (including but not limited to antibodies, interfering RNA, etc., for example).

As used herein, "attenuating or inactivating a gene" refers to reducing the expression level of the gene (when acting as a protein-encoding gene) or its regulatory performance (when acting as a regulatory element) by at least 5%, at least 10%, at least 20%, at least 30%, at least 40%, at least 50%, at least 60%, at least 70%, at least 80%, at least 90%, at least 95%, at least 99% or more, or even rendering it undetectable. Various methods for attenuating or inactivating genes are known in the art, including, for example, inhibiting gene expression such as knockdown (e.g., using small interfering RNA), using weak promoters (when the gene is a polypeptide-encoding gene), and the like; gene knockout, deletion of part or all of the gene sequence; and mutating certain sites in the gene, such as coding sequences to reduce gene expression, regulatory activity, or the activity of expression products, etc.

In one aspect, the present invention provides a genetically engineered pantoic acid-producing strain, having or having enhanced NADH-dependent acetohydroxy acid reductoisomerase activity.

In one embodiment, the NADH-dependent acetohydroxy acid reductoisomerase is derived from microorganisms such as *Escherichia coli, Corynebacterium glutamicum, Bacillus subtilis,* and the like. In a preferred embodiment, the NADH-dependent acetohydroxy acid reductoisomerase is derived from *Thermacetogenium phaeum,* and more preferably includes an amino acid sequence shown in SEQ ID NO: 9 or an amino acid sequence having at least 75%, 80%, 85%, 90%, 91%, 92%, 93%, 94%, 95%, 96%, 97%, 98%, 99% or higher identity thereto and having NADH-dependent acetohydroxy acid reductoisomerase activity.

In one embodiment, the genetically engineered pantoic acid-producing strain expresses or overexpresses a gene encoding the NADH-dependent acetohydroxy acid reductoisomerase. In one embodiment, the gene encoding the NADH-dependent acetohydroxy acid reductoisomerase encodes an NADH-dependent acetohydroxy acid reductoisomerase shown in SEQ ID NO: 9, for example, includes a nucleotide sequence shown in SEQ ID NO: 10 or a degenerate sequence thereof, or a nucleotide sequence having at least 75%, 80%, 85%, 90%, 91%, 92%, 93%, 94%, 95%, 96%, 97%, 98%, 99% or higher identity thereto, and is preferably under the control of a strong promoter, for example, an RBS5 artificial regulatory element (SEQ ID NO: 170).

Herein, in addition to the NADH-dependent acetohydroxy acid reductoisomerase, the genetically engineered pantoic acid-producing strain may further include other genetic modifications required for producing D-pantoic acid, for example, engineered other enzymes or regulatory molecules involved in a pantoic acid production pathway.

In one embodiment, the genetically engineered pantoic acid-producing strain further has or has enhanced activities of: an acetolactate synthase, a dihydroxy acid dehydratase, a 3-methyl-2-oxobutanoate hydroxymethyltransferase, a 2-dehydropantoate-2-reductase, a serine hydroxymethyltransferase, a glycine cleavage enzyme system (e.g., an aminomethyltransferase and/or a glycine decarboxylase), a phosphoglycerate dehydrogenase, a phosphoserine/phosphohydroxythreonine aminotransferase, and a phosphoserine phosphatase.

In one embodiment, the acetolactate synthase includes an acetolactate synthase derived from *Bacillus subtilis.* In particular, the acetolactate synthase derived from *Bacillus subtilis* includes an amino acid sequence shown in SEQ ID NO: 1 or an amino acid sequence having at least 75%, 80%, 85%, 90%, 91%, 92%, 93%, 94%, 95%, 96%, 97%, 98%, 99% or higher identity thereto and having acetolactate synthase activity.

In one embodiment, the acetolactate synthase includes an acetolactate synthase I, an acetolactate synthase II, and/or an L-valine feedback-resistant acetolactate synthase III derived from *Escherichia coli.* In one embodiment, the acetolactate synthase I includes an amino acid sequence shown in SEQ ID NO: 3 or an amino acid sequence having at least 75%, 80%, 85%, 90%, 91%, 92%, 93%, 94%, 95%, 96%, 97%, 98%, 99% or higher identity thereto and having acetolactate synthase I activity. In one embodiment, the acetolactate synthase II includes an amino acid sequence shown in SEQ ID NO: 5 or an amino acid sequence having at least 75%, 80%, 85%, 90%, 91%, 92%, 93%, 94%, 95%, 96%, 97%, 98%, 99% or higher identity thereto and having acetolactate synthase II activity. In one embodiment, the L-valine feedback-resistant acetolactate synthase III includes an amino acid sequence shown in SEQ ID NO: 7 or an amino acid sequence having at least 75%, 80%, 85%, 90%, 91%, 92%, 93%, 94%, 95%, 96%, 97%, 98%, 99% or higher identity thereto and having L-valine feedback-resistant acetolactate synthase III activity.

In one embodiment, the acetolactate synthase includes an acetolactate synthase derived from *Bacillus subtilis,* and an acetolactate synthase I, an acetolactate synthase II, and an L-valine feedback-resistant acetolactate synthase III derived from *Escherichia coli.*

In one embodiment, the dihydroxy acid dehydratase, the 2-dehydropantoate-2-reductase, the glycine cleavage enzyme system (e.g., the aminomethyltransferase and/or the glycine decarboxylase), the phosphoserine/phosphohydroxythreonine aminotransferase, and the phosphoserine phosphatase are derived from *Escherichia coli.*

In one embodiment, the dihydroxy acid dehydratase includes an amino acid sequence shown in SEQ ID NO: 11 or an amino acid sequence having at least 75%, 80%, 85%, 90%, 91%, 92%, 93%, 94%, 95%, 96%, 97%, 98%, 99% or higher identity thereto and having dihydroxy acid dehydratase activity.

In one embodiment, the 2-dehydropantoate-2-reductase includes an amino acid sequence shown in SEQ ID NO: 17 or an amino acid sequence having at least 75%, 80%, 85%, 90%, 91%, 92%, 93%, 94%, 95%, 96%, 97%, 98%, 99% or higher identity thereto and having 2-dehydropantoate-2-reductase activity.

In one embodiment, the aminomethyltransferase includes an amino acid sequence shown in SEQ ID NO: 21 or an amino acid sequence having at least 75%, 80%, 85%, 90%, 91%, 92%, 93%, 94%, 95%, 96%, 97%, 98%, 99% or higher identity thereto and having aminomethyltransferase activity.

In one embodiment, the glycine decarboxylase includes an amino acid sequence shown in SEQ ID NO: 23 or an amino acid sequence having at least 75%, 80%, 85%, 90%, 91%, 92%, 93%, 94%, 95%, 96%, 97%, 98%, 99% or higher identity thereto and having glycine decarboxylase activity.

In one embodiment, the phosphoserine/phosphohydroxythreonine aminotransferase includes an amino acid sequence shown in SEQ ID NO: 27 or an amino acid sequence having at least 75%, 80%, 85%, 90%, 91%, 92%, 93%, 94%, 95%, 96%, 97%, 98%, 99% or higher identity thereto and having phosphoserine/phosphohydroxythreonine aminotransferase activity.

In one embodiment, the phosphoserine phosphatase includes an amino acid sequence shown in SEQ ID NO: 29 or an amino acid sequence having at least 75%, 80%, 85%, 90%, 91%, 92%, 93%, 94%, 95%, 96%, 97%, 98%, 99% or higher identity thereto and having phosphoserine phosphatase activity.

In one embodiment, the 3-methyl-2-oxobutanoate hydroxymethyltransferase includes a 3-methyl-2-oxobutanoate hydroxymethyltransferase derived from *Corynebacterium glutamicum* and/or *Escherichia coli.*

In one embodiment, the 3-methyl-2-oxobutanoate hydroxymethyltransferase includes an amino acid sequence shown in SEQ ID NO: 13 or an amino acid sequence having at least 75%, 80%, 85%, 90%, 91%, 92%, 93%, 94%, 95%, 96%, 97%, 98%, 99% or higher identity thereto and having 3-methyl-2-oxobutanoate hydroxymethyltransferase activity.

In one embodiment, the 3-methyl-2-oxobutanoate hydroxymethyltransferase includes an amino acid sequence shown in SEQ ID NO: 15 or an amino acid sequence having at least 75%, 80%, 85%, 90%, 91%, 92%, 93%, 94%, 95%, 96%, 97%, 98%, 99% or higher identity thereto and having 3-methyl-2-oxobutanoate hydroxymethyltransferase activity.

In one embodiment, the serine hydroxymethyltransferase includes an amino acid sequence shown in SEQ ID NO: 19 or an amino acid sequence having at least 75%, 80%, 85%, 90%, 91%, 92%, 93%, 94%, 95%, 96%, 97%, 98%, 99% or higher identity thereto and having serine hydroxymethyltransferase activity.

In one embodiment, the phosphoglycerate dehydrogenase is derived from *Corynebacterium glutamicum.* In particular, the phosphoglycerate dehydrogenase includes an amino acid sequence shown in SEQ ID NO: 25 or an amino acid sequence having at least 75%, 80%, 85%, 90%, 91%, 92%, 93%, 94%, 95%, 96%, 97%, 98%, 99% or higher identity thereto and having phosphoglycerate dehydrogenase activity.

As used herein, the "having or having enhanced" enzyme activity means that in the genetically engineered pantoic acid-producing strain, each of the enzymes independently has activity or has enhanced activity; that is, one or more of the enzymes may have activity while others have enhanced activity, including the cases where all have activity or all have enhanced activity. In a preferred embodiment, all of the enzymes have enhanced activity.

In some embodiments, in the genetically engineered pantoic acid-producing strain of the present invention, one or more copies of a target gene or a homologous gene thereof may be integrated into a genome (e.g., via homologous recombination), optionally at any locus in the genome (provided that such integration does not significantly adversely affect the growth and production performance of the strain). For example, one copy of any gene in the genome is replaced by one or more copies of a target gene or a homologous gene thereof. Those skilled in the art know how to integrate transgenes and select strains with integrated transgenes.

In some embodiments, the activity of the enzymes is generated or enhanced by expressing or overexpressing the genes encoding the enzymes in the strain. These genes may be inserted into loci in the genome of the strain that encode genes not required for strain survival or pantoic acid production, thereby obtaining a genetically engineered strain that can produce pantoic acid. In some embodiments, these loci include, but are not limited to, loci encoding the following enzymes: an L-serine deaminase I, a propionate kinase, a formate acetyltransferase, an alcohol dehydrogenase, a pyruvate formate lyase, a fumarate reductase, a lactate dehydrogenase, a methylglyoxal synthase, an acetate kinase, a ribokinase, a valine-pyruvate transaminase, a phosphotransacetylase, a branched-chain amino acid aminotransferase.

In one embodiment, a gene encoding the acetolactate synthase (including, for example, a gene encoding the acetolactate synthase derived from *Bacillus subtilis,* and/or genes encoding the acetolactate synthase I, the acetolactate synthase II and/or the L-valine feedback-resistant acetolactate synthase III derived from *Escherichia coli*)*,* a gene encoding the NADH-dependent acetohydroxy acid reductoisomerase derived from the *Thermacetogenium phaeum* strain, a gene encoding the dihydroxy acid dehydratase, a gene encoding the 3-methyl-2-oxobutanoate hydroxymethyltransferase derived from *Corynebacterium glutamicum,* a gene encoding the 2-dehydropantoate-2-reductase, a gene encoding the serine hydroxymethyltransferase, a gene encoding the glycine cleavage enzyme system (e.g., a gene encoding the aminomethyltransferase and/or a gene encoding the glycine decarboxylase), a gene encoding the 3-methyl-2-oxobutanoate hydroxymethyltransferase derived from *Escherichia coli,* a gene encoding the phosphoglycerate dehydrogenase, a gene encoding the phosphoserine/phosphohydroxythreonine aminotransferase, and/or a gene encoding the phosphoserine phosphatase are integrated into the genome of the genetically engineered pantoic acid-producing strain (e.g., *Escherichia coli*)*,* for example, at a position of one or more of loci encoding the following genes: an L-serine deaminase I, a propionate kinase, a formate acetyltransferase, an alcohol dehydrogenase, a pyruvate formate lyase, a fumarate reductase, a lactate dehydrogenase, a methylglyoxal synthase, an acetate kinase, a ribokinase, a valine-pyruvate transaminase, a phosphotransacetylase.

In one embodiment, the genetically engineered pantoic acid-producing strain further has reduced or inactivated activities of: an L-serine deaminase I, a propionate kinase, a formate acetyltransferase, an alcohol dehydrogenase, a pyruvate formate lyase, a fumarate reductase, a lactate dehydrogenase, a methylglyoxal synthase, an acetate kinase, a ribokinase, a valine-pyruvate transaminase, a phosphotransacetylase, and/or a branched-chain amino acid aminotransferase; preferably where the branched-chain amino acid aminotransferase is attenuated.

As used herein, the "having reduced or inactivated activities" of enzymes means that in the genetically engineered pantoic acid-producing strain, each of the enzymes is independently reduced in activity or inactivated (i.e., does not have detectable activity); that is, one or more of the enzymes may be reduced in activity while others are inactivated, including the cases where all are reduced in activity or all are inactivated.

As used herein, the "branched-chain amino acid aminotransferase is attenuated" or "attenuating the branched-chain amino acid aminotransferase" mean that the enzymatic activity of the branched-chain amino acid aminotransferase is reduced compared with the activity of a reference, for example, by at least 10%, 20%, 30%, 40%, 50%, 60%, 70%, 80%, 90%, 95%, 99% or more.

In one embodiment, the present invention provides a genetically engineered pantoic acid-producing strain,
having enhanced (e.g., overexpressed) activities of: an acetolactate synthase, an NADH-dependent acetohydroxy acid reductoisomerase, a dihydroxy acid dehydratase, a 3-methyl-2-oxobutanoate hydroxymethyltransferase, a 2-dehydropantoate-2-reductase, a serine hydroxymethyltransferase, a glycine cleavage enzyme system (e.g., an aminomethyltransferase and/or a glycine decarboxylase), a phosphoglycerate dehydrogenase, a phosphoserine/phosphohydroxythreonine aminotransferase, and a phosphoserine phosphatase;
having an attenuated branched-chain amino acid aminotransferase; and
optionally, having inactivated (e.g., knocked out) activities of: an L-serine deaminase I, a propionate kinase, a formate acetyltransferase, an alcohol dehydrogenase, a pyruvate formate lyase, a fumarate reductase, a lactate dehydrogenase, a methylglyoxal synthase, an acetate kinase, a ribokinase, a valine-pyruvate transaminase, and/or a phosphotransacetylase.

In one embodiment, the attenuated branched-chain amino acid aminotransferase includes an amino acid sequence shown in SEQ ID NO: 31 or an amino acid sequence having at least 75%, 80%, 85%, 90%, 91%, 92%, 93%, 94%, 95%, 96%, 97%, 98%, 99% or higher identity thereto and having attenuated branched-chain amino acid aminotransferase activity.

In one embodiment, the present invention provides a genetically engineered pantoic acid-producing strain,
having enhanced activities of: an acetolactate synthase derived from *Bacillus subtilis,* an acetolactate synthase I, an acetolactate synthase II, and an L-valine feedback-resistant acetolactate synthase III derived from *Escherichia coli,* an NADH-dependent acetohydroxy acid reductoisomerase derived from *Thermacetogenium phaeum,* a dihydroxy acid dehydratase, a 3-methyl-2-oxobutanoate hydroxymethyltransferase derived from *Corynebacterium glutamicum* and a 3-methyl-2-oxobutanoate hydroxymethyltransferase derived from *Escherichia coli,* a 2-dehydropantoate-2-reductase, a serine hydroxymethyltransferase, a glycine cleavage enzyme system (e.g., an aminomethyltransferase and/or a glycine decarboxylase), a phosphoglycerate dehydrogenase derived from *Corynebacterium glutamicum,* a phosphoserine/phosphohydroxythreonine aminotransferase, and a phosphoserine phosphatase;
optionally, having inactivated (e.g., knocked out), if present, activities of: an L-serine deaminase I, a propionate kinase, a formate acetyltransferase, an alcohol dehydrogenase, a pyruvate formate lyase, a fumarate reductase, a lactate dehydrogenase, a methylglyoxal synthase, an acetate kinase, a ribokinase, a valine-pyruvate transaminase, and/or a phosphotransacetylase; and
having an attenuated branched-chain amino acid aminotransferase.

In one embodiment, the genetically engineered pantoic acid-producing strain:
expresses a gene encoding the attenuated branched-chain amino acid aminotransferase, for example, a gene encoding the attenuated branched-chain amino acid aminotransferase shown in SEQ ID NO: 32; and/or
has overexpressed genes encoding: the acetolactate synthase derived from *Bacillus subtilis,* the acetolactate synthase I derived from *Escherichia coli,* the acetolactate synthase II derived from *Escherichia coli,* the L-valine feedback-resistant acetolactate synthase III derived from *Escherichia coli,* the NADH-dependent acetohydroxy acid reductoisomerase from a *Thermacetogenium phaeum* strain, the dihydroxy acid dehydratase, the 3-methyl-2-oxobutanoate hydroxymethyltransferase derived from *Corynebacterium glutamicum,* the 2-dehydropantoate-2-reductase, the serine hydroxymethyltransferase, the glycine cleavage enzyme system (e.g., the aminomethyltransferase and/or the glycine decarboxylase), the 3-methyl-2-oxobutanoate hydroxymethyltransferase derived from *Escherichia coli,* the phosphoglycerate dehydrogenase, the phosphoserine/phosphohydroxythreonine aminotransferase, and the phosphoserine phosphatase, and/or
does not have a gene encoding one or more, and preferably all of the following enzymes, or has an endogenous gene encoding one or more, and preferably all of the following enzymes knocked out: the L-serine deaminase I, the propionate kinase, the formate acetyltransferase, the alcohol dehydrogenase, the pyruvate formate lyase, the fumarate reductase, the lactate dehydrogenase, the methylglyoxal synthase, the a cetate kinase, the ribokinase, the valine-pyruvate transaminase, and the phosphotransacetylase.

In one embodiment, the gene encoding the acetolactate synthase derived from *Bacillus subtilis* encodes an acetolactate synthase shown in SEQ ID NO: 1, for example, includes a nucleotide sequence shown in SEQ ID NO: 2 or a degenerate sequence thereof, or a nucleotide sequence having at least 75%, 80%, 85%, 90%, 91%, 92%, 93%, 94%, 95%, 96%, 97%, 98%, 99% or higher identity thereto, and is preferably placed under the control of a strong promoter, for example, an M1-93 promoter (SEQ ID NO: 169).

In one embodiment, the gene encoding the acetolactate synthase I derived from *Escherichia coli* encodes a large subunit ilvB of an acetolactate synthase I shown in SEQ ID NO: 3, for example, includes a nucleotide sequence shown in SEQ ID NO: 4 or a degenerate sequence thereof, or a nucleotide sequence having at least 75%, 80%, 85%, 90%, 91%, 92%, 93%, 94%, 95%, 96%, 97%, 98%, 99% or higher identity thereto, and is preferably placed under the control of a strong promoter, for example, an M1-93 promoter (SEQ ID NO: 169).

In one embodiment, the gene encoding the acetolactate synthase II derived from *Escherichia coli* encodes a large subunit ilvG of an acetolactate synthase II shown in SEQ ID NO: 5, for example, includes a nucleotide sequence shown in SEQ ID NO: 6 or a degenerate sequence thereof, or a nucleotide sequence having at least 75%, 80%, 85%, 90%, 91%, 92%, 93%, 94%, 95%, 96%, 97%, 98%, 99% or higher identity thereto, and is preferably placed under the control of a strong promoter, for example, an M1-93 promoter (SEQ ID NO: 169).

In one embodiment, the gene encoding the L-valine feedback-resistant acetolactate synthase III derived from *Escherichia coli* encodes an L-valine feedback-resistant acetolactate synthase III shown in SEQ ID NO: 7, for example, includes a nucleotide sequence shown in SEQ ID NO: 8 or a degenerate sequence thereof, or a nucleotide sequence having at least 75%, 80%, 85%, 90%, 91%, 92%, 93%, 94%, 95%, 96%, 97%, 98%, 99% or higher identity thereto.

In one embodiment, the gene encoding the NADH-dependent acetohydroxy acid reductoisomerase encodes an NADH-dependent acetohydroxy acid reductoisomerase shown in SEQ ID NO: 9, for example, includes a nucleotide sequence shown in SEQ ID NO: 10 or a degenerate sequence thereof, or a nucleotide sequence having at least 75%, 80%, 85%, 90%, 91%, 92%, 93%, 94%, 95%, 96%, 97%, 98%, 99% or higher identity thereto, and is preferably under the control of a strong promoter, for example, an RBS5 artificial regulatory element (SEQ ID NO: 170).

In one embodiment, the gene encoding the dihydroxy acid dehydratase encodes a dihydroxy acid dehydratase shown in SEQ ID NO: 11, for example, includes a nucleotide sequence shown in SEQ ID NO: 12 or a degenerate sequence thereof, or a nucleotide sequence having at least 75%, 80%, 85%, 90%, 91%, 92%, 93%, 94%, 95%, 96%, 97%, 98%, 99% or higher identity thereto, and is preferably under the control of a strong promoter, for example, RBSL1 (SEQ ID NO: 171).

In one embodiment, the gene encoding the 3-methyl-2-oxobutanoate hydroxymethyltransferase derived from *Corynebacterium glutamicum* encodes a 3-methyl-2-oxobutanoate hydroxymethyltransferase shown in SEQ ID NO: 13, for example, includes a nucleotide sequence shown in SEQ ID NO: 14 or a degenerate sequence thereof, or a nucleotide sequence having at least 75%, 80%, 85%, 90%, 91%, 92%, 93%, 94%, 95%, 96%, 97%, 98%, 99% or higher identity thereto, and is preferably under the control of a strong promoter, for example, M1-93 (SEQ ID NO: 169).

In one embodiment, the gene encoding the 3-methyl-2-oxobutanoate hydroxymethyltransferase derived from *Escherichia coli* encodes an amino acid sequence shown in SEQ ID NO: 15, for example, includes a nucleotide sequence shown in SEQ ID NO: 16 or a degenerate sequence thereof, or a nucleotide sequence having at least 75%, 80%, 85%, 90%, 91%, 92%, 93%, 94%, 95%, 96%, 97%, 98%, 99% or higher identity thereto, and is preferably under the control of a strong promoter, for example, M1-93 (SEQ ID NO: 169).

In one embodiment, the gene encoding the 2-dehydropantoate-2-reductase encodes a 2-dehydropantoate-2-reductase shown in SEQ ID NO: 17, for example, includes a nucleotide sequence shown in SEQ ID NO: 18 or a degenerate sequence thereof, or a nucleotide sequence having at least 75%, 80%, 85%, 90%, 91%, 92%, 93%, 94%, 95%, 96%, 97%, 98%, 99% or higher identity thereto, and is preferably under the control of a strong promoter, for example, RBSL2 (SEQ ID NO: 172).

In one embodiment, the gene encoding the serine hydroxymethyltransferase encodes a serine hydroxymethyltransferase shown in SEQ ID NO: 19, for example, includes a nucleotide sequence shown in SEQ ID NO: 20 or a degenerate sequence thereof, or a nucleotide sequence having at least 75%, 80%, 85%, 90%, 91%, 92%, 93%, 94%, 95%, 96%, 97%, 98%, 99% or higher identity thereto, and is preferably under the control of a strong promoter, for example, M1-46 (SEQ ID NO: 173).

In one embodiment, the gene encoding the aminomethyltransferase encodes an aminomethyltransferase shown in SEQ ID NO: 21, for example, includes a nucleotide sequence shown in SEQ ID NO: 22 or a degenerate sequence thereof, or a nucleotide sequence having at least 75%, 80%, 85%, 90%, 91%, 92%, 93%, 94%, 95%, 96%, 97%, 98%, 99% or higher identity thereto, and is preferably under the control of a strong promoter, for example, M1-93 (SEQ ID NO: 169).

In one embodiment, the gene encoding the glycine decarboxylase encodes a glycine decarboxylase shown in SEQ ID NO: 23, for example, includes a nucleotide sequence shown in SEQ ID NO: 24 or a degenerate sequence thereof, or a nucleotide sequence having at least 75%, 80%, 85%, 90%, 91%, 92%, 93%, 94%, 95%, 96%, 97%, 98%, 99% or higher identity thereto, and is preferably under the control of a strong promoter, for example, M1-93 (SEQ ID NO: 169).

In one embodiment, the gene encoding the phosphoglycerate dehydrogenase encodes a phosphoglycerate dehydrogenase shown in SEQ ID NO: 25, for example, includes a nucleotide sequence shown in SEQ ID NO: 26 or a degenerate sequence thereof, or a nucleotide sequence having at least 75%, 80%, 85%, 90%, 91%, 92%, 93%, 94%, 95%, 96%, 97%, 98%, 99% or higher identity thereto, and is preferably under the control of a strong promoter, for example, M1-93 (SEQ ID NO: 169).

In one embodiment, the gene encoding the phosphoserine/phosphohydroxythreonine aminotransferase encodes a phosphoserine/phosphohydroxythreonine aminotransferase shown in SEQ ID NO: 27, for example, includes a nucleotide sequence shown in SEQ ID NO: 28 or a degenerate sequence thereof, or a nucleotide sequence having at least 75%, 80%, 85%, 90%, 91%, 92%, 93%, 94%, 95%, 96%, 97%, 98%, 99% or higher identity thereto, and is preferably under the control of a strong promoter, for example, M1-93 (SEQ ID NO: 169).

In one embodiment, the gene encoding the phosphoserine phosphatase encodes a phosphoserine phosphatase shown in SEQ ID NO: 29, for example, includes a nucleotide sequence shown in SEQ ID NO: 30 or a degenerate sequence thereof, or a nucleotide sequence having at least 75%, 80%, 85%, 90%, 91%, 92%, 93%, 94%, 95%, 96%, 97%, 98%, 99% or higher identity thereto, and is preferably under the control of a strong promoter, for example, M1-93 (SEQ ID NO: 169).

In one embodiment, the genetically engineered pantoic acid-producing strain (e.g., *Escherichia coli*):
expresses an enzyme including an amino acid sequence shown in SEQ ID NO: 31, and, for example, expresses a gene including a nucleotide sequence shown in SEQ ID NO: 32;
overexpresses enzymes respectively including amino acid sequences shown in SEQ ID NOs: 1, 3, 5, 7, 9, 11, 13, 15, 17, 19, 21, 23, 25, 27, and 29, and, for example, overexpresses genes respectively including nucleotide sequences shown in SEQ ID NOs: 2, 4, 6, 8, 10, 12, 14, 16, 18, 20, 22, 24, 26, 28, and 30; and
optionally, does not have a gene encoding one or more, and preferably all of the following enzymes, or has an endogenous gene encoding one or more, and preferably all of the following enzymes knocked out: the L-serine deaminase I, the propionate kinase, the formate acetyltransferase, the alcohol dehydrogenase, the pyruvate formate lyase, the fumarate reductase, the lactate dehydrogenase, the methylglyoxal synthase, the a cetate kinase, the ribokinase, the valine-pyruvate transaminase, and the phosphotransacetylase.

In one embodiment, the pantoic acid-producing strain is selected from *Escherichia, Enterobacter, Corynebacterium glutamicum, Bacillus subtilis,* and yeast, and preferably *Escherichia coli.*

In one embodiment, the genetically engineered pantoic acid-producing strain is *Escherichia coli* deposited in the China General Microbiological Culture Collection Center (CGMCC) in Beijing, China, with a deposit number CGMCC No. 26276.

In one aspect, the present invention provides a method for producing a genetically engineered pantoic acid-producing strain, including: conferring or enhancing activity of an NADH-dependent acetohydroxy acid reductoisomerase in a pantoic acid-producing strain.

In one embodiment, the NADH-dependent acetohydroxy acid reductoisomerase is derived from microorganisms such as *Escherichia coli, Corynebacterium glutamicum, Bacillus subtilis,* and the like. In a preferred embodiment, the NADH-dependent acetohydroxy acid reductoisomerase is derived from *Thermacetogenium phaeum,* and more preferably includes an amino acid sequence shown in SEQ ID NO: 9 or an amino acid sequence having at least 75%, 80%, 85%, 90%, 91%, 92%, 93%, 94%, 95%, 96%, 97%, 98%, 99% or higher identity thereto and having NADH-dependent acetohydroxy acid reductoisomerase activity.

In one embodiment, the method includes expressing or overexpressing a gene encoding the NADH-dependent acetohydroxy acid reductoisomerase in the pantoic acid-producing strain. In one embodiment, the gene encoding the NADH-dependent acetohydroxy acid reductoisomerase encodes an NADH-dependent acetohydroxy acid reductoisomerase shown in SEQ ID NO: 9, for example, includes a nucleotide sequence shown in SEQ ID NO: 10 or a degenerate sequence thereof, or a nucleotide sequence having at least 75%, 80%, 85%, 90%, 91%, 92%, 93%, 94%, 95%, 96%, 97%, 98%, 99% or higher identity thereto, and is preferably under the control of a strong promoter, for example, an RBS5 artificial regulatory element (SEQ ID NO: 170).

In one embodiment, the method includes, in the pantoic acid-producing strain: expressing or overexpressing the gene encoding the NADH-dependent acetohydroxy acid reductoisomerase.

The pantoic acid-producing strain may be a strain known in the art or a strain obtainable by technical knowledge in the art.

In addition to conferring or enhancing the NADH-dependent acetohydroxy acid reductoisomerase, the method may further include performing other genetic modifications required for D-pantoic acid production in the genetically engineered pantoic acid-producing strain, for example, modifying other enzymes or regulatory molecules involved in a pantoic acid production pathway. For example, other genetic modifications may be performed and the NADH-dependent acetohydroxy acid reductoisomerase may be conferred or enhanced in a suitable strain (e.g., a strain that does not produce detectable accumulation of D-pantoic acid), enabling the strain to produce a detectable amount of D-pantoic acid accumulation.

For example, in one embodiment, the present invention provides a method for producing a genetically engineered pantoic acid-producing strain, including: in a strain, conferring or enhancing activities of: an NADH-dependent acetohydroxy acid reductoisomerase, an acetolactate synthase, a dihydroxy acid dehydratase, a 3-methyl-2-oxobutanoate hydroxymethyltransferase, a 2-dehydropantoate-2-reductase, a serine hydroxymethyltransferase, a glycine cleavage enzyme system (e.g., an aminomethyltransferase and/or a glycine decarboxylase), a phosphoglycerate dehydrogenase, a phosphoserine/phosphohydroxythreonine aminotransferase, and a phosphoserine phosphatase.

In one embodiment, the NADH-dependent acetohydroxy acid reductoisomerase is derived from microorganisms such as *Escherichia coli, Corynebacterium glutamicum, Bacillus subtilis,* and the like. In a preferred embodiment, the NADH-dependent acetohydroxy acid reductoisomerase is derived from *Thermacetogenium phaeum,* and more preferably includes an amino acid sequence shown in SEQ ID NO: 9 or an amino acid sequence having at least 75%, 80%, 85%, 90%, 91%, 92%, 93%, 94%, 95%, 96%, 97%, 98%, 99% or higher identity thereto and having NADH-dependent acetohydroxy acid reductoisomerase activity.

In one embodiment, the method includes expressing or overexpressing a gene encoding the NADH-dependent acetohydroxy acid reductoisomerase in the pantoic acid-producing strain. In one embodiment, the gene encoding the NADH-dependent acetohydroxy acid reductoisomerase encodes an NADH-dependent acetohydroxy acid reductoisomerase shown in SEQ ID NO: 9, for example, includes a nucleotide sequence shown in SEQ ID NO: 10 or a degenerate sequence thereof, or a nucleotide sequence having at least 75%, 80%, 85%, 90%, 91%, 92%, 93%, 94%, 95%, 96%, 97%, 98%, 99% or higher identity thereto, and is preferably under the control of a strong promoter, for example, an RBS5 artificial regulatory element (SEQ ID NO: 170).

In one embodiment, the acetolactate synthase includes an acetolactate synthase derived from *Bacillus subtilis.* In particular, the acetolactate synthase includes an amino acid sequence shown in SEQ ID NO: 1 or an amino acid sequence having at least 75%, 80%, 85%, 90%, 91%, 92%, 93%, 94%, 95%, 96%, 97%, 98%, 99% or higher identity thereto and having acetolactate synthase activity.

In one embodiment, the acetolactate synthase includes an acetolactate synthase I, an acetolactate synthase II, and/or an L-valine feedback-resistant acetolactate synthase III derived from *Escherichia coli.* In one embodiment, the acetolactate synthase I includes an amino acid sequence shown in SEQ ID NO: 3 or an amino acid sequence having at least 75%, 80%, 85%, 90%, 91%, 92%, 93%, 94%, 95%, 96%, 97%, 98%, 99% or higher identity thereto and having acetolactate synthase I activity. In one embodiment, the acetolactate synthase II includes an amino acid sequence shown in SEQ ID NO: 5 or an amino acid sequence having at least 75%, 80%, 85%, 90%, 91%, 92%, 93%, 94%, 95%, 96%, 97%, 98%, 99% or higher identity thereto and having acetolactate synthase II activity. In one embodiment, the L-valine feedback-resistant acetolactate synthase III includes an amino acid sequence shown in SEQ ID NO: 7 or an amino acid sequence having at least 75%, 80%, 85%, 90%, 91%, 92%, 93%, 94%, 95%, 96%, 97%, 98%, 99% or higher identity thereto and having L-valine feedback-resistant acetolactate synthase III activity.

In one embodiment, the method includes, in a strain, conferring or enhancing activities of the acetolactate synthase derived from *Bacillus subtilis,* and the acetolactate synthase I, the acetolactate synthase II, and the L-valine feedback-resistant acetolactate synthase III derived from *Escherichia coli.*

In one embodiment, the dihydroxy acid dehydratase, the 2-dehydropantoate-2-reductase, the glycine cleavage enzyme system (e.g., the aminomethyltransferase and/or the glycine decarboxylase), the phosphoserine/phosphohydroxythreonine aminotransferase, and the phosphoserine phosphatase are derived from *Escherichia coli.*

In one embodiment, the dihydroxy acid dehydratase includes an amino acid sequence shown in SEQ ID NO: 11 or an amino acid sequence having at least 75%, 80%, 85%, 90%, 91%, 92%, 93%, 94%, 95%, 96%, 97%, 98%, 99% or higher identity thereto and having dihydroxy acid dehydratase activity.

In one embodiment, the 2-dehydropantoate-2-reductase includes an amino acid sequence shown in SEQ ID NO: 17 or an amino acid sequence having at least 75%, 80%, 85%, 90%, 91%, 92%, 93%, 94%, 95%, 96%, 97%, 98%, 99% or higher identity thereto and having 2-dehydropantoate-2-reductase activity.

In one embodiment, the serine hydroxymethyltransferase includes an amino acid sequence shown in SEQ ID NO: 19 or an amino acid sequence having at least 75%, 80%, 85%, 90%, 91%, 92%, 93%, 94%, 95%, 96%, 97%, 98%, 99% or higher identity thereto and having serine hydroxymethyltransferase activity.

In one embodiment, the aminomethyltransferase includes an amino acid sequence shown in SEQ ID NO: 21 or an amino acid sequence having at least 75%, 80%, 85%, 90%, 91%, 92%, 93%, 94%, 95%, 96%, 97%, 98%, 99% or higher identity thereto and having aminomethyltransferase activity.

In one embodiment, the glycine decarboxylase includes an amino acid sequence shown in SEQ ID NO: 23 or an amino acid sequence having at least 75%, 80%, 85%, 90%, 91%, 92%, 93%, 94%, 95%, 96%, 97%, 98%, 99% or higher identity thereto and having glycine decarboxylase activity.

In one embodiment, the phosphoserine/phosphohydroxythreonine aminotransferase includes an amino acid sequence shown in SEQ ID NO: 27 or an amino acid sequence having at least 75%, 80%, 85%, 90%, 91%, 92%, 93%, 94%, 95%, 96%, 97%, 98%, 99% or higher identity thereto and having phosphoserine/phosphohydroxythreonine aminotransferase activity.

In one embodiment, the phosphoserine phosphatase includes an amino acid sequence shown in SEQ ID NO: 29 or an amino acid sequence having at least 75%, 80%, 85%, 90%, 91%, 92%, 93%, 94%, 95%, 96%, 97%, 98%, 99% or higher identity thereto and having phosphoserine phosphatase activity.

In one embodiment, the 3-methyl-2-oxobutanoate hydroxymethyltransferase includes a 3-methyl-2-oxobutanoate hydroxymethyltransferase derived from *Corynebacterium glutamicum* and/or *Escherichia coli.*

In one embodiment, the 3-methyl-2-oxobutanoate hydroxymethyltransferase is derived from *Corynebacterium glutamicum.* In particular, the 3-methyl-2-oxobutanoate hydroxymethyltransferase includes an amino acid sequence shown in SEQ ID NO: 13 or an amino acid sequence having at least 75%, 80%, 85%, 90%, 91%, 92%, 93%, 94%, 95%, 96%, 97%, 98%, 99% or higher identity thereto and having 3-methyl-2-oxobutanoate hydroxymethyltransferase activity.

In one embodiment, the 3-methyl-2-oxobutanoate hydroxymethyltransferase is derived from *Escherichia coli.* In particular, the 3-methyl-2-oxobutanoate hydroxymethyltransferase includes an amino acid sequence shown in SEQ ID NO: 15 or an amino acid sequence having at least 75%, 80%, 85%, 90%, 91%, 92%, 93%, 94%, 95%, 96%, 97%, 98%, 99% or higher identity thereto and having 3-methyl-2-oxobutanoate hydroxymethyltransferase activity.

In one embodiment, the phosphoglycerate dehydrogenase is derived from *Corynebacterium glutamicum.* In particular, the phosphoglycerate dehydrogenase includes an amino acid sequence shown in SEQ ID NO: 25 or an amino acid sequence having at least 75%, 80%, 85%, 90%, 91%, 92%, 93%, 94%, 95%, 96%, 97%, 98%, 99% or higher identity thereto and having phosphoglycerate dehydrogenase activity.

As used herein, the "confer or enhance" means that in the pantoic acid-producing strain, one or more of the enzymes have activity, while the activity of the other enzymes is enhanced, including the cases where the activity of all the enzymes is enhanced. In a preferred embodiment, all of the enzymes have enhanced activity.

To confer or enhance the activity of one or more enzymes, one or more copies of a target gene or a homologous gene thereof may be integrated into a genome (e.g., via homologous recombination), optionally at any locus in the genome (provided that such integration does not significantly adversely affect the growth and the production of the desired compound such as D-pantoic acid of the strain). For example, one copy of any gene in the genome is replaced by one or more copies of a target gene or a homologous gene thereof. Those skilled in the art know how to integrate transgenes and select the locus in the strain for integrating the desired transgene.

The activity of the enzymes is generated or enhanced by expressing or overexpressing the genes encoding the enzymes described herein in the strain. These genes may be inserted into loci in the genome of the strain that encode genes not required for strain survival or pantoic acid production, thereby obtaining a genetically engineered strain that can produce pantoic acid. These loci include, but are not limited to, loci encoding the following enzymes: an L-serine deaminase I, a propionate kinase, a formate acetyltransferase, an alcohol dehydrogenase, a pyruvate formate lyase, a fumarate reductase, a lactate dehydrogenase, a methylglyoxal synthase, an acetate kinase, a ribokinase, a valine-pyruvate transaminase, a phosphotransacetylase, a branched-chain amino acid aminotransferase.

In one embodiment, the method includes further reducing or inactivating activity of one or more of the following enzymes: an L-serine deaminase I, a propionate kinase, a formate acetyltransferase, an alcohol dehydrogenase, a pyruvate formate lyase, a fumarate reductase, a lactate dehydrogenase, a methylglyoxal synthase, an acetate kinase, a ribokinase, a valine-pyruvate transaminase, a phosphotransacetylase, and a branched-chain amino acid transaminase; and preferably attenuating enzymatic activity of a branched-chain amino acid aminotransferase.

As used herein, the "reduce or inactivate" means that in the genetically engineered pantoic acid-producing strain, if the enzymes are present, their activity is reduced or inactivated (i.e., to undetectable activity); that is, one or more of the enzymes may be reduced in activity while others are inactivated, including the cases where all are reduced in activity or all are inactivated.

In one embodiment, the attenuated branched-chain amino acid transaminase includes the amino acid sequence shown in SEQ ID NO: 31, or an amino acid sequence having at least 75%, 80%, 85%, 90%, 91%, 92%, 93%, 94%, 95%, 96%, 97%, 98%, 99% or higher identity thereto and having attenuated branched-chain amino acid transaminase activity.

In one embodiment, the method includes:
expressing or overexpressing, and preferably overexpressing genes encoding: the NADH-dependent acetohydroxy acid reductoisomerase, the acetolactate synthase, the dihydroxy acid dehydratase, the 3-methyl-2-oxobutanoate hydroxymethyltransferase, the 2-dehydropantoate-2-reductase, the serine hydroxymethyltransferase, the glycine cleavage enzyme system (e.g., the aminomethyltransferase and/or the glycine decarboxylase), the phosphoglycerate dehydrogenase, the phosphoserine/phosphohydroxythreonine aminotransferase, and the phosphoserine phosphatase; and
optionally, knocking out a gene, if present, encoding one or more, and preferably all of the following enzymes: the L-serine deaminase I, the propionate kinase, the formate acetyltransferase, the alcohol dehydrogenase, the pyruvate formate lyase, the fumarate reductase, the lactate dehydrogenase, the methylglyoxal synthase, the acetate kinase, the ribokinase, the valine-pyruvate transaminase, and the phosphotransacetylase.

In one embodiment, the method for producing a genetically engineered pantoic acid-producing strain includes, in the strain:
expressing the attenuated branched-chain amino acid transaminase, for example, expressing a protein shown in SEQ ID NO: 31, and in particular, expressing a gene including a nucleotide sequence shown in SEQ ID NO: 32 or a degenerate sequence thereof, or a nucleotide sequence having at least 75%, 80%, 85%, 90%, 91%, 92%, 93%, 94%, 95%, 96%, 97%, 98%, 99% or higher identity thereto; and/or
overexpressing genes encoding: the acetolactate synthase derived from *Bacillus subtilis,* the acetolactate synthase I derived from *Escherichia coli,* the acetolactate synthase II derived from *Escherichia coli,* the L-valine feedback-resistant acetolactate synthase III derived from *Escherichia coli,* the NADH-dependent acetohydroxy acid reductoisomerase from a *Thermacetogenium phaeum* strain, the dihydroxy acid dehydratase, the 3-methyl-2-oxobutanoate hydroxymethyltransferase derived from *Corynebacterium glutamicum,* the 2-dehydropantoate-2-reductase, the serine hydroxymethyltransferase, the glycine cleavage enzyme system (e.g., the aminomethyltransferase and/or the glycine decarboxylase), the 3-methyl-2-oxobutanoate hydroxymethyltransferase derived from *Escherichia coli,* the phosphoglycerate dehydrogenase, the phosphoserine/phosphohydroxythreonine aminotransferase, and the phosphoserine phosphatase, and/or
optionally, knocking out a gene, if present, encoding one or more, and preferably all of the following enzymes: the L-serine deaminase I, the propionate kinase, the formate acetyltransferase, the alcohol dehydrogenase, the pyruvate formate lyase, the fumarate reductase, the lactate dehydrogenase, the methylglyoxal synthase, the acetate kinase, the ribokinase, the valine-pyruvate transaminase, and the phosphotransacetylase.

In one embodiment, the gene encoding the acetolactate synthase derived from *Bacillus subtilis* encodes an acetolactate synthase shown in SEQ ID NO: 1, for example, includes a nucleotide sequence shown in SEQ ID NO: 2 or a degenerate sequence thereof, or a nucleotide sequence having at least 75%, 80%, 85%, 90%, 91%, 92%, 93%, 94%, 95%, 96%, 97%, 98%, 99% or higher identity thereto, and is preferably placed under the control of a strong promoter, for example, an M1-93 promoter (SEQ ID NO: 169).

In one embodiment, the gene encoding the acetolactate synthase I derived from *Escherichia coli* encodes a large subunit ilvB of an acetolactate synthase I shown in SEQ ID NO: 3, for example, includes a nucleotide sequence shown in SEQ ID NO: 4 or a degenerate sequence thereof, or a nucleotide sequence having at least 75%, 80%, 85%, 90%, 91%, 92%, 93%, 94%, 95%, 96%, 97%, 98%, 99% or higher identity thereto, and is preferably placed under the control of a strong promoter, for example, an M1-93 promoter (SEQ ID NO: 169).

In one embodiment, the gene encoding the acetolactate synthase II derived from *Escherichia coli* encodes a large subunit ilvG of an acetolactate synthase II shown in SEQ ID NO: 5, for example, includes a nucleotide sequence shown in SEQ ID NO: 6 or a degenerate sequence thereof, or a nucleotide sequence having at least 75%, 80%, 85%, 90%, 91%, 92%, 93%, 94%, 95%, 96%, 97%, 98%, 99% or higher identity thereto, and is preferably placed under the control of a strong promoter, for example, an M1-93 promoter (SEQ ID NO: 169).

In one embodiment, the gene encoding the L-valine feedback-resistant acetolactate synthase III derived from *Escherichia coli* encodes an L-valine feedback-resistant acetolactate synthase III shown in SEQ ID NO: 7, for example, includes a nucleotide sequence shown in SEQ ID NO: 8 or a degenerate sequence thereof, or a nucleotide sequence having at least 75%, 80%, 85%, 90%, 91%, 92%, 93%, 94%, 95%, 96%, 97%, 98%, 99% or higher identity thereto.

In one embodiment, the gene encoding the NADH-dependent acetohydroxy acid reductoisomerase encodes an NADH-dependent acetohydroxy acid reductoisomerase shown in SEQ ID NO: 9, for example, includes a nucleotide sequence shown in SEQ ID NO: 10 or a degenerate sequence thereof, or a nucleotide sequence having at least 75%, 80%, 85%, 90%, 91%, 92%, 93%, 94%, 95%, 96%, 97%, 98%, 99% or higher identity thereto, and is preferably under the control of a strong promoter, for example, an RBS5 artificial regulatory element (SEQ ID NO: 170).

In one embodiment, the gene encoding the dihydroxy acid dehydratase encodes a dihydroxy acid dehydratase shown in SEQ ID NO: 11, for example, includes a nucleotide sequence shown in SEQ ID NO: 12 or a degenerate sequence thereof, or a nucleotide sequence having at least 75%, 80%, 85%, 90%, 91%, 92%, 93%, 94%, 95%, 96%, 97%, 98%, 99% or higher identity thereto, and is preferably under the control of a strong promoter, for example, RBSL1 (SEQ ID NO: 171).

In one embodiment, the gene encoding the 3-methyl-2-oxobutanoate hydroxymethyltransferase derived from *Corynebacterium glutamicum* encodes a 3-methyl-2-oxobutanoate hydroxymethyltransferase shown in SEQ ID NO: 13, for example, includes a nucleotide sequence shown in SEQ ID NO: 14 or a degenerate sequence thereof, or a nucleotide sequence having at least 75%, 80%, 85%, 90%, 91%, 92%, 93%, 94%, 95%, 96%, 97%, 98%, 99% or higher identity thereto, and is preferably under the control of a strong promoter, for example, M1-93 (SEQ ID NO: 169).

In one embodiment, the gene encoding the 3-methyl-2-oxobutanoate hydroxymethyltransferase derived from *Escherichia coli* encodes a 3-methyl-2-oxobutanoate hydroxymethyltransferase shown in SEQ ID NO: 15, for example, includes a nucleotide sequence shown in SEQ ID NO: 16 or a degenerate sequence thereof, or a nucleotide sequence having at least 75%, 80%, 85%, 90%, 91%, 92%, 93%, 94%, 95%, 96%, 97%, 98%, 99% or higher identity thereto, and is preferably under the control of a strong promoter, for example, M1-93 (SEQ ID NO: 169).

In one embodiment, the gene encoding the 2-dehydropantoate-2-reductase encodes a 2-dehydropantoate-2-reductase shown in SEQ ID NO: 17, for example, includes a nucleotide sequence shown in SEQ ID NO: 18 or a degenerate sequence thereof, or a nucleotide sequence having at least 75%, 80%, 85%, 90%, 91%, 92%, 93%, 94%, 95%, 96%, 97%, 98%, 99% or higher identity thereto, and is preferably under the control of a strong promoter, for example, RBSL2 (SEQ ID NO: 172).

In one embodiment, the gene encoding the serine hydroxymethyltransferase encodes a serine hydroxymethyltransferase shown in SEQ ID NO: 19, for example, includes a nucleotide sequence shown in SEQ ID NO: 20 or a degenerate sequence thereof, or a nucleotide sequence having at least 75%, 80%, 85%, 90%, 91%, 92%, 93%, 94%, 95%, 96%, 97%, 98%, 99% or higher identity thereto, and is preferably under the control of a strong promoter, for example, M1-46 (SEQ ID NO: 173).

In one embodiment, the gene encoding the aminomethyltransferase encodes an aminomethyltransferase shown in SEQ ID NO: 21, for example, includes a nucleotide sequence shown in SEQ ID NO: 22 or a degenerate sequence thereof, or a nucleotide sequence having at least 75%, 80%, 85%, 90%, 91%, 92%, 93%, 94%, 95%, 96%, 97%, 98%, 99% or higher identity thereto, and is preferably under the control of a strong promoter, for example, M1-93 (SEQ ID NO: 169).

In one embodiment, the gene encoding the glycine decarboxylase encodes a glycine decarboxylase shown in SEQ ID NO: 23, for example, includes a nucleotide sequence shown in SEQ ID NO: 24 or a degenerate sequence thereof, or a nucleotide sequence having at least 75%, 80%, 85%, 90%, 91%, 92%, 93%, 94%, 95%, 96%, 97%, 98%, 99% or higher identity thereto, and is preferably under the control of a strong promoter, for example, M1-93 (SEQ ID NO: 169).

In one embodiment, the gene encoding the phosphoglycerate dehydrogenase encodes a phosphoglycerate dehydrogenase shown in SEQ ID NO: 25, for example, includes a nucleotide sequence shown in SEQ ID NO: 26 or a degenerate sequence thereof, or a nucleotide sequence having at least 75%, 80%, 85%, 90%, 91%, 92%, 93%, 94%, 95%, 96%, 97%, 98%, 99% or higher identity thereto, and is preferably under the control of a strong promoter, for example, M1-93 (SEQ ID NO: 169).

In one embodiment, the gene encoding the phosphoserine/phosphohydroxythreonine aminotransferase encodes a phosphoserine/phosphohydroxythreonine aminotransferase shown in SEQ ID NO: 27, for example, includes a nucleotide sequence shown in SEQ ID NO: 28 or a degenerate sequence thereof, or a nucleotide sequence having at least 75%, 80%, 85%, 90%, 91%, 92%, 93%, 94%, 95%, 96%, 97%, 98%, 99% or higher identity thereto, and is preferably under the control of a strong promoter, for example, M1-93 (SEQ ID NO: 169).

In one embodiment, the gene encoding the phosphoserine phosphatase encodes a phosphoserine phosphatase shown in SEQ ID NO: 29, for example, includes a nucleotide sequence shown in SEQ ID NO: 30 or a degenerate sequence thereof, or a nucleotide sequence having at least 75%, 80%, 85%, 90%, 91%, 92%, 93%, 94%, 95%, 96%, 97%, 98%, 99% or higher identity thereto, and is preferably under the control of a strong promoter, for example, M1-93 (SEQ ID NO: 169).

In one embodiment, the method includes, in a pantoic acid-producing strain (e.g., *Escherichia coli*):
expressing an enzyme including an amino acid sequence shown in SEQ ID NO: 31, and, for example, expressing a gene including a nucleotide sequence shown in SEQ ID NO: 32;
overexpressing enzymes respectively including amino acid sequences shown in SEQ ID NOs: 1, 3, 5, 7, 9, 11, 13, 15, 17, 19, 21, 23, 25, 27, and 29, and, for example, overexpressing genes respectively including nucleotide sequences shown in SEQ ID NOs: 2, 4, 6, 8, 10, 12, 14, 16, 18, 20, 22, 24, 26, 28, and 30; and
optionally, knocking out genes encoding the following enzymes: the L-serine deaminase I, the propionate kinase, the formate acetyltransferase, the alcohol dehydrogenase, the pyruvate formate lyase, the fumarate reductase, the lactate dehydrogenase, the methylglyoxal synthase, the acetate kinase, the ribokinase, the valine-pyruvate transaminase, and/or the phosphotransacetylase.

In one embodiment, the method includes performing the steps of conferring or enhancing the enzyme activities and optionally reducing or inactivating the enzyme activities in a strain selected from *Escherichia, Enterobacter, Corynebacterium glutamicum, Bacillus subtilis,* and yeast, so as to obtain the genetically engineered pantoic acid-producing strain.

In one embodiment, the method includes performing the steps of conferring or enhancing enzyme activities and reducing or inactivating enzyme activities in an *Escherichia coli* strain (e.g., Escherichia coli ATCC 8739), so as to obtain the genetically engineered pantoic acid-producing strain.

In one embodiment, the method includes, in *Escherichia coli* deposited in the China General Microbiological Culture Collection Center (CGMCC) in Beijing, China, with a deposit number CGMCC No. 21699, replacing an NADPH-dependent acetohydroxy acid reductoisomerase-encoding gene (e.g., the amino acid sequence shown in SEQ ID NO: 167 and the nucleotide sequence shown in SEQ ID NO: 168) with a gene encoding an NADH-dependent acetohydroxy acid reductoisomerase, so as to obtain the genetically engineered pantoic acid-producing strain. Preferably, the NADH-dependent acetohydroxy acid reductoisomerase is derived from a *Thermacetogenium phaeum* strain, and, for example, includes an amino acid sequence shown in SEQ ID NO: 9 or an amino acid sequence having at least 75%, 80%, 85%, 90%, 91%, 92%, 93%, 94%, 95%, 96%, 97%, 98%, 99% or higher identity thereto and having NADH-dependent acetohydroxy acid reductoisomerase activity.

In one embodiment, the gene encoding the NADH-dependent acetohydroxy acid reductoisomerase includes a nucleotide sequence shown in SEQ ID NO: 10 or a degenerate sequence thereof, or a nucleotide sequence having at least 75%, 80%, 85%, 90%, 91%, 92%, 93%, 94%, 95%, 96%, 97%, 98%, 99% or higher identity thereto.

In one embodiment, the genetically engineered pantoic acid-producing strain obtained by the method is *Escherichia coli* deposited in the China General Microbiological Culture Collection Center (CGMCC) in Beijing, China, with a deposit number CGMCC No. 26276.

In one aspect, the present invention provides a method for producing D-pantoic acid, including culturing the genetically engineered strain producing pantoic acid of the present invention or a genetically engineered strain producing pantoic acid prepared by the method for producing a genetically engineered strain producing pantoic acid of the present invention under conditions suitable for fermentative production of D-pantoic acid, and optionally including isolating and purifying produced D-pantoic acid.

Conditions known in the art for fermentative culture of a strain producing pantoic acid for fermentative production of D-pantoic acid include, but not limited to, pH, temperature, medium components, fermentation time, and the like.

Temperatures known in the art for fermentative production of D-pantoic acid by a strain producing pantoic acid are, for example, about 25-37°C, for example, about 25°C, about 26°C, about 27°C, about 28°C, about 29°C, about 30°C, about 31°C, about 32°C, about 33°C, about 34°C, about 35°C, about 36°C, and about 37°C. In one embodiment, the pantoic acid-producing yeast strain of the present invention is fermented at 37°C to produce D-pantoic acid.

The strain producing pantoic acid of the present invention can be fermented at a suitable pH known in the art, for example, a pH of about 6.5-7.5. In one embodiment, the strain producing pantoic acid of the present invention is fermented at a pH of about 7.0 to produce D-pantoic acid.

For production of D-pantoic acid, the strain producing pantoic acid of the present invention can be fermented for a suitable period of time, for example, about 12-96 hours, such as about 12 hours, about 24 hours, about 36 hours, about 48 hours, about 60 hours, about 72 hours, about 96 hours, about 120 hours, about 144 hours, and about 168 hours.

In one aspect, the present invention provides use of the genetically engineered strain producing pantoic acid of the present invention or a genetically engineered strain producing pantoic acid prepared by the method for producing a genetically engineered strain producing pantoic acid of the present invention in production of D-pantoic acid.

In one aspect, the present invention provides a method for improving D-pantoic acid production of a pantoic acid-producing strain, including: conferring or enhancing activity of an NADH-dependent acetohydroxy acid reductoisomerase in the pantoic acid-producing strain.

In one embodiment, the pantoic acid-producing strain does not have an NADH-dependent acetohydroxy acid reductoisomerase, but has, for example, an NADPH-dependent acetohydroxy acid reductoisomerase.

In one embodiment, the NADH-dependent acetohydroxy acid reductoisomerase is derived from microorganisms such as *Escherichia coli, Corynebacterium glutamicum, Bacillus subtilis,* and the like. In a preferred embodiment, the NADH-dependent acetohydroxy acid reductoisomerase is derived from *Thermacetogenium phaeum,* and more preferably includes an amino acid sequence shown in SEQ ID NO: 9 or an amino acid sequence having at least 75%, 80%, 85%, 90%, 91%, 92%, 93%, 94%, 95%, 96%, 97%, 98%, 99% or higher identity thereto and having NADH-dependent acetohydroxy acid reductoisomerase activity.

In one embodiment, the method includes replacing the NADPH-dependent acetohydroxy acid reductoisomerase in the pantoic acid-producing strain having the NADPH-dependent acetohydroxy acid reductoisomerase with the NADH-dependent acetohydroxy acid reductoisomerase, thereby improving the D-pantoic acid production capacity of the strain. The replacement can be performed using any suitable method known in the art, for example, replacing a gene encoding the NADPH-dependent acetohydroxy acid reductoisomerase in the strain with a gene encoding the NADH-dependent acetohydroxy acid reductoisomerase via homologous recombination.

The gene encoding the NADH-dependent acetohydroxy acid reductoisomerase introduced into the strain can be placed under the control of an appropriate promoter, for example, a strong promoter (e.g., an RBS5 artificial regulatory element (SEQ ID NO: 170)), so as to enable expression or overexpression, and preferably overexpression of the gene in the strain. In one embodiment, the gene encoding the NADH-dependent acetohydroxy acid reductoisomerase encodes an NADH-dependent acetohydroxy acid reductoisomerase shown in SEQ ID NO: 9, and, for example, includes a nucleotide sequence shown in SEQ ID NO: 10 or a degenerate sequence thereof, or a nucleotide sequence having at least 75%, 80%, 85%, 90%, 91%, 92%, 93%, 94%, 95%, 96%, 97%, 98%, 99% or higher identity thereto.

In one embodiment, the method includes replacing the NADPH-dependent acetohydroxy acid reductoisomerase-encoding gene in *Escherichia coli* deposited in the China General Microbiological Culture Collection Center (CGMCC) in Beijing, China, with a deposit number CGMCC No. 21699 with the gene encoding the NADH-dependent acetohydroxy acid reductoisomerase, so as to improve pantoic acid production in *Escherichia coli* CGMCC No. 21699.

As used herein, "optional" or "optionally" means that the subsequently described event or circumstance may or may not occur, and the description includes the cases where the event or circumstance occurs and does not occur. For example, an optionally included step means that the step may be present or absent.

As used herein, the term "about" refers to a numerical range that includes a specific value, which those skilled in the art can reasonably consider to be similar to the specific value. In some embodiments, the term "about" refers to within the standard error of measurement commonly accepted in the art. In some embodiments, "about" refers to +/- 10% of the specific value.

References herein to "one or more" should be understood to disclose specific various combinations correspondingly. For example, "one or more of A, B, C" should be considered to disclose each specific combination such as A, B, C, AB, AC, BC, and ABC.

The ranges disclosed herein should be considered to specifically disclose all possible subranges and individual values within the range. For example, a description of a range from 1 to 6 should be considered to explicitly disclose subranges such as from 1 to 3, from 1 to 4, from 1 to 5, from 2 to 4, from 2 to 6, from 3 to 6, etc., as well as individual numbers within the range, for example, 1, 2, 3, 4, 5, and 6.

### Description of Biological Material Deposit

Taxonomic Name: *Escherichia coli*
Strain Number: Span050
Name of Depositary Institution: China General Microbiological Culture Collection Center
Abbreviation of Depositary Institution: CGMCC
Address of Depositary Institution: No. 3, Courtyard 1, Beichen West Road, Chaoyang District, Beijing, Postcode: 100101
Date of Deposit: January 22, 2021
Registration Number of Depositary Center: CGMCC No. 21699
Taxonomic Name: *Escherichia coli*
Strain Number: Span096
Name of Depositary Institution: China General Microbiological Culture Collection Center
Abbreviation of Depositary Institution: CGMCC
Address of Depositary Institution: No. 3, Courtyard 1, Beichen West Road, Chaoyang District, Beijing, Postcode: 100101
Date of Deposit:December 26, 2022
Registration Number of Depositary Center: CGMCC No. 26276

### Examples

The present invention is further illustrated by the following examples, but any example or combination thereof shall not be construed as limiting the scope or implementation of the present invention. The scope of the present invention is defined by the appended claims. By combining the present specification with common general knowledge in the art, those of ordinary skill in the art can clearly understand the scope defined by the claims. Without departing from the spirit and scope of the present invention, those skilled in the art may make any modification or change to the technical solution of the present invention, and such modifications and changes are also included in the scope of the present invention.

The experimental methods used in the following examples are conventional methods, unless otherwise specified. The materials, reagents, and the like used in the following examples are all commercially available, unless otherwise specified.

**Table 1: Strains and plasmids used in the present invention**

| Strain | Relevant characteristics | Source |
|---|---|---|
| ATCC 8739 | Wild-type strain | Wild-type |
| M1-93 | ATCC 8739, FRT-Km-FRT::M1-93::lacZ | Lu, et al., Appl Microbiol Biotechnol, 2012,93:2455-2462 |
| M1-46 | ATCC 8739, FRT-Km-FRT::M1-46::lacZ | Lu, et al., Appl Microbiol Biotechnol, 2012,93:2455-2462 |
| Span001 | ATCC 8739, tdcDE::cat-sacB | Constructed in the present invention |
| Span002 | Span001, tdcDE::alsS | Constructed in the present invention |
| Span003 | Span002, tdcDE::cat-sacB::alsS | Constructed in the present invention |
| Span004 | Span002, tdcDE::M1-93::alsS | Constructed in the present invention |
| Span005 | Span004, cat-sacB::ilvB | Constructed in the present invention |
| Span006 | Span004, M1-93:: ilvB | Constructed in the present invention |
| Span007 | Span006, cat-sacB::ilvG | Constructed in the present invention |
| Span008 | Span006, M1-93:: ilvG | Constructed in the present invention |
| Span009 | Span008, ilvH::cat-sacB | Constructed in the present invention |
| Span010 | Span008, ilvH:: ilvH* | Constructed in the present invention |
| Span011 | Span010, adhE::cat-sacB | Constructed in the present invention |
| Span012 | Span010, adhE::ilvC | Constructed in the present invention |
| Span013 | Span012, adhE::cat-sacB::ilvC | Constructed in the present invention |
| Span014 | Span012, adhE::M1-46::ilvC | Constructed in the present invention |
| Span015 | Span014, pflB::cat-sacB | Constructed in the present invention |
| Span016 | Span014, pflB:: ilvD | Constructed in the present invention |
| Span017 | Span016, pflB::cat-sacB:: ilvD | Constructed in the present invention |
| Span018 | Span016, pflB::RBSL1:: ilvD | Constructed in the present invention |
| Span019 | Span018, frd::cat-sacB | Constructed in the present invention |
| Span020 | Span018, frd::panB | Constructed in the present invention |
| Span021 | Span020, frd::cat-sacB::panB | Constructed in the present invention |
| Span022 | Span020, frd::M1-93::panB | Constructed in the present invention |
| Span023 | Span022, ldhA::cat-sacB | Constructed in the present invention |
| Span024 | Span022, ldhA::panE | Constructed in the present invention |
| Span025 | Span024, ldhA::cat-sacB::panE | Constructed in the present invention |
| Span026 | Span024, ldhA::RBSL2::panE | Constructed in the present invention |
| Span027 | Span026, mgsA::cat-sacB | Constructed in the present invention |
| Span028 | Span026, mgsA::glyA | Constructed in the present invention |
| Span029 | Span028, mgsA::cat-sacB::glyA | Constructed in the present invention |
| Span030 | Span028, mgsA::M1-46::glyA | Constructed in the present invention |
| Span031 | Span030, gcvTH::cat-sacB | Constructed in the present invention |
| Span032 | Span030, M1-93::gcvTH | Constructed in the present invention |
| Span033 | Span032, gcvP::cat-sacB | Constructed in the present invention |
| Span034 | Span032, M1-93::gcvP | Constructed in the present invention |
| Span035 | Span034, ackA-pta::cat-sacB | Constructed in the present invention |
| Span036 | Span034, ackA-pta::panB-C.glu | Constructed in the present invention |
| Span037 | Span036, ackA-pta::cat-sacB::panB-C.glu | Constructed in the present invention |
| Span038 | Span036, ackA-pta::M1-93::panB-C.glu | Constructed in the present invention |
| Span039 | Span038, ilvE::cat-sacB | Constructed in the present invention |
| Span040 | Span038, ilvE::ilvE*-GTG | Constructed in the present invention |
| Span041 | Span040, ara::cat-sacB | Constructed in the present invention |
| Span042 | Span040, ara::serA197 | Constructed in the present invention |
| Span043 | Span042, ara::cat-sacB::serA197 | Constructed in the present invention |
| Span044 | Span042, ara::Ml-93-serA197 | Constructed in the present invention |
| Span045 | Span044, avtA::cat-sacB | Constructed in the present invention |
| Span046 | Span044, avtA::serCB | Constructed in the present invention |
| Span047 | Span046, avtA::cat-sacB::serCB | Constructed in the present invention |
| Span048 | Span046, avtA::M1-93::serCB | Constructed in the present invention |
| Span049 | Span048, sdaA::cat-sacB | Constructed in the present invention |
| Span050 | Span048, ΔsdaA | Constructed in the present invention |
| Span096 | Span050, adhE: : RBSL5-kari | Constructed in the present invention |

| | | |
|---|---|---|
| Note: In Table 1, ATCC 8739, M1-93, and M1-46 are all *Escherichia coli* strains. | | |

**Table 2: Primers used in the present Invention**

| Primer name | Sequence | SEQ ID NO |
|---|---|---|
| tdcDE-ines-up | ccgtgattggtctgctgaccatcctgaacatcgtatacaaactgttttaaTGTGACG GAAGATCACTTCGCAG | 33 |
| tdcDE-ines-down | ataatgttctttgctacaggaaaatcaacaatatgcgcaccagatgccacTTATTTG TTAACTGTTAATTGTCCT | 34 |
| tdcDE-alsSin-up | ccgtgattggtctgctgaccatcctgaacatcgtatacaaactgttttaaATGTTGA CAAAAGCAACAAAAG | 35 |
| tdcDE-alsSin-down | ataatgttctttgctacaggaaaatcaacaatatgcgcaccagatgccacGCATGA GCTCCTAGAGAGCTTTCGTTTTCATG | 36 |
| XZ-tdcDE-up | TGATGAGCTACCTGGTATGGC | 37 |
| XZ-tdcDE-down | CGCCGACAGAGTAATAGGTTTTAC | 38 |
| alsSPro-CS-down | CCTCTGTTTTCACAAGGATTTTGTTCTTTGTTGC TTTTGTCAACATTTATTGTTAACTGTTAATTGTCCT | 39 |
| alsS-Pro-up | ccgtgattggtctgctgaccatcctgaacatcgtatacaaactgttttaaTTATCTCT GGCGGTGTTGAC | 40 |
| alsS-Pro-down | CCTCTGTTTTCACAAGGATTTTGTTCTTTGTTGC TTTTGTCAACATAGCTGTTTCCTGGTTTAAAC | 41 |
| tdcDE-YZ285-down | GCCTGTTGCCAAGTTAGAGG | 42 |
| ilvB pro-catup | ctgacgaaacctcgctccggcggggtttttgttatctgcaattcagtacTGTGACG GAAGATCACTTCGCA | 43 |
| ilvB pro-catdown | tctgcgccggtaaagcgcttacgcgtcgatgttgtgcccgaacttgccatTTATTTG TTAACTGTTAATTGTCCT | 44 |
| ilvB pro-up | ctgacgaaacctcgctccggcggggtttttgttatctgcaattcagtacTTATCTCT GGCGGTGTTGAC | 45 |
| ilvB pro-down | tctgcgccggtaaagcgcttacgcgtcgatgttgtgcccgaacttgccatAGCTGT TTCCTGGTTTAAAC | 46 |
| ilvB pro-YZup | gttctgcgcggaacacgtatac | 47 |
| ilvB pro-YZdown | ccgctacaggccatacagac | 48 |
| ilvG pro-catup | tgaactaagaggaagggaacaacattcagaccgaaattgaatttttttcaTGTGAC GGAAGATCACTTCGCA | 49 |
| ilvG pro-catdown | ttcacaccctgtgcccgcaacgcatgtaccacccactgtgcgccattcatTTATTTG TTAACTGTTAATTGTCCT | 50 |
| ilvG pro-up | tgaactaagaggaagggaacaacattcagaccgaaattgaatttttttcaTTATCTC TGGCGGTGTTGAC | 51 |
| ilvG pro-down | ttcacaccctgtgcccgcaacgcatgtaccacccactgtgcgccattcatAGCTGT TTCCTGGTTTAAACG | 52 |
| ilvG pro-YZup | gcataagatatcgctgctgtag | 53 |
| ilvG p-YZdown | gccagttttgccagtagcac | 54 |
| ilvH*-cat-up | agaacctgattatgCGCCGGATATTATCAGTCTTACTCGAAAA TGAATCATGTGACGGAAGATCACTTCGCA | 55 |
| ilvH*-cat-down | TTCATCGCCCACGGTCTGGATGGTCATACGCGATAATG TCGGATCGTCGGTTATTTGTTAACTGTTAATTGTCCT | 56 |
| ilvH*-mut-up | agaacctgattatgCGCCGGATATTATCAGTCTTACTCGAAAA TGAATCAGaCGCGTTATtCCGCGTGATTGGC | 57 |
| ilvH*-mut-down | CACACCAGAGCGAGCAACCTC | 58 |
| ilvH*-mutYZ-up | atgagctggaaagcaaacttagc | 59 |
| adhE-cs-up | ATAACTCTAATGTTTAAACTCTTTTAGTAAATCACAGT GAGTGTGAGCGCTGTGACGGAAGATCACTTCGCA | 60 |
| adhE-cs-down | CCGTTTATGTTGCCAGACAGCGCTACTGATTAAGCGGA TTTTTTCGCTTTTTATTTGTTAACTGTTAATTGTCCT | 61 |
| adhE-ilvC-up | ATAACTCTAATGTTTAAACTCTTTTAGTAAATCACAGT GAGTGTGAGCGCATGGCTAACTACTTCAATAC | 62 |
| adhE-ilvC-down | CCGTTTATGTTGCCAGACAGCGCTACTGATTAAGCGGA TTTTTTCGCTTTTTAACCCGCAACAGCAATACG | 63 |
| XZ-adhE-up | CATGCTAATGTAGCCACCAAA | 64 |
| XZ-adhE-down | TTGCACCACCATCCAGATAA | 65 |
| ilvC-ProCS-down | agctgtgccagctgctggcgcagattcagtGTATTGAAGTAGTTAGCCA TTTATTTGTTAACTGTTAATTGTCCT | 66 |
| ilvC-Pro-up | ATAACTCTAATGTTTAAACTCTTTTAGTAAATCACAGT GAGTGTGAGCGCTTATCTCTGGCGGTGTTGAC | 67 |
| ilvC-Pro-down | agctgtgccagctgctggcgcagattcagtGTATTGAAGTAGTTAGCCA TAGCTGTTTCCTGGTTTAAACCG | 68 |
| ilvC-YZ347-down | cgcactacatcagagtgctg | 69 |
| pflB-CS-up | aaacgaccaccattaatggttgtcgaagtacgcagtaaataaaaaatccaTGTGAC GGAAGATCACTTCGCAG | 70 |
| pflB-CS-down | CGGTCCGAACGGCGCGCCAGCACGACGACCGTCTGG GGTGTTACCCGTTTTTATTTGTTAACTGTTAATTGTCCT | 71 |
| pflB-ilvD-up | aaacgaccaccattaatggttgtcgaagtacgcagtaaataaaaaatccaatgcctaagt accgttccgc | 72 |
| pflB-ilvD-down | CGGTCCGAACGGCGCGCCAGCACGACGACCGTCTGG GGTGTTACCCGTTTttaaccccccagtttcgatttatc | 73 |
| XZ-pflB-up600 | CTGCGGAGCCGATCTCTTTAC | 74 |
| XZ-pflB-down | CGAGTAATAACGTCCTGCTGCT | 75 |
| pflB-Pcs-down | CCCGCCATATTACGACCATGAGTGGTGGCGGAAC GGTACTTAGGCATTTATTGTTAACTGTTAATTGTCCT | 76 |
| pflB-Pro-up | AAACGACCACCATTAATGGTTGTCGAAGTACGCAGTA AATAAAAAATCCATTATCTCTGGCGGTGTTGAC | 77 |
| ilvD-Pro-down | cccgccatattacgaccatgagtggtggtggcggaacggtacttaggcatTGCTG ACCTCCTGGTTTAAACGTACATG | 78 |
| ilvD-YZ496-down | caaccagatcgagcttgatg | 79 |
| XZ-frd-up | TGCAGAAAACCATCGACAAG | 80 |
| XZ-frd-down | CACCAATCAGCGTGACAACT | 81 |
| frd-cs-up | GAAGGCGAATGGCTGAGATGAAAAACCTGAAAATTG AGGTGGTGCGCTATTGTGACGGAAGATCACTTCGCA | 82 |
| frd-cs-down | TCTCAGGCTCCTTACCAGTACAGGGCAACAAACAGGA TTACGATGGTGGCTTATTTGTTAACTGTTAATTGTCCT | 83 |
| Frd-panB-up | GAAGGCGAATGGCTGAGATGAAAAACCTGAAAATTG AGGTGGTGCGCTATatgAAACCGACCACCATCTC | 84 |
| Frd-panB-down | TCTCAGGCTCCTTACCAGTACAGGGCAACAAACAGGA TTACGATGGTGGCttaATGGAAACTGTGTTCTTCGC | 85 |
| panB-Pcs-down | TTTTTTTCCTGTTTGTACTTCTGCAGTAAGGAGATGGT GGTCGGTTTcatTTATTTGTTAACTGTTAATTGTCCT | 86 |
| panB-Pro-up | GAAGGCGAATGGCTGAGATGAAAAACCTGAAAATTG AGGTGGTGCGCTATTTATCTCTGGCGGTGTTGAC | 87 |
| panB-Pro-down | TTTTTTTCCTGTTTGTACTTCTGCAGTAAGGAGATGGT GGTCGGTTTcatAGCTGTTTCCTGGTTTAAAC | 88 |
| panB-YZ130-down | CCACCAGCATGACGTTAAGC | 89 |
| ldhA-csin-up | attatatttgaaattttgtaaaatatttttagtagcttaaatgtgattcaTGTGACGGA AGATCACTTCGCAG | 90 |
| ldhA-csin-down | AACCAGTTCGTTCGGGCAGGTTTCGCCTTTTTCCAGA GCATGAGCTCCTaTTATTTGTTAACTGTTAATTGTCCT | 91 |
| ldhA-panE-up | attatatttgaaattttgtaaaatatttttagtagcttaaatgtgattcaatgAAAATTAC CGTATTGGG | 92 |
| ldhA-panE-down | AACCAGTTCGTTCGGGCAGGTTTCGCCTTTTTCCAGA GCATGAGCTCCTactaCCAGGGGCGAGGCAAAC | 93 |
| XZ-ldhA-up | GATAACGGAGATCGGGAATG | 94 |
| XZ-ldhA-down | CTTTGGCTGTCAGTTCACCA | 95 |
| panE-ProCS-down | GTAAGCCATAATTGCCCTAAGGCACCGCATCCCAATAC GGTAATTTTcatTTATTTGTTAACTGTTAATTGTCCT | 96 |
| panE-Pro-up | attatatttgaaattttgtaaaatatttttagtagcttaaatgtgattcaTTATCTCTGG CGGTGTTGAC | 97 |
| panE-Pro-down | GTAAGCCATAATTGCCCTAAGGCACCGCATCCCAATAC | 98 |
| | GGTAATTTTcatTCGAACCCTCCTGGTTTAAAC | |
| panE-YZ245-down | CTTTTGACGGCATCGGAAAC | 99 |
| mgsA-cs-up | gtaggaaagttaactacggatgtacattatggaactgacgactcgcacttTGTGAC GGAAGATCACTTCGCAG | 100 |
| mgsA-cs-down | gcgtttgccacctgtgcaatattacttcagacggtccgcgagataacgctTTATTTG TTAACTGTTAATTGTCCT | 101 |
| XZ-mgsA-up | cagctcatcaaccaggtcaa | 102 |
| XZ-mgsA-down | aaaagccgtcacgttattgg | 103 |
| mgsA-glyA-up | gtaggaaagttaactacggatgtacattatggaactgacgactcgcacttatgTTAAA GCGTGAAATGAAC | 104 |
| mgsA-glyA-down | gcgtttgccacctgtgcaatattacttcagacggtccgcgagataacgctttaTGCGT AAACCGGGTAAC | 105 |
| glyA-ProCS-down | TGCCACAGTTCGGCATCATAATCGGCAATGTTCATTTC ACGCTTTAAcatTTATTTGTTAACTGTTAATTGTCCT | 106 |
| glyA-Pro-up | gtaggaaagttaactacggatgtacattatggaactgacgactcgcacttTTATCTC TGGCGGTGTTGAC | 107 |
| glyA-Pro-down | TGCCACAGTTCGGCATCATAATCGGCAATGTTCATTTC ACGCTTTAAcatAGCTGTTTCCTGGTTTAAAC | 108 |
| glyA-YZ364-down | CCAGGTTCATACCCAGAACG | 109 |
| gcvT-Pcat-up | ttgatttagtgttttttgacatttttttagctcttaatattgtcttattcTGTGACGGAAG ATCACTTCGCA | 110 |
| gcvT-PsacB-down | cgagcgccgcaaagcgtgtgttgttcgtacaaaggagtctgttgtgccatTTATTTG TTAACTGTTAATTGTCCT | 111 |
| gcvT-M93-up | ttgatttagtgtttttgacatttttttagctcttaatattgtcttattcTTATCTCTGGC GGTGTTGAC | 112 |
| gcvT-M93-down | cgagcgccgcaaagcgtgtgttgttcgtacaaaggagtctgttgtgccatAGCTGT TTCCTGGTTTAAACG | 113 |
| gcvT-up-500 | ccaggcaatgggattaaacg | 114 |
| gcvT-350-down | gtggcggagttaacaacgag | 115 |
| gcvP-Pcat-up | aatcactgctggatgcgaccgcatacgaagcattgttagaagacgagtaaTGTGA CGGAAGATCACTTCGCA | 116 |
| gcvP-PsacB-down | cgttcaataaaagcgccgctgttttcaagctggcttaacgtctgtgtcatTTATTTGT TAACTGTTAATTGTCCT | 117 |
| gcvP-M93-up | aatcactgctggatgcgaccgcatacgaagcattgttagaagacgagtaaTTATCT CTGGCGGTGTTGAC | 118 |
| gcvP-M93-down | cgttcaataaaagcgccgctgttttcaagctggcttaacgtctgtgtcatAGCTGTT TCCTGGTTTAAACG | 119 |
| gcvH-up | atgagcaacgtaccagcagaac | 120 |
| gcvP-390-down | gaagttgagcagtgcttcaag | 121 |
| ackA-cs-up | aggtacttccatgtcgagtaagttagtactggttctgaactgcggtagttTGTGACG GAAGATCACTTCGCAG | 122 |
| pta-cs-down | ggtcggcagaacgctgtaccgctttgtaggtggtgttaccggtgttcagaTTATTTG TTAACTGTTAATTGTCCT | 123 |
| ackA-panBC-up | AGGTACTTCCATGTCGAGTAAGTTAGTACTGGTTCTGA ACTGCGGTAGTTatgcccatgtcaggcattgatg | 124 |
| ackA-panBC-down | GGTCGGCAGAACGCTGTACCGCTTTGTAGGTGGTGTT ACCGGTGTTCAGAttaaaaggactccgcttcgc | 125 |
| XZ-ackA-up | cgggacaacgttcaaaacat | 126 |
| XZ-pta-down | attgcccatcttcttgttgg | 127 |
| panBC-Pro-up | AGGTACTTCCATGTCGAGTAAGTTAGTACTGGTTCTGA ACTGCGGTAGTTTTATCTCTGGCGGTGTTGAC | 128 |
| panBC-Pro-down | cggaaatgacgggtgcggattttctttgcatcaatgcctgacatgggcatAGCTGT TTCCTGGTTTAAAC | 129 |
| panBC-ProCS-down | cggaaatgacgggtgcggattttctttgcatcaatgcctgacatgggcatTTATTTG TTAACTGTTAATTGTCCT | 130 |
| panBC-YZ425-down | accggaattccagcatcaac | 131 |
| ilvE-cat-up | cacaaccacatcacaacaaatccgcgcctgagcgcaaaaggaatataaaaTGTGA CGGAAGATCACTTCGCA | 132 |
| ilvE-sacB-down | cgaaccatctccccattgaaccaaatgtaatcagctttcttcgtggtcatTTATTTGT TAACTGTTAATTGTCCT | 133 |
| ilvEGTG-up | cacaaccacatcacaacaaatccgcgcctgagcgcaaaaggaatataaaaGtgacca cgaagaaagctgattac | 134 |
| ilvE-down | ttattgattaacttgatctaaccagcc | 135 |
| ilvM-up | atgatgcaacatcaggtcaatg | 136 |
| araBCD-CS-up | GCCGAAAACCCGAACGCGATGTTCGTATTGTGGAAAG ACCACACTGCGGTTGTGACGGAAGATCACTTCGCA | 137 |
| araBCD-CS-down | TCACGCATGTTATCGCCAAAACGGCAGACTTTCAGAT GACGGGTATCCTGTTATTTGTTAACTGTTAATTGTCCT | 138 |
| araBCD-serA197-up | GCCGAAAACCCGAACGCGATGTTCGTATTGTGGAAAG | 139 |
| | ACCACACTGCGGTATGAGCCAGAATGGCCGTCC | |
| araBCD-serA197-down | TCACGCATGTTATCGCCAAAACGGCAGACTTTCAGAT GACGGGTATCCTGTTAAGCCAGATCCATCCACAC | 140 |
| araBCD-YZ300-up | CACCAGCGTAGAGTGGTATC | 141 |
| araBCD-YZ468-down | CTGCAGACCGGTTGACATCAC | 142 |
| serA197-ProCS-down | TGCGCAAGCTTATCGGCGATGAGGACTACCGGACGGC CATTCTGGCTCATTTATTTGTTAACTGTTAATTGTCCT | 143 |
| serA197-Pro-up | GCCGAAAACCCGAACGCGATGTTCGTATTGTGGAAAG ACCACACTGCGGTTTATCTCTGGCGGTGTTGAC | 144 |
| serA197-Pro-down | TGCGCAAGCTTATCGGCGATGAGGACTACCGGACGGC CATTCTGGCTCATAGCTGTTTCCTGGTTTAAAC | 145 |
| SerA197-YZ358-down | TCTGGCGAGCAGTAGACAGC | 146 |
| avtA-CS-up | atgacattctcccttttggtgacaaatttacccgccactccggcattacTGTGACG GAAGATCACTTCGCA | 147 |
| avtA-CS-down | ttagtgactttcagcccaggctctttctatctcttccgccagaatcttcaTTATTTGTT AACTGTTAATTGTCCT | 148 |
| serC-down | GCACCAGGTAATGTTAGGCATGTTTGTTCTCCTTTTGT CGACTTAACCGTGACGGCGTTCGAAC | 149 |
| serB-up | GTTCGAACGCCGTCACGGTTAAGTCGACAAAAGGAG AACAAACATGCCTAACATTACCTGGTGC | 150 |
| avtA-serCB-up | gatatcccgctatgacattctcccttttggtgacaaatttacccgccacATGGCTCA AATCTTCAATTTTAG | 151 |
| avtA-serCB-down | ttagtgactttcagcccaggctctttctatctcttccgccagaatcttcaTTACTTCT GATTCAGGCTGCCTG | 152 |
| avtA-YZ-up | gttcggatatgaactggcagg | 153 |
| avtA-YZ-down | caaacacgttgcattggctg | 154 |
| serCB-ProCS-down | TCTGCCGGTAGCATTGCCGGACCAGAACTAAAATTGA AGATTTGAGCCATTTATTTGTTAACTGTTAATTGTCCT | 155 |
| serCB-YZ317-down | CAGAGAGTTGCCATTCACGC | 156 |
| serCB-Pro-up | gatatcccgctatgacattctcccttttggtgacaaatttacccgccacTTATCTCT GGCGGTGTTGAC | 157 |
| serCB-Pro-down | TCTGCCGGTAGCATTGCCGGACCAGAACTAAAATTGA AGATTTGAGCCATAGCTGTTTCCTGGTTTAAAC | 158 |
| sdaA-delcat-up | tgttattagttcgttactggaagtccagtcaccttgtcaggagtattatcTGTGACGG AAGATCACTTCGCA | 159 |
| sdaA-delsacB-down | aaagcgggtataaattcgcccatccgttgcagatgggcgagtaagaagtaTTATTT GTTAACTGTTAATTGTCCT | 160 |
| SdaAdel-down | aagcgggtataaattcgcccatccgttgcagatgggcgagtaagaagtagataatactc ctgacaaggtg | 161 |
| sdaA-YZ-up | ccagtgaagatgaagtctcg | 162 |
| sdaA-YZ-down | atggatcgcacagtttggag | 163 |
| adhE-del-down | CCGTTTATGTTGCCAGACAGCGCTACTGATTAAGCGGA TTTTTTCGCTTTGCGCTCACACTCACTGTGATTTAC | 164 |
| adhE-RBS5-up | ATAACTCTAATGTTTAAACTCTTTTAGTAAATCACAGT GAGTGTGAGCGCTTATCTCTGGCGGTGTTGAC | 165 |
| adhE-kari-down | CCGTTTATGTTGCCAGACAGCGCTACTGATTAAGCGGA TTTTTTCGCTTTTTAGATAACTTTTTTCTTCA | 166 |

In the following text, the two primers separated by "/" form a corresponding primer pair for amplifying target fragments.

### Example 1: Insertion of acetolactate synthase gene alsS at the loci of propionate kinase-encoding gene tdcD and formate acetyltransferase-encoding gene tdcE and knockout of the tdcDE operon in strain ATCC 8739

Starting from *Escherichia coli* ATCC 8739, the acetolactate synthase gene *alsS* derived from *Bacillus subtilis* 168 (from ATCC, No. 23857) was inserted into the loci of propionate kinase-encoding gene *tdcD* and formate acetyltransferase-encoding gene *tdcE* on the chromosome using a two-step homologous recombination method. The specific steps were as follows:
In the first step, using plasmid DNA of pXZ-CS (Tan, et al., Appl Environ Microbiol, 2013, 79:4838-4844) as a template, a 2719 bp DNA fragment I was amplified with primers tdcDE-incs-up/tdcDE-incs-down. This fragment included a 50 bp upstream homology arm of tdcDE, a 2619 bp cat-sacB DNA fragment containing the chloramphenicol resistance gene (cat) and the levansucrase gene (sacB), and a 50 bp downstream homology arm of tdcDE, which was used for the first homologous recombination.

The amplification system was as follows: 10 µl of Phusion 5X buffer (New England Biolabs), 1 µl of dNTP (each dNTP at 10 mM), 20 ng of DNA template, 2 µl of each primer (10 µM), 0.5 µl of Phusion High-Fidelity DNA polymerase (2.5 U/µl), and 33.5 µl of distilled water, with a total volume of 50 µl.

The amplification conditions were as follows: pre-denaturation at 98°C for 2 minutes (1 cycle); denaturation at 98°C for 10 seconds, annealing at 56°C for 10 seconds, extension at 72°C for 2 minutes (30 cycles); and extension at 72°C for 10 minutes (1 cycle).

The above DNA fragment I was used for the first homologous recombination: first, the pKD46 plasmid (from CGSC *Escherichia coli* Stock Center, Yale University, USA, CGSC#7739) was transformed into *Escherichia coli* ATCC 8739 by electroporation, and then the DNA fragment I was electroporated into *Escherichia coli* ATCC 8739 harboring pKD46.

The electroporation conditions were as follows: first, electrocompetent cells of *Escherichia coli* ATCC 8739 harboring the pKD46 plasmid were prepared (preparation method according to Dower et al., 1988, Nucleic Acids Res 16:6127-6145); 50 µl of the competent cells were placed on ice, 50 ng of the DNA fragment I was added, and the mixture was left on ice for 2 minutes, and then transferred to a 0.2 cm Bio-Rad electroporation cuvette. A MicroPulser electroporator (Bio-Rad) was used with an electric shock parameter of 2.5 kV. Immediately after the electric shock, 1 ml of LB medium was transferred into the electroporation cuvette, pipetted 5 times, then transferred to a test tube, and incubated at 30°C for 2 hours with shaking at 75 rpm. 200 µl of the bacterial solution was taken and spread on an LB plate containing ampicillin (final concentration 100 µg/ml) and chloramphenicol (final concentration 34 µg/ml). After overnight culture at 30°C, single colonies were selected for PCR verification using primers XZ-tdcDE-up/XZ-tdcDE-down. The correct colony amplification product was a 3615 bp fragment, which included an 845 bp upstream homology arm of tdcDE, a 2619 bp cat-sacB fragment, and a 151 bp downstream homology arm of tdcDE. A correct single colony was selected and named Span001.

In the second step, using genomic DNA of wild-type *Bacillus subtilis* 168 as a template, a 1826 bp DNA fragment II was amplified with primers tdcDE-alsSin-up/tdcDE-alsSin-down, which included a 50 bp upstream homology arm of tdcDE, a 1716 bp alsS gene, a total of 10 bp consisting of the sacI restriction site and protective bases, and a 50 bp downstream homology arm of tdcDE. The DNA fragment II was used for the second homologous recombination. The amplification conditions and system were the same as those described in the first step. The DNA fragment II was electroporated into the strain Span001.

The electroporation conditions were as follows: first, electrocompetent cells of Span001 harboring the pKD46 plasmid were prepared; 50 µl of the competent cells were placed on ice, 50 ng of the DNA fragment II was added, and the mixture was left on ice for 2 minutes, and then transferred to a 0.2 cm Bio-Rad electroporation cuvette. A MicroPulser electroporator (Bio-Rad) was used with an electric shock parameter of 2.5 kV. Immediately after the electric shock, 1 ml of LB medium was transferred into the electroporation cuvette, pipetted 5 times, then transferred to a test tube, and incubated at 30°C for 4 hours with shaking at 75 rpm. The bacterial solution was transferred to LB liquid medium containing 10% sucrose and no sodium chloride (50 ml medium in a 250 ml flask). After 24 hours of culture, streaking and culture were performed on LB solid medium containing 6% sucrose and no sodium chloride. After PCR verification using primers XZ-tdcDE-up/XZ-tdcDE-down, the correct colony amplification product was a 2722 bp fragment, which included an 845 bp upstream homology arm of tdcDE, a total of 1726 bp consisting of the alsS gene and sacI restriction site, and a 151 bp downstream homology arm of tdcDE. A correct single colony was selected and named Span002.

Span002 is a recombinant strain obtained by integrating the acetolactate synthase gene (*alsS* gene, whose nucleotide sequence is SEQ ID NO: 2 in the sequence listing and encodes the alsS protein shown in SEQ ID NO: 1) into the loci of the propionate kinase-encoding gene *tdcD* and formate acetyltransferase-encoding gene *tdcE* of *Escherichia coli* ATCC 8739. In this recombinant strain, both the propionate kinase-encoding gene *tdcD* (encoding the protein sequence NCBI ACA76259.1, coded_by=CP000946.1:626900..628108) and the formate acetyltransferase-encoding gene *tdcE* (encoding the protein sequence NCBI ACA76260.1, coded_by=CP000946.1:628142..630436) were knocked out.

### Example 2: Regulation of acetolactate synthase gene alsS

Starting from Span002, the expression of the acetolactate synthase-encoding gene alsS integrated at the tdcDE locus was regulated using artificial regulatory elements. The specific steps were as follows:
In the first step, using pXZ-CS plasmid DNA as a template, a 2719 bp DNA fragment I was amplified with primers tdcDE-incs-up/alsSPro-CS-down, which included a 50 bp upstream homology arm of tdcDE, a 2619 bp cat-sacB fragment, and a 50 bp downstream homology arm of the alsS gene, and was used for the first homologous recombination. The amplification system and amplification conditions were consistent with those described in Example 1. The DNA fragment I was electroporated into Span002.

The DNA fragment I was used for the first homologous recombination: first, the pKD46 plasmid was transformed into *Escherichia coli* Span002 by electroporation, and then the DNA fragment I was electroporated into *Escherichia coli* Span002 harboring pKD46.

The electroporation conditions and steps were consistent with those of the first-step method described in Example 1 for the integration of *alsS* at the *tdcDE* locus. 200 µl of the bacterial solution was taken and spread on an LB plate containing ampicillin (final concentration 100 µg/ml) and chloramphenicol (final concentration 34 µg/ml). After overnight incubation at 30°C, single colonies were selected for PCR verification using primers XZ-tdcDE-up/tdcDE-YZ285-down. The correct PCR product should be 3749 bp, which includes an 845 bp upstream homology arm of tdcDE, a 2619 bp cat-sacB fragment, and a 285 bp downstream homology arm of alsS. A correct single colony was selected and named Span003.

In the second step, using genomic DNA of M1-93 (Lu, et al., Appl Microbiol Biotechnol, 2012, 93: 2455-2462) as a template, an 188 bp DNA fragment II was amplified with primers alsS-Pro-up/alsS-Pro-down, which included a 50 bp upstream homology arm of tdcDE, an 88 bp M1-93 promoter, and a 50 bp downstream homology arm of alsS. The DNA fragment II was used for the second homologous recombination. The DNA fragment II was electroporated into the strain Span003.

The electroporation conditions and steps were consistent with those of the second-step method described in Example 1 for the integration of *alsS* at the *tdcDE* locus. Colony PCR was performed to verify the clones using primers XZ-tdcDE-up/tdcDE-YZ285-down. The correct colony amplification product was a 1218 bp fragment, which included an 8454 bp upstream homology arm of tdcDE, an 88 bp M1-93 promoter sequence, and a 285 bp downstream homology arm of alsS. A correct single colony was selected and named Span004.

Span004 is a recombinant strain obtained by integrating the M1-93 promoter (whose nucleotide sequence is SEQ ID NO: 169 in the sequence listing) upstream of the *alsS* gene in *Escherichia coli* Span002. In this recombinant strain, the M1-93 promoter drives the expression of the *alsS* gene.

### Example 3: Regulation of acetolactate synthase gene ilvB

The expression of the acetolactate synthase I large subunit gene ilvB was regulated using the artificial regulatory element M1-93 through a two-step homologous recombination method. The specific steps were as follows:
In the first step, using pXZ-CS plasmid DNA as a template, a 2719 bp DNA fragment I was amplified with primers ilvB pro-catup/ilvB pro-catdown, which was used for the first homologous recombination. The DNA fragment I included a 50 bp upstream homology arm of ilvB, a 2619 bp cat-sacB fragment, and a 50 bp downstream homology arm of ilvB. The amplification system and amplification conditions were consistent with those described in Example 1.

The DNA fragment I was used for the first homologous recombination: first, the pKD46 plasmid was transformed into *Escherichia coli* Span004 by electroporation, and then the DNA fragment I was electroporated into *Escherichia coli* Span004 harboring pKD46.

The electroporation conditions and steps were consistent with those of the first-step method described in Example 1 for the integration of alsS at the tdcDE locus. 200 µl of the bacterial solution was taken and spread on an LB plate containing ampicillin (final concentration 100 µg/ml) and chloramphenicol (final concentration 34 µg/ml). After overnight incubation at 30°C, single colonies were selected for PCR verification using primers ilvB pro-YZup/ilvB pro-YZdown. The correct PCR product should be 2996 bp, which includes a 123 bp upstream homology arm of ilvB, a 2619 bp cat-sacB fragment, and a 254 bp downstream homology arm of ilvB. A correct single colony was selected and named Span005.

In the second step, using genomic DNA of M1-93 (Lu, et al., Appl Microbiol Biotechnol, 2012, 93: 2455-2462) as a template, an 188 bp DNA fragment II was amplified with primers ilvB pro-up/ilvB pro-down. The DNA fragment II included a 50 bp upstream homology arm of ilvB, an 88 bp M1-93 promoter, and a 50 bp downstream homology arm of ilvB. The DNA fragment II was used for the second homologous recombination. The DNA fragment II was electroporated into the strain Span005.

The electroporation conditions and steps were consistent with those of the second-step method described in Example 1 for the integration of alsS at the tdcDE locus. Colony PCR was performed to verify the clones using primers ilvB pro-YZup/ilvB pro-YZdown. The correct colony amplification product was a 465 bp fragment, which included a 123 bp upstream homology arm of ilvB, an 88 bp M1-93 promoter, and a 254 bp downstream homology arm of ilvB. A correct single colony was selected and named Span006.

Span006 is a recombinant strain obtained by integrating the M1-93 promoter (whose nucleotide sequence is SEQ ID NO: 169 in the sequence listing) upstream of the acetolactate synthase gene ilvB (encoding the protein sequence shown in SEQ ID NO: 3, NCBI ACA75715.1, coded_by=CP000946.1:28583..30271) in *Escherichia coli* Span004. In this recombinant strain, the M1-93 promoter can drive the expression of the ilvB gene.

### Example 4: Regulation of acetolactate synthase gene ilvG

The expression of the acetolactate synthase II large subunit gene ilvG was regulated using the artificial regulatory element M1-93 through a two-step homologous recombination method. The specific steps were as follows:
In the first step, using pXZ-CS plasmid DNA as a template, a 2719 bp DNA fragment I was amplified with primers ilvG pro-catup/ilvG pro-catdown, which was used for the first homologous recombination. The DNA fragment I included a 50 bp upstream homology arm of ilvG, a 2619 bp cat-sacB fragment, and a 50 bp downstream homology arm of ilvG. The amplification system and amplification conditions were consistent with those described in Example 1.

The DNA fragment I was used for the first homologous recombination: first, the pKD46 plasmid was transformed into *Escherichia coli* Span006 by electroporation, and then the DNA fragment I was electroporated into *Escherichia coli* Span006 harboring pKD46.

The electroporation conditions and steps were consistent with those of the first-step method described in Example 1 for the integration of alsS at the tdcDE locus. 200 µl of the bacterial solution was taken and spread on an LB plate containing ampicillin (final concentration 100 µg/ml) and chloramphenicol (final concentration 34 µg/ml). After overnight incubation at 30°C, single colonies were selected for PCR verification using primers ilvG pro-YZup/ilvG p-YZdown. The correct PCR product should be 2993 bp, which includes a 179 bp upstream homology arm of ilvG, a 2619 bp cat-sacB fragment, and a 195 bp downstream homology arm of ilvG. A correct single colony was selected and named Span007.

In the second step, using the genomic DNA/plasmid DNA of M1-93 (Lu, et al., Appl Microbiol Biotechnol, 2012, 93: 2455-2462) as a template, an 188 bp DNA fragment II was amplified with primers ilvG pro-up/ilvG pro-down. The DNA fragment II included a 50 bp upstream homology arm of ilvG, an 88 bp M1-93 promoter, and a 50 bp downstream homology arm of ilvG. The DNA fragment II was used for the second homologous recombination. The DNA fragment II was electroporated into the strain Span007.

The electroporation conditions and steps were consistent with those of the second-step method described in Example 1 for the integration of alsS at the tdcDE locus. Colony PCR was performed to verify the clones using primers ilvG pro-YZup/ilvG p-YZdown. The correct colony amplification product was a 462 bp fragment, which included a 179 bp upstream homology arm of ilvG, an 88 bp M1-93 fragment, and a 195 bp downstream homology arm of ilvG. A correct single colony was selected and named Span008.

Span008 is a recombinant strain obtained by integrating the M1-93 promoter (whose nucleotide sequence is SEQ ID NO: 169 in the sequence listing) upstream of the acetolactate synthase gene ilvG (encoding the protein sequence shown in SEQ ID NO: 5, NCBI ACA79830.1, coded_by=CP000946.1:4677780..4679426) in *Escherichia coli* Span006. In this recombinant strain, the M1-93 promoter can drive the expression of the ilvG gene.

### Example 5: Mutation of acetolactate synthase gene ilvH

A mutation was introduced into the acetolactate synthase III regulatory subunit gene ilvH through a two-step homologous recombination method to relieve feedback inhibition by L-valine. The specific steps were as follows:
In the first step, using pXZ-CS plasmid DNA as a template, a 2719 bp DNA fragment I was amplified with primers ilvH*-cat-up/ilvH*-cat-down, which was used for the first homologous recombination. The DNA fragment I included a 50 bp upstream homology arm of ilvH, a 2619 bp cat-sacB fragment, and a 50 bp downstream homology arm of ilvH. The amplification system and amplification conditions were consistent with those described in Example 1.

The DNA fragment I was used for the first homologous recombination: first, the pKD46 plasmid was transformed into *Escherichia coli* Span008 by electroporation, and then the DNA fragment I was electroporated into *Escherichia coli* Span008 harboring pKD46.

The electroporation conditions and steps were consistent with those of the first-step method described in Example 1 for the integration of alsS at the tdcDE locus. 200 µl of the bacterial solution was taken and spread on an LB plate containing ampicillin (final concentration 100 µg/ml) and chloramphenicol (final concentration 34 µg/ml). After overnight incubation at 30°C, single colonies were selected for PCR verification using primers ilvH*-mutYZ-up/ilvH*-mut-down. The correct PCR product should be 3165 bp, which includes a 202 bp upstream homology arm of ilvH, a 2619 bp cat-sacB fragment, and a 344 bp downstream homology arm of ilvH. A correct single colony was selected and named Span009.

In the second step, using DNA from wild-type *Escherichia coli* ATCC 8739 as a template, a 467 bp DNA fragment II was amplified with primers ilvH*-mut-up/ilvH*-mut-down. The DNA fragment II was the ilvH gene containing the mutation. The DNA fragment II was used for the second homologous recombination. The DNA fragment II was electroporated into the strain Span009.

The electroporation conditions and steps were consistent with those of the second-step method described in Example 1 for the integration of alsS at the tdcDE locus. Colony PCR was performed to verify the clones using primers ilvH*-mutYZ-up/ilvH*-mut-down. The correct colony amplification product was a 619 bp fragment, which included 163 bp upstream of the ilvH gene and 456 bp of the ilvH gene. A correct single colony was selected and named Span010.

Span010 is a recombinant strain obtained by mutating the acetolactate synthase gene ilvH of *Escherichia coli* Span008 into the ilvH_{*} gene (i.e., the mutated ilvH gene). In this recombinant strain, the sequence of the ilvH_{*} gene is SEQ ID NO: 8 in the sequence listing, which encodes the ilvH_{*} protein shown in SEQ ID NO: 7.

### Example 6: Integration of acetohydroxy acid reductoisomerase-encoding gene ilvC at the alcohol dehydrogenase adhE locus and knockout of the adhE gene

Starting from Span010, the acetohydroxy acid reductoisomerase-encoding gene ilvC derived from *Escherichia coli* was integrated into the alcohol dehydrogenase adhE locus through a two-step homologous recombination method. The specific steps were as follows:
In the first step, using pXZ-CS plasmid DNA as a template, a 2719 bp DNA fragment I was amplified with primers adhE-CS-up/adhE-CS-down, which was used for the first homologous recombination. The DNA fragment I included a 50 bp upstream homology arm of adhE, a 2619 bp cat-sacB fragment, and a 50 bp downstream homology arm of adhE. The amplification system and amplification conditions were consistent with those described in Example 1. The DNA fragment I was electroporated into Span010.

The DNA fragment I was used for the first homologous recombination: first, the pKD46 plasmid (Datsenko and Wanner 2000, Proc Natl Acad Sci USA 97:6640-6645; the plasmid was purchased from the CGSC *Escherichia coli* Stock Center at Yale University, USA, CGSC#7739) was transformed into *Escherichia coli* Span010 by electroporation, and then the DNA fragment I was electroporated into *Escherichia coli* Span010 harboring pKD46.

The electroporation conditions and steps were consistent with those of the first-step method described in Example 1 for the integration of alsS at the tdcDE locus. 200 µl of the bacterial solution was taken and spread on an LB plate containing ampicillin (final concentration 100 µg/ml) and chloramphenicol (final concentration 34 µg/ml). After overnight culture at 30°C, single colonies were selected for PCR verification using primers XZ-adhE-up/XZ-adhE-down. The correct PCR product should be 3167 bp, which includes a 221 bp upstream homology arm of adhE, a 2619 bp cat-sacB fragment, and a 327 bp downstream homology arm of adhE. A correct single colony was selected and named Span011.

In the second step, using genomic DNA of wild-type *Escherichia coli* ATCC 8739 as a template, a 1576 bp DNA fragment II was amplified with primers adhE-ilvC-up/adhE-ilvC-down. The DNA fragment II included a 50 bp upstream homology arm of adhE, a 1476 bp ilvC gene, and a 50 bp downstream homology arm of adhE. The DNA fragment II was used for the second homologous recombination. The DNA fragment II was electroporated into the strain Span011.

The electroporation conditions and steps were consistent with those of the second-step method described in Example 1 for the integration of alsS at the tdcDE locus. Colony PCR was performed to verify the clones using primers XZ-adhE-up/XZ-adhE-down. The correct colony amplification product was a 2024 bp fragment, which included a 221 bp upstream homology arm of adhE, a 1476 bp ilvC gene, and a 327 bp downstream homology arm of adhE. A correct single colony was selected and named Span012.

Span012 is a recombinant strain obtained by integrating the acetohydroxy acid reductoisomerase-encoding gene ilvC (encoding the protein sequence shown in SEQ ID NO: 167, NCBI ACA79824.1, coded_by=CP000946.1:4670539..4672014) into the adhE locus of *Escherichia coli* Span010. In this recombinant strain, the alcohol dehydrogenase gene adhE (encoding the protein sequence NCBI ACA78022.1, coded_by=CP000946.1:2627307..2629982) is simultaneously knocked out.

### Example 7: Regulation of acetohydroxy acid reductoisomerase-encoding gene ilvC

Starting from Span012, the expression of the acetohydroxy acid reductoisomerase-encoding gene ilvC integrated at the alcohol dehydrogenase gene adhE locus was regulated using artificial regulatory elements. The specific steps were as follows:
In the first step, using pXZ-CS plasmid (Tan, et al., Appl Environ Microbiol, 2013, 79:4838-4844) DNA as a template, a 2719 bp DNA fragment I was amplified with primers adhE-cs-up/ilvC-ProCS-down, which was used for the first homologous recombination. The DNA fragment I included a 50 bp upstream homology arm of adhE, a 2619 bp cat-sacB fragment, and a 50 bp downstream homology arm of ilvC. The amplification system and amplification conditions were consistent with those described in Example 1. The DNA fragment I was electroporated into Span012.

The DNA fragment I was used for the first homologous recombination: first, the pKD46 plasmid (Datsenko and Wanner 2000, Proc Natl Acad Sci USA 97:6640-6645; the plasmid was purchased from the CGSC *Escherichia coli* Stock Center at Yale University, USA, CGSC#7739) was transformed into *Escherichia coli* Span012 by electroporation, and then the DNA fragment I was electroporated into *Escherichia coli* Span012 harboring pKD46.

The electroporation conditions and steps were consistent with those of the first-step method described in Example 1 for the integration of alsS at the tdcDE locus. 200 µl of the bacterial solution was taken and spread on an LB plate containing ampicillin (final concentration 100 µg/ml) and chloramphenicol (final concentration 34 µg/ml). After overnight culture at 30°C, single colonies were selected for PCR verification using primers XZ-adhE-up/ilvC-YZ347-down. The correct PCR product should be 3187 bp, which includes a 221 bp upstream homology arm of adhE, a 2619 bp cat-sacB fragment, and a 347 bp downstream homology arm of ilvC. A correct single colony was selected and named Span013.

In the second step, using genomic DNA of M1-46 (Lu, et al., Appl Microbiol Biotechnol, 2012, 93: 2455-2462) as a template, an 188 bp DNA fragment II was amplified with primers ilvC-Pro-up/ilvC-Pro-down. The DNA fragment II included a 50 bp upstream homology arm of adhE, an 88 bp M1-46 promoter sequence, and a 50 bp downstream homology arm of ilvC. The DNA fragment II was used for the second homologous recombination. The DNA fragment II was electroporated into the strain Span013.

The electroporation conditions and steps were consistent with those of the second-step method described in Example 1 for the integration of alsS at the tdcDE locus. Colony PCR was performed to verify the clones using primers XZ-adhE-up/ilvC-YZ347-down. The correct colony amplification product was a 656 bp fragment, which included a 221 bp upstream homology arm of adhE, an 88 bp M1-46 promoter, and a 347 bp downstream homology arm of ilvC. A correct single colony was selected and named Span014.

Span014 is a recombinant strain obtained by integrating the M1-46 promoter (whose nucleotide sequence is SEQ ID NO: 173 in the sequence listing) upstream of the ilvC gene in *Escherichia coli* Span012. In this recombinant strain, the M1-46 promoter drives the expression of the ilvC gene.

### Example 8: Integration of dihydroxy acid dehydratase-encoding gene ilvD at the

### pyruvate formate lyase-encoding gene pflB locus and knockout of the pflB gene

Starting from Span014, the dihydroxy acid dehydratase-encoding gene ilvD derived from *Escherichia coli* was integrated into the pyruvate formate lyase-encoding gene pflB locus and the pflB gene was knocked out through a two-step homologous recombination method. The specific steps were as follows:
In the first step, using pXZ-CS plasmid DNA as a template, a 2719 bp DNA fragment I was amplified with primers pflB-CS-up/pflB-CS-down, which was used for the first homologous recombination. The DNA fragment I included a 50 bp upstream homology arm of pflB, a 2619 bp cat-sacB fragment, and a 50 bp downstream homology arm of pflB. The amplification system and amplification conditions were consistent with those described in Example 1. The DNA fragment I was electroporated into Span014.

The DNA fragment I was used for the first homologous recombination: first, the pKD46 plasmid was transformed into *Escherichia coli* Span014 by electroporation, and then the DNA fragment I was electroporated into *Escherichia coli* Span014 harboring pKD46.

The electroporation conditions and steps were consistent with those of the first-step method described in Example 1 for the integration of alsS at the tdcDE locus. 200 µl of the bacterial solution was taken and spread on an LB plate containing ampicillin (final concentration 100 µg/ml) and chloramphenicol (final concentration 34 µg/ml). After overnight incubation at 30°C, single colonies were selected for PCR verification using primers XZ-pflB-up600/XZ-pflB-down. The correct PCR product should be 3675 bp, which includes a 641 bp upstream homology arm of pflB, a 2619 bp cat-sacB fragment, and a 415 bp downstream homology arm of pflB. A correct single colony was selected and named Span015.

In the second step, using the gene of *Escherichia coli* MG1655 (from ATCC, No. 700926) as a template, a 1951 bp DNA fragment I was amplified with primers pflB-ilvD-up/pflB-ilvD-down. The DNA fragment II included a 50 bp upstream homology arm of pflB, a 1851 bp ilvD gene, and a 50 bp downstream homology arm of pflB. The DNA fragment II was used for the second homologous recombination. The DNA fragment II was electroporated into the strain Span015.

The electroporation conditions and steps were consistent with those of the second-step method described in Example 1 for the integration of alsS at the tdcDE locus. Colony PCR was performed to verify the clones using primers XZ-pflB-up600/XZ-pflB-down. The correct colony amplification product was a 2996 bp fragment, which included a 641 bp upstream homology arm of pflB, a 1851 bp ilvD gene, and a 415 bp downstream homology arm of pflB. A correct single colony was selected and named Span016.

Span016 is a recombinant strain obtained by integrating the dihydroxy acid dehydratase-encoding gene ilvD (SEQ ID NO: 12) (encoding the protein sequence shown in SEQ ID NO: 11, NCBI QPA17447.1, coded_by=CP032679.1:3943375..3945225) into the pflB locus of *Escherichia coli* Span014. In this recombinant strain, the pyruvate formate lyase-encoding gene pflB (encoding the protein sequence NCBI ACA78322.1, coded_by=CP000946.1:2956804..2959086) is simultaneously knocked out.

### Example 9: Expression regulation of dihydroxy acid dehydratase-encoding gene ilvD

Starting from Span016, the expression of the dihydroxy acid dehydratase-encoding gene ilvD integrated at the pyruvate formate lyase-encoding gene pflB locus was regulated using artificial regulatory elements. The specific steps were as follows:
In the first step, using pXZ-CS plasmid DNA as a template, a 2719 bp DNA fragment I was amplified with primers pflB-CS-up/pflB-Pcs-down, which was used for the first homologous recombination. The DNA fragment I included a 50 bp upstream homology arm of pflB, a 2619 bp cat-sacB fragment, and a 50 bp downstream homology arm of ilvD. The amplification system and amplification conditions were consistent with those described in Example 1. The DNA fragment I was electroporated into Span016.

The DNA fragment I was used for the first homologous recombination: first, the pKD46 plasmid was transformed into *Escherichia coli* Span016 by electroporation, and then the DNA fragment I was electroporated into *Escherichia coli* Span016 harboring pKD46.

The electroporation conditions and steps were consistent with those of the first-step method described in Example 1 for the integration of alsS at the tdcDE locus. 200 µl of the bacterial solution was taken and spread on an LB plate containing ampicillin (final concentration 100 µg/ml) and chloramphenicol (final concentration 34 µg/ml). After overnight incubation at 30°C, single colonies were selected for PCR verification using primers XZ-pflB-up600/ilvD-YZ496-down. The correct PCR product should be 3756 bp, which includes a 641 bp upstream homology arm of pflB, a 2619 bp cat-sacB fragment, and a 496 bp downstream homology arm of ilvD. A correct single colony was selected and named Span017.

In the second step, using genomic DNA of M1-93 (Lu, et al., Appl Microbiol Biotechnol, 2012, 93: 2455-2462) as a template, an 189 bp DNA fragment II was amplified with primers pflB-Pro-up/ilvD-Pro-down. The DNA fragment II included a 50 bp upstream homology arm of pflB, an 89 bp artificial regulatory element RBSL1 sequence, and a 50 bp downstream homology arm of ilvD. The DNA fragment II was used for the second homologous recombination. The DNA fragment II was electroporated into the strain Span017.

The electroporation conditions and steps were consistent with those of the second-step method described in Example 1 for the integration of alsS at the tdcDE locus. Colony PCR was performed to verify the clones using primers XZ-pflB-up600/ilvD-YZ496-down. The correct colony amplification product was a 1226 bp fragment, which included a 641 bp upstream homology arm of pflB, an 89 bp RBSL1 sequence, and a 496 bp downstream homology arm of ilvD. A correct single colony was selected and named Span018.

Span018 is a recombinant strain obtained by integrating the RBSL1 promoter (whose nucleotide sequence is SEQ ID NO: 171 in the sequence listing) upstream of the ilvD gene in *Escherichia coli* Span016. In this recombinant strain, the RBSL1 promoter drives the expression of the ilvD gene.

### Example 10: Integration of 3-methyl-2-oxobutanoate hydroxymethyltransferase gene panB at the fumarate reductase-encoding gene frd locus and knockout of the frd gene

Starting from Span018, the 3-methyl-2-oxobutanoate hydroxymethyltransferase-encoding gene panB was integrated into the fumarate reductase-encoding gene frd locus. The specific steps were as follows:
In the first step, using pXZ-CS plasmid DNA as a template, a 2719 bp DNA fragment I was amplified with primers frd-cs-up/frd-cs-down, which was used for the first homologous recombination. The DNA fragment I included a 50 bp upstream homology arm of frd, a 2619 bp cat-sacB fragment, and a 50 bp downstream homology arm of frd. The amplification system and amplification conditions were consistent with those described in Example 1. The DNA fragment I was electroporated into Span018.

The DNA fragment I was used for the first homologous recombination: first, the pKD46 plasmid was transformed into *Escherichia coli* Span018 by electroporation, and then the DNA fragment I was electroporated into *Escherichia coli* Span018 harboring pKD46.

The electroporation conditions and steps were consistent with those of the first-step method described in Example 1 for the integration of alsS at the tdcDE locus. 200 µl of the bacterial solution was taken and spread on an LB plate containing ampicillin (final concentration 100 µg/ml) and chloramphenicol (final concentration 34 µg/ml). After overnight culture at 30°C, single colonies were selected for PCR verification using primers XZ-frd-up/XZ-frd-down. The correct PCR product should be 3440 bp, which includes a 426 bp upstream homology arm of frd, a 2619 bp cat-sacB fragment, and a 395 bp downstream homology arm of frd. A correct single colony was selected and named Span019.

In the second step, using genomic DNA of *Escherichia coli* MG1655 (from ATCC, No. 700926) as a template, an 895 bp DNA fragment II was amplified with primers frd-panB-up/frd-panB-down. The DNA fragment II included a 50 bp upstream homology arm of frd, a 795 bp panB gene, and a 50 bp downstream homology arm of frd. The DNA fragment II was used for the second homologous recombination. The DNA fragment II was electroporated into the strain Span019.

The electroporation conditions and steps were consistent with those of the second-step method described in Example 1 for the integration of alsS at the tdcDE locus. Colony PCR was performed to verify the clones using primers XZ-frd-up/XZ-frd-down. The correct colony amplification product was a 1661 bp fragment, which included a 426 bp upstream homology arm of frd, a 795 bp panB gene, and a 395 bp downstream homology arm of frd. A correct single colony was selected and named Span020.

Span020 is a recombinant strain obtained by integrating the 3-methyl-2-oxobutanoate hydroxymethyltransferase gene panB (encoding the protein sequence shown in SEQ ID NO: 15, NCBI QPA14045.1, coded_by=CP032679.1:148806..149600) into the frd locus of *Escherichia coli* Span018. In this recombinant strain, the fumarate reductase-encoding gene frd (encoding the protein sequence NCBI ACA79462.1, coded_by=CP000946.1:4217304..4217699) is simultaneously knocked out.

### Example 11: Expression regulation of 3-methyl-2-oxobutanoate hydroxymethyltransferase gene panB

Starting from Span020, the expression of the 3-methyl-2-oxobutanoate hydroxymethyltransferase gene panB integrated at the fumarate reductase-encoding gene frd locus was regulated using artificial regulatory elements. The specific steps were as follows:
In the first step, using pXZ-CS plasmid DNA as a template, a 2719 bp DNA fragment I was amplified with primers frd-cs-up/panB-Pcs-down, which was used for the first homologous recombination. The DNA fragment I included a 50 bp upstream homology arm of frd, a 2619 bp cat-sacB fragment, and a 50 bp downstream homology arm of panB. The amplification system and amplification conditions were consistent with those described in Example 1. The DNA fragment I was electroporated into Span020.

The DNA fragment I was used for the first homologous recombination: first, the pKD46 plasmid was transformed into *Escherichia coli* Span020 by electroporation, and then the DNA fragment I was electroporated into *Escherichia coli* Span020 harboring pKD46.

The electroporation conditions and steps were consistent with those of the first-step method described in Example 1 for the integration of alsS at the tdcDE locus. 200 µl of the bacterial solution was taken and spread on an LB plate containing ampicillin (final concentration 100 µg/ml) and chloramphenicol (final concentration 34 µg/ml). After overnight culture at 30°C, single colonies were selected for PCR verification using primers XZ-frd-up/panB-YZ130-down. The correct PCR product should be 3175 bp, which includes a 426 bp upstream homology arm of frd, a 2619 bp cat-sacB fragment, and a 130 bp downstream homology arm of panB. A correct single colony was selected and named Span021.

In the second step, using genomic DNA of M1-93 (Lu, et al., Appl Microbiol Biotechnol, 2012, 93: 2455-2462) as a template, an 188 bp DNA fragment II was amplified with primers panB-Pro-up/panB-Pro-down. The DNA fragment II included a 50 bp upstream homology arm of frd, an 88 bp M1-93 promoter sequence, and a 50 bp downstream homology arm of panB. The DNA fragment II was used for the second homologous recombination. The DNA fragment II was electroporated into the strain Span021.

The electroporation conditions and steps were consistent with those of the second-step method described in Example 1 for the integration of alsS at the tdcDE locus. Colony PCR was performed to verify the clones using primers XZ-frd-up/panB-YZ130-down. The correct colony amplification product was a 644 bp fragment, which included a 426 bp upstream homology arm of frd, an 88 bp M1-93 promoter sequence, and a 130 bp downstream homology arm of panB. A correct single colony was selected and named Span022.

Span022 is a recombinant strain obtained by integrating the M1-93 promoter (whose nucleotide sequence is SEQ ID NO: 169 in the sequence listing) upstream of the panB gene in *Escherichia coli* Span020. In this recombinant strain, the M1-93 promoter drives the expression of the panB gene.

### Example 12: Integration of 2-dehydropantoate-2-reductase gene panE at the lactate dehydrogenase gene IdhA locus and knockout of the IdhA gene

Starting from Span022, the 2-dehydropantoate-2-reductase gene panE was integrated into the lactate dehydrogenase gene ldhA locus. The specific steps were as follows:
In the first step, using pXZ-CS plasmid DNA as a template, a 2719 bp DNA fragment I was amplified with primers ldhA-csin-up/ldhA-csin-down, which was used for the first homologous recombination. The DNA fragment I included a 50 bp upstream homology arm of ldhA, a 2619 bp cat-sacB fragment, and a 50 bp downstream homology arm of ldhA. The amplification system and amplification conditions were consistent with those described in Example 1. The DNA fragment I was electroporated into Span022.

The DNA fragment I was used for the first homologous recombination: first, the pKD46 plasmid was transformed into *Escherichia coli* Span022 by electroporation, and then the DNA fragment I was electroporated into *Escherichia coli* Span022 harboring pKD46.

The electroporation conditions and steps were consistent with those of the first-step method described in Example 1 for the integration of alsS at the tdcDE locus. 200 µl of the bacterial solution was taken and spread on an LB plate containing ampicillin (final concentration 100 µg/ml) and chloramphenicol (final concentration 34 µg/ml). After overnight culture at 30°C, single colonies were selected for PCR verification using primers XZ-ldhA-up/XZ-ldhA-down. The correct PCR product should be 3415 bp, which includes a 380 bp upstream homology arm of ldhA, a 2619 bp cat-sacB fragment, and a 416 bp downstream homology arm of ldhA. A correct single colony was selected and named Span023.

In the second step, using genomic DNA of *Escherichia coli* MG1655 (from ATCC, No. 700926) as a template, a 1012 bp DNA fragment II was amplified with primers ldhA-panE-up/ldhA-panE-down. The DNA fragment II included a 50 bp upstream homology arm of ldhA, a 912 bp panE gene, and a 50 bp downstream homology arm of ldhA. The DNA fragment II was used for the second homologous recombination. The DNA fragment II was electroporated into the strain Span023.

The electroporation conditions and steps were consistent with those of the second-step method described in Example 1 for the integration of alsS at the tdcDE locus. Colony PCR was performed to verify the clones using primers XZ-ldhA-up/XZ-ldhA-down. The correct colony amplification product was a 1708 bp fragment, which included a 380 bp upstream homology arm of ldhA, a 912 bp panE gene, and a 416 bp downstream homology arm of ldhA. A correct single colony was selected and named Span024.

Span024 is a recombinant strain obtained by integrating the 2-dehydropantoate-2-reductase gene panE (encoding the protein sequence shown in SEQ ID NO: 17, NCBI QPA14304.1, coded_by=CP032679.1:443607..444518) into the ldhA locus of *Escherichia coli* Span022. In this recombinant strain, the lactate dehydrogenase gene ldhA (encoding the protein sequence NCBI ACA77911.1, coded_by=CP000946.1:2508048..2509037) is simultaneously knocked out.

### Example 13: Expression regulation of 2-dehydropantoate-2-reductase gene panE

Starting from Span024, the expression of the 2-dehydropantoate-2-reductase gene panE integrated at the lactate dehydrogenase gene ldhA locus was regulated using artificial regulatory elements. The specific steps were as follows:
In the first step, using pXZ-CS plasmid DNA as a template, a 2719 bp DNA fragment I was amplified with primers ldhA-csin-up/panE-ProCS-down, which was used for the first homologous recombination. The DNA fragment I included a 50 bp upstream homology arm of ldhA, a 2619 bp cat-sacB fragment, and a 50 bp downstream homology arm of panE. The amplification system and amplification conditions were consistent with those described in Example 1. The DNA fragment I was electroporated into Span024.

The DNA fragment I was used for the first homologous recombination: first, the pKD46 plasmid was transformed into *Escherichia coli* Span024 by electroporation, and then the DNA fragment I was electroporated into *Escherichia coli* Span024 harboring pKD46.

The electroporation conditions and steps were consistent with those of the first-step method described in Example 1 for the integration of alsS at the tdcDE locus. 200 µl of the bacterial solution was taken and spread on an LB plate containing ampicillin (final concentration 100 µg/ml) and chloramphenicol (final concentration 34 µg/ml). After overnight culture at 30°C, single colonies were selected for PCR verification using primers XZ-ldhA-up/panE-YZ245-down. The correct PCR product should be 3244 bp, which includes a 380 bp upstream homology arm of ldhA, a 2619 bp cat-sacB fragment, and a 245 bp downstream homology arm of panE. A correct single colony was selected and named Span025.

In the second step, using genomic DNA of M1-93 (Lu, et al., Appl Microbiol Biotechnol, 2012, 93: 2455-2462) as a template, a 189 bp DNA fragment II was amplified with primers panE-Pro-up/panE-Pro-down. The DNA fragment II included a 50 bp upstream homology arm of ldhA, an 89 bp artificial promoter RBSL2 sequence, and a 50 bp downstream homology arm of panE. The DNA fragment II was used for the second homologous recombination. The DNA fragment II was electroporated into the strain Span025.

The electroporation conditions and steps were consistent with those of the second-step method described in Example 1 for the integration of alsS at the tdcDE locus. Colony PCR was performed to verify the clones using primers XZ-ldhA-up/panE-YZ245-down. The correct colony amplification product was a 714 bp fragment, which included a 380 bp upstream homology arm of ldhA, an 89 bp artificial promoter RBSL2 sequence, and a 245 bp downstream homology arm of panE. A correct single colony was selected and named Span026.

Span026 is a recombinant strain obtained by integrating the RBSL2 promoter (whose nucleotide sequence is SEQ ID NO: 172 in the sequence listing) upstream of the panE gene in *Escherichia coli* Span024. In this recombinant strain, the RBSL2 promoter drives the expression of the panE gene.

### Example 14: Integration of serine hydroxymethyltransferase gene glyA at the methylglyoxal synthase gene mgsA locus and knockout of the mgsA gene

Starting from Span026, the serine hydroxymethyltransferase gene glyA was integrated at the methylglyoxal synthase gene mgsA locus. The specific steps were as follows:
In the first step, using pXZ-CS plasmid DNA as a template, a 2719 bp DNA fragment I was amplified with primers mgsA-cs-up/mgsA-cs-down, which was used for the first homologous recombination. The DNA fragment I included a 50 bp upstream homology arm of mgsA, a 2619 bp cat-sacB fragment, and a 50 bp downstream homology arm of mgsA. The amplification system and amplification conditions were consistent with those described in Example 1. The DNA fragment I was electroporated into Span026.

The DNA fragment I was used for the first homologous recombination: first, the pKD46 plasmid was transformed into *Escherichia coli* Span026 by electroporation, and then the DNA fragment I was electroporated into *Escherichia coli* Span026 harboring pKD46.

The electroporation conditions and steps were consistent with those of the first-step method described in Example 1 for the integration of alsS at the tdcDE locus. 200 µl of the bacterial solution was taken and spread on an LB plate containing ampicillin (final concentration 100 µg/ml) and chloramphenicol (final concentration 34 µg/ml). After overnight culture at 30°C, single colonies were selected for PCR verification using primers XZ-mgsA-up/XZ-mgsA-down. The correct PCR product should be 3646 bp, which includes a 516 bp upstream homology arm of mgsA, a 2619 bp cat-sacB fragment, and a 511 bp downstream homology arm of mgsA. A correct single colony was selected and named Span027.

In the second step, using genomic DNA of wild-type *Escherichia coli* ATCC8739 as a template, a 1354 bp DNA fragment II was amplified with primers mgsA-glyA-up/mgsA-glyA-down. The DNA fragment II included a 50 bp upstream homology arm of mgsA, a 1254 bp glyA fragment, and a 50 bp downstream homology arm of mgsA. The DNA fragment II was used for the second homologous recombination. The DNA fragment II was electroporated into the strain Span027.

The electroporation conditions and steps were consistent with those of the second-step method described in Example 1 for the integration of alsS at the tdcDE locus. Colony PCR was performed to verify the clones using primers XZ-mgsA-up/XZ-mgsA-down. The correct colony amplification product was a 2281 bp fragment, which included a 516 bp upstream homology arm of mgsA, a 1254 bp glyA fragment, and a 511 bp downstream homology arm of mgsA. A correct single colony was selected and named Span028.

Span028 is a recombinant strain obtained by integrating the serine hydroxymethyltransferase gene glyA (encoding the protein sequence shown in SEQ ID NO: 19, NCBI ACA76793.1, coded_by=CP000946.1:1227416..1228669) into the mgsA locus of *Escherichia coli* Span026. In this recombinant strain, the mgsA gene (encoding the protein sequence NCBI ACA78263.1, coded_by=CP000946.1:2883345..2883803) is simultaneously knocked out.

### Example 15: Regulation of serine hydroxymethyltransferase gene glyA

Starting from Span028, the expression of the serine hydroxymethyltransferase gene glyA integrated at the mgsA locus was regulated using artificial regulatory elements. The specific steps were as follows:
In the first step, using pXZ-CS plasmid DNA as a template, a 2719 bp DNA fragment I was amplified with primers mgsA-cs-up/glyA-ProCS-down, which was used for the first homologous recombination. The DNA fragment I included a 50 bp upstream homology arm of mgsA, a 2619 bp cat-sacB fragment, and a 50 bp downstream homology arm of glyA. The amplification system and amplification conditions were consistent with those described in Example 1. The DNA fragment I was electroporated into Span028.

The DNA fragment I was used for the first homologous recombination: first, the pKD46 plasmid was transformed into *Escherichia coli* Span028 by electroporation, and then the DNA fragment I was electroporated into *Escherichia coli* Span028 harboring pKD46.

The electroporation conditions and steps were consistent with those of the first-step method described in Example 1 for the integration of alsS at the tdcDE locus. 200 µl of the bacterial solution was taken and spread on an LB plate containing ampicillin (final concentration 100 µg/ml) and chloramphenicol (final concentration 34 µg/ml). After overnight culture at 30°C, single colonies were selected for PCR verification using primers XZ-mgsA-up/glyA-YZ364-down. The correct PCR product should be 3499 bp, which includes a 516 bp upstream homology arm of mgsA, a 2619 bp cat-sacB fragment, and a 364 bp downstream homology arm of glyA. A correct single colony was selected and named Span029.

In the second step, using genomic DNA of M1-46 (Lu, et al., Appl Microbiol Biotechnol, 2012, 93: 2455-2462) as a template, an 188 bp DNA fragment II was amplified with primers glyA-Pro-up/glyA-Pro-down. The DNA fragment II included a 50 bp upstream homology arm of mgsA, an 88 bp M1-46 promoter, and a 50 bp downstream homology arm of glyA. The DNA fragment II was used for the second homologous recombination. The DNA fragment II was electroporated into the strain Span029.

The electroporation conditions and steps were consistent with those of the second-step method described in Example 1 for the integration of alsS at the tdcDE locus. Colony PCR was performed to verify the clones using primers XZ-mgsA-up/glyA-YZ364-down. The correct colony amplification product was a 968 bp fragment, which included a 516 bp upstream homology arm of mgsA, an 88 bp M1-46 promoter, and a 364 bp downstream homology arm of glyA. A correct single colony was selected and named Span030.

Span030 is a recombinant strain obtained by integrating the M1-46 promoter (whose nucleotide sequence is SEQ ID NO: 173 in the sequence listing) upstream of the glyA gene in Span028. In this recombinant strain, the M1-46 promoter drives the expression of the glyA gene.

### Example 16: Regulation of wild-type aminomethyltransferase gene gcvT in Escherichia coli

Starting from Span030, the expression of the wild-type aminomethyltransferase gene gcvT in *Escherichia coli* was regulated using artificial regulatory elements. The specific steps were as follows:
In the first step, using pXZ-CS plasmid DNA as a template, a 2719 bp DNA fragment I was amplified with primers gcvT-Pcat-up/gcvT-PsacB-down, which was used for the first homologous recombination. The DNA fragment I included a 50 bp upstream homology arm of the gcvT gene, a 2619 bp cat-sacB fragment, and a 50 bp downstream homology arm of gcvT. The amplification system and amplification conditions were consistent with those described in Example 1. The DNA fragment I was electroporated into Span030.

The DNA fragment I was used for the first homologous recombination: first, the pKD46 plasmid was transformed into *Escherichia coli* Span030 by electroporation, and then the DNA fragment I was electroporated into *Escherichia coli* Span030 harboring pKD46.

The electroporation conditions and steps were consistent with those of the first-step method described in Example 1 for the integration of alsS at the tdcDE locus. 200 µl of the bacterial solution was taken and spread on an LB plate containing ampicillin (final concentration 100 µg/ml) and chloramphenicol (final concentration 34 µg/ml). After overnight culture at 30°C, single colonies were selected for PCR verification using primers gcvT-up-500/gcvT-350-down. The correct PCR product should be 3197 bp, which includes a 228 bp upstream homology arm of the gcvT gene, a 2619 bp cat-sacB fragment, and a 350 bp downstream homology arm of gcvT. A correct single colony was selected and named Span031.

In the second step, using genomic DNA of M1-93 (Lu, et al., Appl Microbiol Biotechnol, 2012, 93: 2455-2462) as a template, a 188 bp DNA fragment II was amplified with primers gcvT-M93-up/gcvT-M93-down. The DNA fragment II included a 50 bp upstream homology arm of the gcvT gene, an 88 bp M1-93 promoter, and a 50 bp downstream homology arm of gcvT. The DNA fragment II was used for the second homologous recombination. The DNA fragment II was electroporated into the strain Span031.

The electroporation conditions and steps were consistent with those of the second-step method described in Example 1 for the integration of alsS at the tdcDE locus. Colony PCR was performed to verify the clones using primers gcvT-up-500/gcvT-350-down. The correct colony amplification product was a 666 bp fragment, which included a 228 bp upstream homology arm of the gcvT gene, an 88 bp M1-93 promoter, and a 350 bp downstream homology arm of gcvT. A correct single colony was selected and named Span032.

Span032 is a recombinant strain obtained by integrating the M1-93 promoter (whose nucleotide sequence is SEQ ID NO: 169 in the sequence listing) upstream of the aminomethyltransferase gene gcvT (encoding the protein sequence shown in SEQ ID NO: 21, NCBI ACA76476.1, coded_by=CP000946.1:862077..863171) in *Escherichia coli* Span030. In this recombinant strain, the M1-93 promoter can drive the expression of the gcvT gene.

### Example 17: Regulation of wild-type glycine decarboxylase gene gcvP in Escherichia coli

Starting from Span032, the expression of the wild-type glycine decarboxylase gene gcvP in *Escherichia coli* was regulated using artificial regulatory elements. The specific steps were as follows:
In the first step, using pXZ-CS plasmid DNA as a template, a 2719 bp DNA fragment I was amplified with primers gcvP-Pcat-up/gcvP-PsacB-down, which was used for the first homologous recombination. The DNA fragment I included a 50 bp upstream homology arm of the gcvP gene, a 2619 bp cat-sacB fragment, and a 50 bp downstream homology arm of gcvP. The amplification system and amplification conditions were consistent with those described in Example 1. The DNA fragment I was electroporated into Span032.

The DNA fragment I was used for the first homologous recombination: first, the pKD46 plasmid was transformed into *Escherichia coli* Span032 by electroporation, and then the DNA fragment I was electroporated into *Escherichia coli* Span032 harboring pKD46.

The electroporation conditions and steps were consistent with those of the first-step method described in Example 1 for the integration of alsS at the tdcDE locus. 200 µl of the bacterial solution was taken and spread on an LB plate containing ampicillin (final concentration 100 µg/ml) and chloramphenicol (final concentration 34 µg/ml). After overnight culture at 30°C, single colonies were selected for PCR verification using primers gcvH-up/gcvP-390-down. The correct PCR product should be 3399 bp, which includes a 390 bp upstream homology arm of the gcvP gene, a 2619 bp cat-sacB fragment, and a 390 bp downstream homology arm of gcvP. A correct single colony was selected and named Span033.

In the second step, using genomic DNA of M1-93 (Lu, et al., Appl Microbiol Biotechnol, 2012, 93: 2455-2462) as a template, a 188 bp DNA fragment II was amplified with primers gcvP-M93-up/gcvP-M93-down. The DNA fragment II included a 50 bp upstream homology arm of the gcvP gene, an 88 bp M1-93 promoter, and a 50 bp downstream homology arm of gcvP. The DNA fragment II was used for the second homologous recombination. The DNA fragment II was electroporated into the strain Span033.

The electroporation conditions and steps were consistent with those of the second-step method described in Example 1 for the integration of alsS at the tdcDE locus. Colony PCR was performed to verify the clones using primers gcvH-up/gcvP-390-down. The correct colony amplification product was an 868 bp fragment, which included a 390 bp upstream homology arm of the gcvP gene, an 88 bp M1-93 promoter, and a 390 bp downstream homology arm of gcvP. A correct single colony was selected and named Span034.

Span034 is a recombinant strain obtained by integrating the M1-93 promoter (whose nucleotide sequence is SEQ ID NO: 169 in the sequence listing) upstream of the glycine decarboxylase gene gcvP (encoding the protein sequence shown in SEQ ID NO: 23, NCBI ACA76478.1, coded_by=CP000946.1:863703..866576) in *Escherichia coli* Span032. In this recombinant strain, the M1-93 promoter can drive the expression of the gcvP gene.

### Example 18: Integration of 3-methyl-2-oxobutanoate hydroxymethyltransferase gene panB derived from Corynebacterium glutamicum at the loci of phosphate acetyltransferase-encoding gene pta and acetate kinase-encoding gene ackA, and knockout of the ackA gene and the pta gene

Starting from Span034, the 3-methyl-2-oxobutanoate hydroxymethyltransferase gene panB derived from *Corynebacterium glutamicum* at the loci of phosphate acetyltransferase-encoding gene pta and acetate kinase-encoding gene ackA. The specific steps were as follows:
In the first step, using pXZ-CS plasmid DNA as a template, a 2719 bp DNA fragment I was amplified with primers ackA-cs-up/pta-cs-down, which was used for the first homologous recombination. The DNA fragment I included a 50 bp upstream homology arm of the ackA-pta genes, a 2619 bp cat-sacB fragment, and a 50 bp downstream homology arm of ackA-pta. The amplification system and amplification conditions were consistent with those described in Example 1. The DNA fragment I was electroporated into Span034.

The DNA fragment I was used for the first homologous recombination: first, the pKD46 plasmid was transformed into *Escherichia coli* Span034 by electroporation, and then the DNA fragment I was electroporated into *Escherichia coli* Span034 harboring pKD46.

The electroporation conditions and steps were consistent with those of the first-step method described in Example 1 for the integration of alsS at the tdcDE locus. 200 µl of the bacterial solution was taken and spread on an LB plate containing ampicillin (final concentration 100 µg/ml) and chloramphenicol (final concentration 34 µg/ml). After overnight culture at 30°C, single colonies were selected for PCR verification using primers XZ-ackA-up/XZ-pta-down. The correct PCR product should be 3350 bp, which includes a 320 bp upstream homology arm of the ackA-pta genes, a 2619 bp cat-sacB fragment, and a 411 bp downstream homology arm of ackA-pta. A correct single colony was selected and named Span035.

In the second step, using genomic DNA of Corynebacterium glutamicum ATCC13032 (ATCC product) as a template, a 916 bp DNA fragment II was amplified with primers ackA-panBC-up/ackA-panBC-down. The DNA fragment II included a 50 bp upstream homology arm of the ackA-pta genes, an 816 bp panB gene derived from *Corynebacterium glutamicum,* and a 50 bp downstream homology arm of ackA-pta. The DNA fragment II was used for the second homologous recombination. The DNA fragment II was electroporated into the strain Span035.

The electroporation conditions and steps were consistent with those of the second-step method described in Example 1 for the integration of alsS at the tdcDE locus. Colony PCR was performed to verify the clones using primers XZ-ackA-up/XZ-pta-down. The correct colony amplification product was a 1547 bp fragment, which included a 320 bp upstream homology arm of the ackA-pta genes, an 816 bp panB gene derived from *Corynebacterium glutamicum,* and a 411 bp downstream homology arm of ackA-pta. A correct single colony was selected and named Span036.

Span036 is a recombinant strain obtained by integrating the 3-methyl-2-oxobutanoate hydroxymethyltransferase gene panB from *Corynebacterium glutamicum* (the panB gene, whose nucleotide sequence is SEQ ID NO: 14 in the sequence listing and encodes the panB protein shown in SEQ ID NO: 13) into the loci of phosphate acetyltransferase-encoding gene pta and acetate kinase-encoding gene ackA in *Escherichia coli* Span034. In this recombinant strain, both the pta gene (encoding the protein sequence NCBI ACA77021.1, coded_by=CP000946.1:1484032..1486176) and the ackA gene (encoding the protein sequence NCBI ACA77022.1, coded_by=CP000946.1:1486251..1487453) are knocked out.

### Example 19: Regulation of 3-methyl-2-oxobutanoate hydroxymethyltransferase gene panB derived from Corynebacterium glutamicum

Starting from Span036, the expression of the 3-methyl-2-oxobutanoate hydroxymethyltransferase gene panB integrated at the ackA-pta locus was regulated using artificial regulatory elements. The specific steps were as follows:
In the first step, using pXZ-CS plasmid DNA as a template, a 2719 bp DNA fragment I was amplified with primers ackA-cs-up/panBC-ProCS-down, which was used for the first homologous recombination. The DNA fragment I included a 50 bp upstream homology arm of the ackA-pta genes, a 2619 bp cat-sacB fragment, and a 50 bp downstream homology arm of the panB gene derived from *Corynebacterium glutamicum.* The amplification system and amplification conditions were consistent with those described in Example 1. The DNA fragment I was electroporated into Span036.

The DNA fragment I was used for the first homologous recombination: first, the pKD46 plasmid was transformed into *Escherichia coli* Span036 by electroporation, and then the DNA fragment I was electroporated into *Escherichia coli* Span036 harboring pKD46.

The electroporation conditions and steps were consistent with those of the first-step method described in Example 1 for the integration of alsS at the tdcDE locus. 200 µl of the bacterial solution was taken and spread on an LB plate containing ampicillin (final concentration 100 µg/ml) and chloramphenicol (final concentration 34 µg/ml). After overnight culture at 30°C, single colonies were selected for PCR verification using primers XZ-ackA-up/panBC-YZ425-down. The correct PCR product should be 3364 bp, which includes a 320 bp upstream homology arm of the ackA-pta genes, a 2619 bp cat-sacB fragment, and a 425 bp downstream homology arm of panB. A correct single colony was selected and named Span037.

In the second step, using genomic DNA of M1-93 (Lu, et al., Appl Microbiol Biotechnol, 2012, 93: 2455-2462) as a template, a 188 bp DNA fragment II was amplified with primers panBC-Pro-up/panBC-Pro-down. The DNA fragment II included a 50 bp upstream homology arm of the ackA-pta genes, an 88 bp M1-93 promoter, and a 50 bp downstream homology arm of panB. The DNA fragment II was used for the second homologous recombination. The DNA fragment II was electroporated into the strain Span037.

The electroporation conditions and steps were consistent with those of the second-step method described in Example 1 for the integration of alsS at the tdcDE locus. Colony PCR was performed to verify the clones using primers XZ-ackA-up/panBC-YZ425-down. The correct colony amplification product was an 833 bp fragment, which included a 320 bp upstream homology arm of the ackA-pta genes, an 88 bp M1-93 promoter, and a 425 bp downstream homology arm of panB. A correct single colony was selected and named Span038.

Span038 is a recombinant strain obtained by integrating the M1-93 promoter (whose nucleotide sequence is SEQ ID NO: 169 in the sequence listing) upstream of the panB gene in *Escherichia coli* Span036. In this recombinant strain, the M1-93 promoter drives the expression of the panB gene.

### Example 20: Attenuation of expression of branched-chain amino acid aminotransferase gene ilvE

Starting from Span038, the expression of the branched-chain amino acid aminotransferase gene ilvE was attenuated. The specific steps were as follows:
In the first step, using pXZ-CS plasmid DNA as a template, a 2719 bp DNA fragment I was amplified with primers ilvE-cat-up/ilvE-sacB-down, which was used for the first homologous recombination. The DNA fragment I included a 50 bp upstream homology arm of the ilvE gene, a 2619 bp cat-sacB fragment, and a 50 bp downstream homology arm of the ilvE gene. The amplification system and amplification conditions were consistent with those described in Example 1. The DNA fragment I was electroporated into Span038.

The DNA fragment I was used for the first homologous recombination: first, the pKD46 plasmid was transformed into *Escherichia coli* Span038 by electroporation, and then the DNA fragment I was electroporated into *Escherichia coli* Span038 harboring pKD46.

The electroporation conditions and steps were consistent with those of the first-step method described in Example 1 for the integration of alsS at the tdcDE locus. 200 µl of the bacterial solution was taken and spread on an LB plate containing ampicillin (final concentration 100 µg/ml) and chloramphenicol (final concentration 34 µg/ml). After overnight incubation at 30°C, single colonies were selected for PCR verification using primers ilvM-up/ilvE-down. The correct PCR product should be 3832 bp, which includes a 283 bp upstream homology arm of the ilvE gene, a 2619 bp cat-sacB fragment, and a 930 bp downstream homology arm of the ilvE gene. A correct single colony was selected and named Span039.

In the second step, using genomic DNA of wild-type *Escherichia coli* ATCC 8739 as a template, a 980 bp DNA fragment II was amplified with primers ilvEGTG-up/ilvE-down. The DNA fragment II included the ilvE gene with its start codon ATG changed to GTG. The DNA fragment II was used for the second homologous recombination. The DNA fragment II was electroporated into the strain Span039.

The electroporation conditions and steps were consistent with those of the second-step method described in Example 1 for the integration of alsS at the tdcDE locus. Colony PCR was performed to verify the clones using primers ilvM-up/ilvE-down. The correct colony amplification product was a 1213 bp fragment, which included a 283 bp upstream homology arm of the ilvE gene and a total of 930 bp of the ilvE gene with its start codon ATG replaced by GTG. A correct single colony was selected and named Span040.

Span040 is a recombinant strain obtained by mutating the start codon ATG of ilvE in Span038 to GTG. The mutated gene is designated as the ilvE* gene (whose sequence is SEQ ID NO: 32 in the sequence listing), and the ilvE* gene encodes the ilvE* protein (whose sequence is SEQ ID NO: 31 in the sequence listing).

### Example 21: Integration of phosphoglycerate dehydrogenase gene serA derived from Corynebacterium glutamicum at the ribokinase ara locus and knockout of the ara gene

Starting from Span040, the phosphoglycerate dehydrogenase gene serA derived from *Corynebacterium glutamicum* was integrated at the ribokinase ara locus. The specific steps were as follows:
In the first step, using pXZ-CS plasmid DNA as a template, a 2719 bp DNA fragment I was amplified with primers araBCD-CS-up/araBCD-CS-down, which was used for the first homologous recombination. The DNA fragment I included a 50 bp upstream homology arm of the ara locus, a 2619 bp cat-sacB fragment, and a 50 bp downstream homology arm of the ara locus. The amplification system and amplification conditions were consistent with those described in Example 1. The DNA fragment I was electroporated into Span041.

The DNA fragment I was used for the first homologous recombination: first, the pKD46 plasmid was transformed into *Escherichia coli* Span041 by electroporation, and then the DNA fragment I was electroporated into *Escherichia coli* Span041 harboring pKD46.

The electroporation conditions and steps were consistent with those of the first-step method described in Example 1 for the integration of alsS at the tdcDE locus. 200 µl of the bacterial solution was taken and spread on an LB plate containing ampicillin (final concentration 100 µg/ml) and chloramphenicol (final concentration 34 µg/ml). After overnight culture at 30°C, single colonies were selected for PCR verification using primers araBCD-YZ300-up/araBCD-YZ468-down. The correct PCR product should be 3378 bp, which includes a 291 bp upstream homology arm of the ara locus, a 2619 bp cat-sacB fragment, and a 468 bp downstream homology arm of the ara locus. A correct single colony was selected and named Span041.

In the second step, using genomic DNA of *Corynebacterium glutamicum* ATCC13032 (ATCC product) as a template, a 1102 bp DNA fragment II was amplified with primers araBCD-serA197-up/araBCD-serA197-down. The DNA fragment II included a 50 bp upstream homology arm of the ara locus, a 1002 bp serA gene, and a 50 bp downstream homology arm of the ara locus. The DNA fragment II was used for the second homologous recombination. The DNA fragment II was electroporated into the strain Span041.

The electroporation conditions and steps were consistent with those of the second-step method described in Example 1 for the integration of alsS at the tdcDE locus. Colony PCR was performed to verify the clones using primers araBCD-YZ300-up/araBCD-YZ468-down. The correct colony amplification product was a 1761 bp fragment, which included a 291 bp upstream homology arm of the ara locus, a 1002 bp serA gene, and a 468 bp downstream homology arm of the ara locus. A correct single colony was selected and named Span042.

Span042 is a recombinant strain obtained by integrating the phosphoglycerate dehydrogenase gene serA from *Corynebacterium glutamicum* (the serA gene, whose nucleotide sequence is SEQ ID NO: 26 in the sequence listing and encodes the serA protein shown in SEQ ID NO: 25) into the ara locus of *Escherichia coli* Span040. In this recombinant strain, the ara genes (encoding the protein sequences NCBI ACA79208.1, coded_by=CP000946.1:3929533..3931233; and NCBI ACA79209.1, coded_by=CP000946.1:3931244..3932746) are simultaneously knocked out.

### Example 22: Regulation of phosphoglycerate dehydrogenase gene serA derived from Corynebacterium glutamicum

Starting from Span042, the expression of the phosphoglycerate dehydrogenase gene serA integrated at the ara locus was regulated using artificial regulatory elements. The specific steps were as follows:
In the first step, using pXZ-CS plasmid DNA as a template, a 2719 bp DNA fragment I was amplified with primers araBCD-CS-up/serA197-ProCS-down, which was used for the first homologous recombination. The DNA fragment I included a 50 bp upstream homology arm of the ara locus, a 2619 bp cat-sacB fragment, and a 50 bp downstream homology arm of the serA locus. The amplification system and amplification conditions were consistent with those described in Example 1. The DNA fragment I was electroporated into Span042.

The DNA fragment I was used for the first homologous recombination: first, the pKD46 plasmid was transformed into *Escherichia coli* Span042 by electroporation, and then the DNA fragment I was electroporated into *Escherichia coli* Span042 harboring pKD46.

The electroporation conditions and steps were consistent with those of the first-step method described in Example 1 for the integration of alsS at the tdcDE locus. 200 µl of the bacterial solution was taken and spread on an LB plate containing ampicillin (final concentration 100 µg/ml) and chloramphenicol (final concentration 34 µg/ml). After overnight culture at 30°C, single colonies were selected for PCR verification using primers araBCD-YZ300-up/SerA197-YZ358-down. The correct PCR product should be 3268 bp, which includes a 291 bp upstream homology arm of the ara locus, a 2619 bp cat-sacB fragment, and a 358 bp downstream homology arm of the serA locus. A correct single colony was selected and named Span043.

In the second step, using genomic DNA of M1-93 (Lu, et al., Appl Microbiol Biotechnol, 2012, 93: 2455-2462) as a template, a 188 bp DNA fragment II was amplified with primers serA197-Pro-up/serA197-Pro-down. The DNA fragment II included a 50 bp upstream homology arm of the ara locus, an 88 bp M1-93 promoter, and a 50 bp downstream homology arm of the serA gene. The DNA fragment II was used for the second homologous recombination. The DNA fragment II was electroporated into the strain Span043.

The electroporation conditions and steps were consistent with those of the second-step method described in Example 1 for the integration of alsS at the tdcDE locus. Colony PCR was performed to verify the clones using primers araBCD-YZ300-up/SerA197-YZ358-down. The correct colony amplification product was a 737 bp fragment, which included a 291 bp upstream homology arm of the ara locus, an 88 bp M1-93 promoter, and a 358 bp downstream homology arm of the serA locus. A correct single colony was selected and named Span044.

Span044 is a recombinant strain obtained by integrating the M1-93 promoter (whose nucleotide sequence is SEQ ID NO: 169 in the sequence listing) upstream of the serA gene in *Escherichia coli* Span042. In this recombinant strain, the M1-93 promoter drives the expression of the serA gene.

### Example 23: Integration of phosphoserine/phosphohydroxythreonine aminotransferase gene serC and phosphoserine phosphatase gene serB derived from Escherichia coli at the valine-pyruvate transaminase gene avtA locus, and knockout of the avtA gene

Starting from Span044, the phosphoserine/phosphohydroxythreonine aminotransferase gene serC and phosphoserine phosphatase gene serB derived from *Escherichia coli* were integrated at the valine-pyruvate transaminase gene avtA locus. The specific steps were as follows:
In the first step, using pXZ-CS plasmid DNA as a template, a 2719 bp DNA fragment I was amplified with primers avtA-CS-up/avtA-CS-down, which was used for the first homologous recombination. The DNA fragment I included a 50 bp upstream homology arm of the avtA locus, a 2619 bp cat-sacB fragment, and a 50 bp downstream homology arm of the avtA locus. The amplification system and amplification conditions were consistent with those described in Example 1. The DNA fragment I was electroporated into Span044.

The DNA fragment I was used for the first homologous recombination: first, the pKD46 plasmid was transformed into *Escherichia coli* Span044 by electroporation, and then the DNA fragment I was electroporated into *Escherichia coli* Span044 harboring pKD46.

The electroporation conditions and steps were consistent with those of the first-step method described in Example 1 for the integration of alsS at the tdcDE locus. 200 µl of the bacterial solution was taken and spread on an LB plate containing ampicillin (final concentration 100 µg/ml) and chloramphenicol (final concentration 34 µg/ml). After overnight culture at 30°C, single colonies were selected for PCR verification using primers avtA-YZ-up/avtA-YZ-down. The correct PCR product should be 3454 bp, which includes a 416 bp upstream homology arm of the avtA locus, a 2619 bp cat-sacB fragment, and a 419 bp downstream homology arm of the avtA locus. A correct single colony was selected and named Span045.

In the second step, using genomic DNA of *Escherichia coli* MG1655 (from ATCC, No. 700926) as a template, a 1181 bp fragment II was amplified with primers avtA-serCB-up/serC-down. Using genomic DNA of *Escherichia coli* MG1655 (from ATCC, No. 700926) as a template, a 1062 bp fragment III was amplified with primers serB-up/avtA-serCB-down. PCR amplification was performed with primers avtA-serCB-up/avtA-serCB-down using equimolar amounts of the fragment II and the fragment III as templates. Fusion PCR was performed using the amplification system and conditions as described in Example 1 to obtain fragment IV. The fragment IV was a 2179 bp DNA fragment, which was used for the second homologous recombination. The fragment IV included a 50 bp upstream homology arm of avtA, a 1089 bp serC gene, a 21 bp RBS sequence for the translation initiation of the serB gene and a 969 bp serB gene, and a 50 bp downstream homology arm of avtA. The DNA fragment IV was electroporated into the strain Span045.

The electroporation conditions and steps were consistent with those of the second-step method described in Example 1 for the integration of alsS at the tdcDE locus. Colony PCR was performed to verify the clones using primers avtA-YZ-up/avtA-YZ-down. The correct colony amplification product was a 2914 bp fragment, which included a 416 bp upstream homology arm of the avtA locus, a 1089 bp serC gene, a 21 bp RBS sequence for the translation initiation of the serB gene and a 969 bp serB gene, and a 419 bp downstream homology arm of the avtA locus. A correct single colony was selected and named Span046.

Span046 is a recombinant strain obtained by integrating the phosphoserine/phosphohydroxythreonine aminotransferase gene serC and phosphoserine phosphatase gene serB from Escherichia coli (the serCB gene cluster, whose nucleotide sequence is SEQ ID NO: 174 in the sequence listing; the serCB gene cluster encodes the serC protein shown in SEQ ID NO: 27 and the serB protein shown in SEQ ID NO: 29) into the avtA locus of *Escherichia coli* Span044. In this recombinant strain, the avtA gene (encoding the protein sequence NCBI ACA75824.1, coded_by=CP000946.1:153868..155121) is simultaneously knocked out.

In SEQ ID NO: 174, positions 1-88 are the M1-93 promoter sequence, positions 89-1177 are the serC gene sequence, positions 1178-1198 are the RBS sequence for the translation initiation of the serB gene, and positions 1199-2167 are the serB gene sequence.

### Example 24: Expression regulation of the serCB gene cluster integrated at the avtA locus

Starting from Span046, the expression of the gene cluster consisting of phosphoserine/phosphohydroxythreonine aminotransferase gene serC and phosphoserine phosphatase gene serB derived from *Escherichia coli* integrated at the avtA locus was regulated using artificial regulatory elements. The specific steps were as follows:
In the first step, using pXZ-CS plasmid DNA as a template, a 2719 bp DNA fragment I was amplified with primers avtA-CS-up/serCB-ProCS-down, which was used for the first homologous recombination. The DNA fragment I included a 50 bp upstream homology arm of the avtA locus, a 2619 bp cat-sacB fragment, and a 50 bp downstream homology arm of the serC gene. The amplification system and amplification conditions were consistent with those described in Example 1. The DNA fragment I was electroporated into Span046.

The DNA fragment I was used for the first homologous recombination: first, the pKD46 plasmid was transformed into *Escherichia coli* Span046 by electroporation, and then the DNA fragment I was electroporated into *Escherichia coli* Span046 harboring pKD46.

The electroporation conditions and steps were consistent with those of the first-step method described in Example 1 for the integration of alsS at the tdcDE locus. 200 µl of the bacterial solution was taken and spread on an LB plate containing ampicillin (final concentration 100 µg/ml) and chloramphenicol (final concentration 34 µg/ml). After overnight culture at 30°C, single colonies were selected for PCR verification using primers avtA-YZ-up/serCB-YZ317-down. The correct PCR product should be 3456 bp, which includes a 416 bp upstream homology arm of the avtA locus, a 2619 bp cat-sacB fragment, and a 421 bp downstream homology arm of the serC gene. A correct single colony was selected and named Span047.

In the second step, using genomic DNA of M1-93 (Lu, et al., Appl Microbiol Biotechnol, 2012, 93: 2455-2462) as a template, a 188 bp DNA fragment II was amplified with primers serCB-Pro-up/serCB-Pro-down. The DNA fragment II included a 50 bp upstream homology arm of the avtA locus, an 88 bp M1-93 promoter sequence, and a 50 bp downstream homology arm of the serC gene. The DNA fragment II was used for the second homologous recombination. The DNA fragment II was electroporated into the strain Span047.

The electroporation conditions and steps were consistent with those of the second-step method described in Example 1 for the integration of alsS at the tdcDE locus. Colony PCR was performed to verify the clones using primers avtA-YZ-up/serCB-YZ317-down. The correct colony amplification product was a 925 bp fragment, which included a 416 bp upstream homology arm of the avtA locus, an 88 bp M1-93 promoter sequence, and a 421 bp downstream homology arm of the serC gene. A correct single colony was selected and named Span048.

Span048 is a recombinant strain obtained by integrating the M1-93 promoter upstream of the serCB gene cluster in *Escherichia coli* Span046. It contains the serCB gene cluster expression cassette shown in SEQ ID NO: 174. In this recombinant strain, the M1-93 promoter (whose nucleotide sequence is positions 1-88 of SEQ ID NO: 174 in the sequence listing) drives the expression of the serC and serB genes in the serCB gene cluster.

### Example 25: Knockout of L-serine deaminase I gene sdaA

Starting from Span048, the coding gene sdaA of L-serine deaminase I was knocked out. The specific steps were as follows:
In the first step, using pXZ-CS plasmid DNA as a template, a 2719 bp DNA fragment I was amplified with primers sdaA-delcat-up/sdaA-delsacB-down, which was used for the first homologous recombination. The DNA fragment I included a 50 bp upstream homology arm of the sdaA locus, a 2619 bp cat-sacB fragment, and a 50 bp downstream homology arm of the sdaA gene. The amplification system and amplification conditions were consistent with those described in Example 1. The DNA fragment I was electroporated into Span048.

The DNA fragment I was used for the first homologous recombination: first, the pKD46 plasmid was transformed into *Escherichia coli* Span048 by electroporation, and then the DNA fragment I was electroporated into *Escherichia coli* Span048 harboring pKD46.

The electroporation conditions and steps were consistent with those of the first-step method described in Example 1 for the integration of alsS at the tdcDE locus. 200 µl of the bacterial solution was taken and spread on an LB plate containing ampicillin (final concentration 100 µg/ml) and chloramphenicol (final concentration 34 µg/ml). After overnight culture at 30°C, single colonies were selected for PCR verification using primers sdaA-YZ-up/sdaA-YZ-down. The correct PCR product should be 3428 bp, which includes a 383 bp upstream homology arm of the sdaA locus, a 2619 bp cat-sacB fragment, and a 426 bp downstream homology arm of the sdaA gene. A correct single colony was selected and named Span049.

In the second step, using genomic DNA of *Escherichia coli* ATCC 8739 as a template, a 433 bp DNA fragment II was amplified with primers sdaA-YZ-up/SdaAdel-down. The DNA fragment II included a 383 bp upstream homology arm and a 50 bp downstream homology arm of sdaA. The DNA fragment II was used for the second homologous recombination. The DNA fragment II was electroporated into the strain Span049.

The electroporation conditions and steps were consistent with those of the second-step method described in Example 1 for the integration of alsS at the tdcDE locus. Colony PCR was performed to verify the clones using primers sdaA-YZ-up/sdaA-YZ-down. The correct colony amplification product was an 809 bp fragment, which included a 383 bp upstream homology arm of the sdaA locus and a 426 bp downstream homology arm of the sdaA gene. A correct single colony was selected and named Span050.

Span050 is a recombinant strain obtained by knocking out the L-serine deaminase I gene sdaA (encoding the protein sequence NCBI ACA77468.1, coded_by=CP000946.1:2018393..2019757) from *Escherichia coli* Span048. This recombinant strain does not contain the sdaA gene.

Span050 was deposited in the China General Microbiological Culture Collection Center on January 22, 2021, with a deposit number CGMCC No. 21699.

### Example 26: Production of pantoic acid using Span050

The seed medium consists of the following components (with water as the solvent):
Macronutrients: 20 g/L of glucose, 3.5 g/L of (NH4)2HPO4, 3.91 g/L of KH2PO4, 4.48 g/L of K2HPO4, 0.18 g/L of MgSO4·7H2O, and 0.15 g/L of betaine-HCl;
Micronutrients: 1.5 µ g/L of FeCl3·6H2O, 0.1 µ g/L of CoCl2·6H2O, 0.1 µ g/L of CuCl2·2H2O, 0.1 µg/L of ZnCl2, 0.1 µg/L of Na2MoO4·2H2O, 0.2 µg/L of MnCl2·4H2O, and 0.05 µg/L of H3BO3.

The fermentation medium is mostly the same as the seed medium, with the differences being that the glucose concentration is 50 g/L and the fermentation medium additionally contains 5 g/L of serine.

The fermentation of Span050 included the following steps:
(1) Seed culture: A fresh clone from the LB plate was inoculated into a test tube containing 4 ml of seed medium and cultured overnight at 37°C with shaking at 250 rpm. Then, the culture was transferred to a 250 ml Erlenmeyer flask containing 30 ml of seed medium at an inoculum size of 2% (V/V) and cultured at 37°C with shaking at 250 rpm for 12 hours to obtain a seed culture solution for inoculation of the fermentation medium.
(2) Fermentation culture: The volume of fermentation medium in a 250 ml Erlenmeyer flask was 25 ml. The seed culture solution was inoculated into the fermentation medium at an inoculum size corresponding to a final concentration of OD550=0.1, followed by fermentation at 37°C with shaking at 250 rpm for 60 hours to obtain a fermentation broth.

Analysis method: An Agilent (Agilent-1260) high-performance liquid chromatograph was used to determine the components in the fermentation broth after 3 days of fermentation. The concentrations of glucose and pantoic acid in the fermentation broth were determined using an Aminex HPX-87H organic acid analysis column from Bio-Rad.

Results showed that the Span050 strain was capable of producing 1.2 g/L of pantoic acid after 3 days of fermentation. It can thus be seen that the pantoic acid synthesis pathway in the Span050 strain had been established, and the accumulation of pantoic acid could be achieved during the fermentation process.

### Example 27: Fermentation of Span050 in a 5-L fermenter

The composition and formulation, and analysis method of the seed medium were the same as those described in Example 26.

Fermentation medium: 30 g/L of glucose, 5 g/L of magnesium sulfate, 10.5 g/L of potassium dihydrogen phosphate, 20 g/L of yeast extract, 6 g/L of diammonium hydrogen phosphate, and 1.84 g/L of citric acid monohydrate; the micronutrients were the same as those in the fermentation medium of Example 26, with water as the solvent.

Feed medium: 600 g/L of glucose, with water as the solvent.

Fermentation was carried out in a 5-L fermenter (Baoxing, Shanghai, Model: BIOTECH-5BG) and included the following steps:
(1) Seed culture: The seed medium in the 500-mL Erlenmeyer flask was 50 mL of, and was sterilized at 115°C for 15 min. After cooling, recombinant *Escherichia coli* Span050 was inoculated into the seed medium at an inoculum size of 1% (V/V), and cultured at 37°C with shaking at 250 rpm for 12 hours to obtain a seed solution for inoculation of the fermentation medium.
(2) Fermentation culture: The fermentation medium in the 5-L fermenter had a volume of 3 L, and was sterilized at 115°C for 25 min. The seed solution was inoculated into the fermentation medium at an inoculum size corresponding to a final concentration of OD550=0.2. The dissolved oxygen was maintained at 30%, ammonia water was used as a neutralizer to keep the pH at 7.0, and the glucose concentration inside the fermenter was controlled below 5 g/L through the feed medium. Culture was carried out at 37°C for 3 days to obtain the fermentation broth. The fermentation broth was all the substances inside the fermenter.

Results showed that after 3 days of fermentation by Span050, the yield of pantoic acid reached 22 g/L, indicating that the strain has good industrial application potential.

### Example 28: Construction of strain Span096

Starting from Span050, the NADPH-dependent IlvC from *Escherichia coli* (SEQ ID NO: 167) was replaced with an exogenous NADH-dependent acetohydroxy acid reductoisomerase KARI (SEQ ID NO: 9), and a new recombinant *Escherichia coli* strain Span096 capable of producing D-pantoic acid was obtained. The process of strain construction was as follows:
In the first step, using pXZ-CS plasmid (Tan, et al., Appl Environ Microbiol, 2013, 79:4838-4844) DNA as a template, a 2719 bp DNA fragment I was amplified with primers adhE-cs-up/adhE-cs-down, which was used for the first homologous recombination.

The amplification system was as follows: 10 µl of New England Biolabs Phusion 5X buffer, 1 µl of dNTP (each dNTP at 10 mM), 20 ng of DNA template, 2 µl of each primer (10 µM), 0.5 µl of Phusion High-Fidelity DNA polymerase (2.5 U/µl), and 33.5 µl of distilled water, with a total volume of 50 µl.

The amplification conditions were as follows: pre-denaturation at 98°C for 2 minutes (1 cycle); denaturation at 98°C for 10 seconds, annealing at 56°C for 10 seconds, extension at 72°C for 2 minutes (30 cycles); and extension at 72°C for 10 minutes (1 cycle).

The above DNA fragment I was used for the first homologous recombination: first, the pKD46 plasmid (Datsenko and Wanner 2000, Proc Natl Acad Sci USA 97:6640-6645; the plasmid was purchased from the CGSC *Escherichia coli* Stock Center at Yale University, USA, CGSC#7739) was transformed into Span050 by electroporation, and then the DNA fragment I was electroporated into Span050 harboring pKD46.

The electroporation conditions were as follows: first, electrocompetent cells of recombinant*Escherichia coli* Span050 harboring the pKD46 plasmid were prepared (Dower et al., 1988, Nucleic Acids Res 16:6127-6145); 50 µl of the competent cells were placed on ice, 50 ng of the DNA fragment I was added, and the mixture was left on ice for 2 minutes, and then transferred to a 0.2 cm Bio-Rad electroporation cuvette. A MicroPulser electroporator (Bio-Rad) was used with an electric shock parameter of 2.5 kV. Immediately after the electric shock, 1 ml of LB medium was transferred into the electroporation cuvette, pipetted 5 times, then transferred to a test tube, and incubated at 30°C for 2 hours with shaking at 75 rpm. 200 µl of the bacterial solution was taken and spread on an LB plate containing ampicillin (final concentration 100 µg/ml) and chloramphenicol (final concentration 34 µg/ml). After overnight incubation at 30°C, single colonies were selected for PCR verification using primers XZ-adhE-up/XZ-adhE-down. The correct colony amplification product was a 3167 bp fragment. A correct single colony was selected and named Span050-CS.

In the second step, the acetohydroxy acid reductoisomerase-encoding kari gene was integrated into the Span050-CS engineering strain. The acetohydroxy acid reductoisomerase-encoding kari gene was obtained by total gene synthesis after codon optimization based on the kari sequence derived from the *Thermacetogenium phaeum* strain reported in the literature (Brinkmann-Chen, S., Cahn, J.K.B. & Arnold, F.H. Uncovering rare NADH-preferring ketol-acid reductoisomerases. Metab Eng 26, 17-22, doi:10.1016/j.ymben.2014.08.003 (2014).) (SEQ ID NO: 10). During synthesis, an artificial regulatory element RBS5 (SEQ ID NO: 170) was added before the kari gene to initiate the expression of the kari gene. This construct was inserted into the pUC57 vector to construct the plasmid pUC57-RBS5-kari (gene synthesis and vector construction were performed by Nanjing GenScript Biotech Corporation). The RBS5 artificial regulatory element and the kari gene were co-integrated into the Span050-CS engineering strain to achieve the expression of the kari gene.

Using pUC57-RBS5-kari plasmid DNA as a template, a 1188 bp DNA fragment II was amplified with primers adhE-RBS5-up/adhE-kari-down. The DNA fragment II was used for the second homologous recombination. The DNA fragment II was electroporated into Span050-CS. The electroporation conditions were as follows: first, electrocompetent cells of Span050-CS harboring the pKD46 plasmid were prepared using the same procedure as in the first step. Then, 50 µl of the competent cells were placed on ice, 50 ng of the DNA fragment II was added, and the mixture was left on ice for 2 minutes, and then transferred to a 0.2 cm Bio-Rad electroporation cuvette. A MicroPulser electroporator (Bio-Rad) was used with an electric shock parameter of 2.5 kV. Immediately after the electric shock, 1 ml of LB medium was transferred into the electroporation cuvette, pipetted 5 times, then transferred to a test tube, and incubated at 30°C for 4 hours with shaking at 75 rpm. The bacterial solution was transferred to LB liquid medium containing 10% sucrose and no sodium chloride. After 24 hours of culture, streaking and culture were performed on LB solid medium containing 6% sucrose and no sodium chloride. PCR verification was performed using XZ-adhE-up/XZ-adhE-down. The correct colony amplification product was a 1454 bp fragment. The PCR products were sequenced, and a correct single colony was selected and named Span096.

The strain Span096 was deposited in the China General Microbiological Culture Collection Center (CGMCC) with a deposit number CGMCC No. 26276.

### Example 29: Production of pantoic acid using Span096

The seed medium consists of the following components (with water as the solvent):
Macronutrients: 20 g/L of glucose, 3.5 g/L of (NH4)2HPO4, 3.91 g/L of KH2PO4, 4.48 g/L of K2HPO4, 0.18 g/L of MgSO4·7H2O, and 0.15 g/L of betaine-HCl;
Micronutrients: 1.5 µ g/L of FeCl3·6H2O, 0.1 µ g/L of CoCl2·6H2O, 0.1 µ g/L of CuCl2·2H2O, 0.1 µg/L of ZnCl2, 0.1 µg/L of Na2MoO4·2H2O, 0.2 µg/L of MnCl2·4H2O, and 0.05 µg/L of H3BO3.

The fermentation medium is mostly the same as the seed medium, with the differences being that the glucose concentration is 50 g/L and the fermentation medium additionally contains 5 g/L of serine.

The fermentation of Span096 included the following steps:
(1) Seed culture: A fresh clone from the LB plate was inoculated into a test tube containing 4 ml of seed medium and cultured overnight at 37°C with shaking at 250 rpm. Then, the culture was transferred to a 250 ml Erlenmeyer flask containing 30 ml of seed medium at an inoculum size of 2% (V/V) and cultured at 37°C with shaking at 250 rpm for 12 hours to obtain a seed culture solution for inoculation of the fermentation medium.
(2) Fermentation culture: The volume of fermentation medium in a 250 ml Erlenmeyer flask was 25 ml. The seed culture solution was inoculated into the fermentation medium at an inoculum size corresponding to a final concentration of OD550=0.1, followed by fermentation at 37°C with shaking at 250 rpm for 60 hours to obtain a fermentation broth.

Analysis method: An Agilent (Agilent-1260) high-performance liquid chromatograph was used to determine the components in the fermentation broth after 3 days of fermentation. The concentrations of glucose and pantoic acid in the fermentation broth were determined using an Aminex HPX-87H organic acid analysis column from Bio-Rad.

Results showed that after 3 days of fermentation, the strain Span096 was able to produce 1.8 g/L of pantoic acid, and the pantoic acid yield was increased by 50% compared with that of the original strain Span050.

### Example 30: Fermentation of Span096 in a 5-L fermenter

The composition and formulation, and analysis method of the seed medium were the same as those described in Example 2.

Fermentation medium: 30 g/L of glucose, 5 g/L of magnesium sulfate, 10.5 g/L of potassium dihydrogen phosphate, 20 g/L of yeast extract, 6 g/L of diammonium hydrogen phosphate, and 1.84 g/L of citric acid monohydrate; the micronutrients were the same as those in the fermentation medium of Example 26, with water as the solvent.

Feed medium: 600 g/L of glucose, with water as the solvent.

Fermentation was carried out in a 5-L fermenter (Baoxing, Shanghai, Model: BIOTECH-5BG) and included the following steps:
(1) Seed culture: The seed medium in the 500-mL Erlenmeyer flask was 50 mL of, and was sterilized at 115°C for 15 min. After cooling, recombinant *Escherichia coli* Span096 was inoculated into the seed medium at an inoculum size of 1% (V/V), and cultured at 37°C with shaking at 250 rpm for 12 hours to obtain a seed solution for inoculation of the fermentation medium.
(2) Fermentation culture: The fermentation medium in the 5-L fermenter had a volume of 3 L, and was sterilized at 115°C for 25 min. The seed solution was inoculated into the fermentation medium at an inoculum size corresponding to a final concentration of OD550=0.2. The dissolved oxygen was maintained at 30%, ammonia water was used as a neutralizer to keep the pH at 7.0, and the glucose concentration inside the fermenter was controlled below 5 g/L through the feed medium. Culture was carried out at 37°C for 3 days to obtain the fermentation broth. The fermentation broth was all the substances inside the fermenter.

Results showed that after 3 days of fermentation by Span096, the yield of pantoic acid reached 31 g/L, indicating that the strain has good industrial application potential.

### Sequence Information:

Acetolactate synthase (alsS) from *Bacillus subtilis,* amino acid sequence (SEQ ID NO: 1): 
MLTKATKEQKSLVKNRGAELVVDCLVEQGVTHVFGIPGAKIDAVFDALQDKGPEIIVARHEQNAAFMAQAVGRLTG
KPGVVLVTSGPGASNLATGLLTANTEGDPVVALAGNVIRADRLKRTHQSLDNAALFQPITKYSVEVQDVKNIPEAVT
NAFRIASAGQAGAAFVSFPQDVVNEVTNTKNVRAVAAPKLGPAADDAISAAIAKIQTAKLPVVLVGMKGGRPEAIK
AVRKLLKKVQLPFVETYQAAGTLSRDLEDQYFGRIGLFRNQPGDLLLEQADVVLTIGYDPIEYDPKFWNINGDRTIIH
LDEIIADIDHAYQPDLELIGDIPSTINHIEHDAVKVEFAEREQKILSDLKQYMHEGEQVPADWKSDRAHPLEIVKELRN
AVDDHVTVTCDIGSHAIWMSRYFRSYEPLTLMISNGMQTLGVALPWAIGASLVKPGEKVVSVSGDGGFLFSAMELE
TAVRLKAPIVHIVWNDSTYDMVAFQQLKKYNRTSAVDFGNIDIVKYAESFGATGLRVESPDQLADVLRQGMNAEGP
VIIDVPVDYSDNINLASDKLPKEFGELMKTKAL
Acetolactate synthase (alsS) from *Bacillus subtilis,* nucleotide sequence (SEQ ID NO: 2): 
atgttgacaaaagcaacaaaagaacaaaaatcccttgtgaaaaacagaggggcggagcttgttgttgattgcttagtggagcaaggtgtcacacatgtatttggcattccaggtgcaaa
aattgatgcggtatttgacgctttacaagataaaggacctgaaattatcgttgcccggcacgaacaaaacgcagcattcatggcccaagcagtcggccgtttaactggaaaaccggga
 
gtcgtgttagtcacatcaggaccgggtgcctctaacttggcaacaggcctgctgacagcgaacactgaaggagaccctgtcgttgcgcttgctggaaacgtgatccgtgcagatcgttt
aaaacggacacatcaatctttggataatgcggcgctattccagccgattacaaaatacagtgtagaagttcaagatgtaaaaaatataccggaagctgttacaaatgcatttaggatagc
gtcagcagggcaggctggggccgcttttgtgagctttccgcaagatgttgtgaatgaagtcacaaatacgaaaaacgtgcgtgctgttgcagcgccaaaactcggtcctgcagcagat
gatgcaatcagtgcggccatagcaaaaatccaaacagcaaaacttcctgtcgttttggtcggcatgaaaggcggaagaccggaagcaattaaagcggttcgcaagcttttgaaaaag
gttcagcttccatttgttgaaacatatcaagctgccggtaccctttctagagatttagaggatcaatattttggccgtatcggtttgttccgcaaccagcctggcgatttactgctagagcag
gcagatgttgttctgacgatcggctatgacccgattgaatatgatccgaaattctggaatatcaatggagaccggacaattatccatttagacgagattatcgctgacattgatcatgcttac
cagcctgatcttgaattgatcggtgacattccgtccacgatcaatcatatcgaacacgatgctgtgaaagtggaatttgcagagcgtgagcagaaaatcctttctgatttaaaacaatatat
gcatgaaggtgagcaggtgcctgcagattggaaatcagacagagcgcaccctcttgaaatcgttaaagagttgcgtaatgcagtcgatgatcatgttacagtaacttgcgatatcggttc
gcacgccatttggatgtcacgttatttccgcagctacgagccgttaacattaatgatcagtaacggtatgcaaacactcggcgttgcgcttccttgggcaatcggcgcttcattggtgaaa
ccgggagaaaaagtggtttctgtctctggtgacggcggtttcttattctcagcaatggaattagagacagcagttcgactaaaagcaccaattgtacacattgtatggaacgacagcaca
tatgacatggttgcattccagcaattgaaaaaatataaccgtacatctgcggtcgatttcggaaatatcgatatcgtgaaatatgcggaaagcttcggagcaactggcttgcgcgtagaat
caccagaccagctggcagatgttctgcgtcaaggcatgaacgctgaaggtcctgtcatcatcgatgtcccggttgactacagtgataacattaatttagcaagtgacaagcttccgaaa
gaattcggggaactcatgaaaacgaaagctctctag
Acetolactate synthase I from *Escherichia coli,* amino acid sequence (SEQ ID NO: 3): 
MASSGTTSTRKRFTGAEFIVHFLEQQGIKIVTGIPGGSILPVYDALSQSTQIRHILARHEQGAGFIAQGMARTDGKPAV
CMACSGPGATNLVTAIADARLDSIPLICITGQVPASMIGTDAFQEVDTYGISIPITKHNYLVRHIEELPQVMSDAFRIAQ
SGRPGPVWIDIPKDVQTAVFEIEAQPAVAEKAAAPAFSEESIRDAATMINAAKRPVLYLGGGVINAPARVRELAEKAQ
LPTTMTLMALGMLPKAHPLSLGMLGMHGVRSTNYILQEADLLIVLGARFDDRAIGKTEQFCPNAKIIHVDIDRAEL
GKIKQPHVAIQADVDDVLAQLIPLVEAQPRAEWHQLVADLQREFPCPIPKACDPLSHYGLINAVAACVDDNAIITTDV
GQHQMWTAQAYPLNRPRQWLTSGGLGTMGFGLPAAIGAALANPDRKVLCFSGDGSLMMNIQEMATASENQLDVK
IILMNNEALGLVHQQQSLFYEQGVFAATYPGKINFMQIAAGFGLETCDLNNEADPQAALQEIINRPGPALIHVRIDAE
EKVYPMVPPGAANTEMVGE
Acetolactate synthase I from *Escherichia coli,* nucleotide sequence (SEQ ID NO: 4): 
atggcaagttcgggcacaacatcgacgcgtaagcgctttaccggcgcagaatttatcgttcatttcctggaacagcagggcattaagattgtgacgggcattccgggcggttctatcct
gcctgtttacgatgccttaagccaaagtacgcaaatccgccatattctggctcgccatgaacagggcgcgggatttatcgctcagggaatggcgcgcaccgacggtaaaccggcggt
ctgtatggcctgtagcggaccgggtgcgactaacctggtgaccgccattgccgatgcgcggctggactccatcccgctgatttgcatcactggtcaggttcccgcctcgatgatcggc
accgacgccttccaggaagtcgacacctacggcatctctatccccatcaccaaacacaactatctggtcagacatatcgaagaactcccgcaggtcatgagcgatgccttccgcattg
cgcaatcaggccgcccaggcccggtgtggatagacattcctaaggatgtgcaaacggcggtttttgagattgaagctcagcccgcggtggcagaaaaagccgctgcacccgccttt
agcgaagaaagcattcgtgacgcagctacaatgattaacgctgccaaacgcccggtgctttatctgggtggtggtgtgatcaatgcgcctgcgcgggtgcgtgaactggcggagaaa
gcgcaactgcctaccaccatgactttaatggcgctgggcatgctgccaaaagcgcatccgttgtcgctgggtatgctggggatgcacggcgtgcgcagcactaactatatcttgcagg
aggcggatttactgattgtgctcggtgcgcgttttgatgaccgggcgattggcaaaaccgagcagttctgtccgaatgccaaaatcattcatgtcgatatcgaccgtgcagagctgggta
aaatcaagcagccgcatgtggcgattcaggcggatgttgatgacgtgctggcgcagttgatcccgctggtggaagcgcaaccgcgtgcagagtggcaccagttggtagcggatttg
cagcgtgagtttccgtgtccaatcccgaaagcgtgcgatccattaagccattacggcctgatcaacgccgttgccgcctgtgtcgatgacaatgcgattatcaccaccgatgtggggca
gcatcagatgtggaccgcgcaagcttatccgctcaatcgcccacgccagtggctgacctccggtgggctgggcacgatgggttttggcctgcctgcggcgattggcgcggcgctgg
cgaacccggatcgcaaagtgttgtgtttctccggcgacggcagcctgatgatgaatattcaggagatggcgaccgccagtgaaaatcagctggatgtcaaaatcattctgatgaacaa
cgaagcgctggggctggtgcatcagcaacagagtctgttctacgagcaaggcgtttttgccgccacctatccgggcaaaatcaactttatgcagattgccgccggattcggcctcgaa
acctgtgatttgaataacgaagccgatccgcaggctgcattgcaggaaatcatcaatcgccctggcccggcgctgatccatgtgcgcattgatgccgaagaaaaagtttacccgatgg
tgccgccaggtgcggcgaatactgaaatggtgggggaataa
Acetolactate synthase II from *Escherichia coli,* amino acid sequence (SEQ ID NO: 5): 
MNGAQWVVHALRAQGVNTVFGYPGGAIMPVYDALYDGGVEHLLCRHEQGAAMAAIGYARATGKTGVCIATSGP
GATNLITGLADALLDSIPVVAITGQVSAPFIGTDAFQEVDVLGLSLACTKHSFLVQSLEELPRIMAEAFDVASSGRPGP
VLVDIPKDIQLASGDLEPWFTTVENEVTFPHAEVEQARQMLAKAQKPMLYVGGGVGMAQAVPALREFLATTKMPA
TCTLKGLGAVEADYPYYLGMLGMHGTKAANFAVQECDLLIAVGARFDDRVTGKLNTFAPHASVIHMDIDPAEMNK
LRQAHVALQGDLNALLPALQQPLNINDWQQHCAQLRDEHAWRYDHPGDAIYAPLLLKQLSDRKPADCVVTTDVG
QHQMWAAQHIAHTRPENFITSSGLGTMGFGLPAAVGAQVARPNDTVVCISGDGSFMMNVQELGTVKRKQLPLKIV
LLDNQRLGMVRQWQQLFFQERYSETTLTDNPDFLMLASAFGIPGQHITRKDQVEAALNTMLNSDGPYLLHVSIDEL
 ENVWPLVPPGASNSEMLEKLS
Acetolactate synthase II from *Escherichia coli,* nucleotide sequence (SEQ ID NO: 6): 
atgaatggcgcacagtgggtggtacatgcgttgcgggcacagggtgtgaataccgttttcggttatccgggtggcgcaattatgccggtttacgatgcattgtatgacggcggcgtgga
gcacttgctgtgccgacatgaacagggtgcggcaatggcggctatcggttatgcccgtgctactggcaaaactggcgtatgtatcgccacgtctggtccgggcgcaaccaacctgat
aaccgggcttgcggacgcactgttagattccatccccgttgttgccatcaccggtcaagtgtccgcaccgtttatcggcacggacgcatttcaggaagtggatgtcctgggattgtcgct
agcctgtaccaagcacagcttcctggtgcagtcgctggaagagttgccgcgcatcatggctgaagcattcgacgttgccagctcaggtcgtcctggtccggttctggtcgatatcccaa
aagatatccaattagccagcggcgacctggaaccgtggttcaccaccgttgaaaacgaagtgactttcccacatgccgaagtcgagcaagcgcgccagatgctggcaaaagcgca
aaaaccgatgctgtacgttggtggtggcgtgggtatggcgcaggcagttcctgctttacgagaatttctcgctaccacaaaaatgcctgccacctgcacgctgaaagggctgggcgca
gttgaagcagattatccgtactatctgggcatgctgggaatgcatggcaccaaagcggcgaacttcgcggtgcaggagtgcgacttgctgatcgccgtgggtgcacgttttgatgacc
gggtgaccggcaaactgaacaccttcgcaccacacgccagtgttatccatatggatatcgacccggcagaaatgaacaagctgcgtcaggcacatgtggcattacaaggtgatttaa
atgctctgttaccagcattacagcagccgttaaatatcaatgactggcagcaacactgcgcgcagctgcgtgatgaacatgcctggcgttacgaccatcccggtgacgctatctacgcg
ccgttgttgttaaaacaactgtcggatcgtaaacctgcggattgcgtcgtgaccacagatgtggggcagcaccagatgtgggctgcgcagcacatcgcccacactcgcccggaaaat
ttcatcacctccagcggcttaggtaccatgggttttggtttaccggcggcggttggcgcacaagtcgcgcgaccgaacgataccgttgtctgtatctccggtgacggctctttcatgatg
aatgtgcaagagctgggcaccgtaaaacgcaagcagttaccgttgaaaatcgtcttactcgataaccaacggttagggatggttcgacaatggcagcaactgtttttcaggaacgata
cagcgaaaccacccttactgataaccccgatttcctcatgttagccagcgccttcggcatccctggccaacacatcacccgtaaagaccaggttgaagcggcactcaacaccatgctg
aacagtgatgggccatacctgcttcatgtctcaatcgacgaacttgagaacgtctggccgctggtgccgccaggtgccagtaattcagaaatgttggagaaattatcatga
L-valine feedback-resistant acetolactate synthase III, amino acid sequence (SEQ ID NO: 7): 
MRRILSVLLENESDALFRVIGLFSQRGYNIESLTVAPTDDPTLSRMTIQTVGDEKVLEQIEKQLHKLVDVLRVSELGQ
GAHVEREIMLVKIQASGYGRDEVKRNTEIFRGQIIDVTPSLYTVQLAGTSGKLDAFLASIRDVAKIVEVARSGVVGLS
RGDKIMR
L-valine feedback-resistant acetolactate synthase III, nucleotide sequence (SEQ ID NO: 8): 
atgcgccggatattatcagtcttactcgaaaatgaatcagacgcgttattccgcgtgattggcctttttcccagcgtggctacaacattgaaagcctgaccgttgcgccaaccgacgatc
cgacattatcgcgtatgaccatccagaccgtgggcgatgaaaaagtacttgagcagatcgaaaagcaattacacaagctggtcgatgtcttgcgcgtgagtgagttggggcagggcg
cgcatgttgagcgggaaatcatgctggtgaaaattcaggccagcggttacgggcgtgacgaagtgaaacgtaatacggaaatattccgtgggcaaattatcgatgtcacaccctcgct
ttataccgttcaattagcaggcaccagcggtaagcttgatgcatttttagcatcgattcgcgatgtggcgaaaattgtggaggttgctcgctctggtgtggtcggactttcgcgcggcgat
aaaataatgcgttga
NADH-dependent acetohydroxy acid reductoisomerase, amino acid sequence (SEQ ID NO: 9): 
MKIYYDQDADLQYLDGKTVAVIGYGSQGHAQSQNLRDSGVKVVVADIPSSENWKKAEEAQFQPLTADEAAREADII
QILVPDEKQAALYRESIAPNLRPGKALVFSHGFNIHFKQIVPPPDVDVFMVAPKGPGHLVRRMYEEGAGVPSLVAVEQ
DYSGQALNLALAYAKGIGATRAGVIQTTFKEETETDLFGEQAVLCGGITELIRAGFDTLVDAGYQPEIAYFECLHEMK
LIVDLIYEGGISTMRYSISDTAEYGDLTRGKRIITEATREEMKKILKEIQDGVFAREWLLENQVGRPVYNALRRKEQN
HLIETVGARLRGMMPWLKKKVI
NADH-dependent acetohydroxy acid reductoisomerase, nucleotide sequence (SEQ ID NO: 10): 
atgaaaatctattacgatcaggacgcggatctgcaatatctggatggcaaaaccgtggctgttatcggctacggttcacagggccatgcgcagtcgcaaaatctgcgtgacagc
ggtgttaaagtggttgtcgcggatattccgagctctgaaaactggaaaaaagctgaagaagcgcagttccaaccgctgacggctgacgaagcagcccgcgaagcggatattatcca
gattctggtgccggatgaaaaacaagcagctctgtatcgtgaatcaatcgccccgaatctgcgcccgggcaaagcactggtgtttagccacggcttcaacattcactttaaacagatcg
tgccgccgccggacgtcgatgtgtttatggtcgcaccgaaaggtccgggtcacctggtgcgtcgcatgtacgaagaaggcgccggtgttccgtctctggttgcagtcgaacaggact
atagtggtcaagccctgaatctggcgctggcctacgcaaaaggcattggtgccacccgtgcaggcgtcatccagaccacgttcaaagaagaaaccgaaaccgacctgtttggtgaa
caagccgtcctgtgcggcggtattaccgaactgatccgcgcaggcttcgacaccctggtggatgctggttatcagccggaaattgcgtactttgaatgtctgcatgaaatgaaactgatt
gttgacctgatctatgaaggcggtatttccaccatgcgttatagtatctccgacaccgctgaatacggcgatctgacgcgtggtaaacgcattatcaccgaagcgacgcgcgaagaaat
gaagaaaattctgaaagaaatccaggatggcgtgttcgcccgtgaatggctgctggaaaaccaagtgggtcgcccggtttataatgccctgcgtcgcaaagaacagaaccacctgat
tgaaaccgtgggcgcacgtctgcgcggtatgatgccgtggctgaagaaaaaagttatctaa
Dihydroxy acid dehydratase, amino acid sequence (SEQ ID NO: 11): 
MPKYRSATTTHGRNMAGARALWRATGMTDADFGKPIIAVVNSFTQFVPGHVHLRDLGKLVAEQIEAAGGVAKEFN
TIAVDDGIAMGHGGMLYSLPSRELIADSVEYMVNAHCADAMVCISNCDKITPGMLMASLRLNIPVIFVSGGPMEAG
KTKLSDQIIKLDLVDAMIQGADPKVSDSQSDQVERSACPTCGSCSGMFTANSMNCLTEALGLSQPGNGSLLATHAD
RKQLFLNAGKRIVELTKRYYEQNDESALPRNIASKAAFENAMTLDIAMGGSTNTVLHLLAAAQEAEIDFTMSDIDK
LSRKVPQLCKVAPSTQKYHMEDVHRAGGVIGILGELDRAGLLNRDVKNVLGLTLPQTLEQYDVMLTQDDAVKNMF
RAGPAGIRTTQAFSQDCRWDTLDDDRANGCIRSLEHAYSKDGGLAVLYGNFAENGCIVKTAGVDDSILKFTGPAKV
YESQDDAVEAILGGKVVAGDVVVIRYEGPKGGPGMQEMLYPTSFLKSMGLGKACALITDGRFSGGTSGLSIGHVSP
EAASGGSIGLIEDGDLIAIDIPNRGIQLQVSDAELAARREAQDARGDKAWTPKNRERQVSFALRAYASLATSADKGA
VRDKSKLGG
Dihydroxy acid dehydratase, nucleotide sequence (SEQ ID NO: 12): 
atgcctaagtaccgttccgccaccaccactcatggtcgtaatatggcgggtgctcgtgcgctgtggcgcgccaccggaatgaccgacgccgatttcggtaagccgattatcgcggttg
tgaactcgttcacccaatttgtaccgggtcacgtccatctgcgcgatctcggtaaactggtcgccgaacaaattgaagcggctggcggcgttgccaaagagttcaacaccattgcggtg
gatgatgggattgccatgggccacggggggatgctttattcactgccatctcgcgaactgatcgctgattccgttgagtatatggtcaacgcccactgcgccgacgccatggtctgcat
ctctaactgcgacaaaatcaccccggggatgctgatggcttccctgcgcctgaatattccggtgatctttgtttccggcggcccgatggaggccgggaaaaccaaactttccgatcaga
tcatcaagctcgatctggttgatgcgatgatccagggcgcagacccgaaagtatctgactcccagagcgatcaggttgaacgttccgcgtgtccgacctgcggttcctgctccgggat
gtttaccgctaactcaatgaactgcctgaccgaagcgctgggcctgtcgcagccgggcaacggctcgctgctggcaacccacgccgaccgtaagcagctgttccttaatgctggtaa
acgcattgttgaattgaccaaacgttattacgagcaaaacgacgaaagtgcactgccgcgtaatatcgccagtaaggcggcgtttgaaaacgccatgacgctggatatcgcgatgggt
ggatcgactaacaccgtacttcacctgctggcggcggcgcaggaagcggaaatcgacttcaccatgagtgatatcgataagctttcccgcaaggttccacagctgtgtaaagttgcgc
cgagcacccagaaataccatatggaagatgttcaccgtgctggtggtgttatcggtattctcggcgaactggatcgcgcggggttactgaaccgtgatgtgaaaaacgtacttggcctg
acgttgccgcaaacgctggaacaatacgacgttatgctgacccaggatgacgcggtaaaaaatatgttccgcgcaggtcctgcaggcattcgtaccacacaggcattctcgcaagatt
gccgttgggatacgctggacgacgatcgcgccaatggctgtatccgctcgctggaacacgcctacagcaaagacggcggcctggcggtgctctacggtaactttgcggaaaacgg
ctgcatcgtgaaaacggcaggcgtcgatgacagcatcctcaaattcaccggcccggcgaaagtgtacgaaagccaggacgatgcggtagaagcgattctcggcggtaaagttgtc
gccggagatgtggtagtaattcgctatgaaggcccgaaaggcggtccggggatgcaggaaatgctctacccaaccagcttcctgaaatcaatgggtctcggcaaagcctgtgcgct
gatcaccgacggtcgtttctctggtggcacctctggtctttccatcggccacgtctcaccggaagcggcaagcggcggcagcattggcctgattgaagatggtgacctgatcgctatc
gacatcccgaaccgtggcattcagttacaggtaagcgatgccgaactggcggcgcgtcgtgaagcgcaggacgctcgaggtgacaaagcctggacgccgaaaaatcgtgaacgt
caggtctcctttgccctgcgtgcttatgccagcctggcaaccagcgccgacaaaggcgcggtgcgcgataaatcgaaactggggggttaa
3-Methyl-2-oxobutanoate hydroxymethyltransferase from *Corynebacterium glutamicum,* amino acid sequence (SEQ ID NO: 13): 
MPMSGIDAKKIRTRHFREAKVNGQKVSVLTSYDALSARIFDEAGVDMLLVGDSAANVVLGRDTTLSITLDEMIVLA
KAVTIATKRALVVVDLPFGTYEVSPNQAVESAIRVMRETGAAAVKIEGGVEIAQTIRRIVDAGIPVVGHIGYTPQSEHS
LGGHVVQGRGASSGKLIADARALEQAGAFAVVLEMVPAEAAREVTEDLSITTIGIGAGNGTDGQVLVWQDAFGLN
RGKKPRFVREYATLGDSLHDAAQAYIADIHAGTFPGEAESF
3-Methyl-2-oxobutanoate hydroxymethyltransferase from *Corynebacterium glutamicum,* nucleotide sequence (SEQ ID NO: 14): 
atgcccatgtcaggcattgatgcaaagaaaatccgcacccgtcatttccgcgaagctaaagtaaacggccagaaagtttcggttctcaccagctatgatgcgctttcggcgcgcattttt
gatgaggctggcgtcgatatgctccttgttggtgattccgctgccaacgttgtgctgggtcgcgataccaccttgtcgatcaccttggatgagatgattgtgctggccaaggcggtgacg
atcgctacgaagcgtgcgcttgtggtggttgatctgccgtttggtacctatgaggtgagcccaaatcaggcggtggagtccgcgatccgggtcatgcgtgaaacgggtgcggctgcg
gtgaagatcgagggtggcgtggagatcgcgcagacgattcgacgcattgttgatgctggaattccggttgtcggccacatcgggtacaccccgcagtccgagcattccttgggcggc
cacgtggttcagggtcgtggcgcgagttctggaaagctcatcgccgatgcccgcgcgttggagcaggcgggtgcgtttgcggttgtgttggagatggttccagcagaggcagcgcg
cgaggttaccgaggatctttccatcaccactatcggaatcggtgccggcaatggcacagatgggcaggttttggtgtggcaggatgccttcggcctcaaccgcggcaagaagccac
gcttcgtccgcgagtacgccaccttgggcgattccttgcacgacgccgcgcaggcctacatcgccgatatccacgcgggtaccttcccaggcgaagcggagtcctttttaa
3-Methyl-2-oxobutanoate hydroxymethyltransferase from *Escherichia coli,* amino acid sequence (SEQ ID NO: 15): MKPTTISLLQKYKQEKKRFATITAYDYSFAKLFADEGLNVMLVGDSLGMTVQGHDSTLPVTVADIAYHTAAVRRGA 
PNCLLLADLPFMAYATPEQAFENAATVMRAGANMVKIEGGEWLVETVQMLTERAVPVCGHLGLTPQSVNIFGGYK
VQGRGDEAGDQLLSDALALEAAGAQLLVLECVPVELAKRITEALAIPVIGIGAGNVTDGQILVMHDAFGITGGHIPK
FAKNFLAETGDIRAAVRQYMAEVESGVYPGEEHSFH
3-Methyl-2-oxobutanoate hydroxymethyltransferase from *Escherichia coli,* nucleotide sequence (SEQ ID NO: 16): 
ttaatggaaactgtgttcttcgcccggataaacgccggactccacttcagccatatactgccgcacagccgcgcggatgtcgcccgtttcggcgaggaaatttttagcgaatttaggaat
gtgaccgccggtaataccaaaggcgtcgtgcatcacgaggatctgcccgtcagtgacgttgcctgcgccaatgccaataaccgggatcgccagtgcttcggtaatacgttttgccagt
tcaaccggcacgcattccagcaccagcagctgtgccccagcagcttctaaggctaatgcatcgctgagcagttgatcgcccgcttcatcgccgcgcccctgaactttgtagccaccga
aaatattcactgactgtggtgttaaacctaagtgaccacatacaggaacggcacgttcggtcagcatttgtacggtttctaccagccactcaccgccttcaattttgaccatgttagcaccg
gcacgcataaccgttgcggcgttttcgaaggcttgttccggcgtggcatacgccataaacggcaggtcagccagcagcaggcagtttggtgcgccgcgacgtacggcggcagtgtg
gtaggcgatatcggcaacggtaactggcagggtggagtcgtgcccctgaaccgtcatgcccagcgaatcgcccaccagcatgacgttaagcccttcatcagcaaagagtttggcga
agctatagtcataagcggtgatggtcgcgaaacgtttttttcctgtttgtacttctgcagtaaggagatggtggtcggtttcat
2-Dehydropantoate-2-reductase, amino acid sequence (SEQ ID NO: 17): 
MKITVLGCGALGQLWLTALCKQGHEVQGWLRVPQPYCSVNLVETDGSIFNESLTANDPDFLATSDLLLVTLKAWQV
SDAVKSLASTLPVTTPILLIHNGMGTIEELQNIQQPLLMGTTTHAARRDGNVIIHVANGITHIGPARQQDGDYSYLADI
LQTVLPDVAWHNNIRAELWRKLAVNCVINPLTAIWNCPNGELRHHPQEIMQICEEVAAVIEREGHHTSAEDLRDYVM
QVIDATAENISSMLQDIRALRHTEIDYINGFLLRRARAHGIAVPENTRLFEMVKRKESEYERIGTGLPRPW
2-Dehydropantoate-2-reductase, nucleotide sequence (SEQ ID NO: 18): 
atgaaaattaccgtattgggatgcggtgccttagggcaattatggcttacagcactttgcaaacagggtcatgaagttcagggctggctgcgcgtaccgcaaccttattgtagcgtgaat
ctggttgagacagatggttcgatatttaacgaatcgctgaccgccaacgatcccgattttctcgccaccagcgatctgctcctggtgacgctgaaagcatggcaggtttccgatgccgtc
aaaagcctcgcgtccacactgcctgtaactacgccaatactgttaattcacaacggcatgggcaccatcgaagagttgcaaaacattcagcagccattactgatgggcaccaccaccc
atgcagcccgccgcgacggcaatgtcattattcatgtggcaaacggtatcacgcatattggcccggcacggcaacaggacggggattacagttatctggcggatattttgcaaaccgt
gttgcctgacgttgcctggcataacaatattcgcgccgagctgtggcgcaagctggcagtcaactgcgtgattaatccactgactgccatctggaattgcccgaacggtgaattacgtc
atcatccgcaagaaattatgcagatatgcgaagaagtcgcggcggtgatcgaacgcgaagggcatcatacttcagcagaagatttgcgtgattacgtgatgcaggtgattgatgccac
agcggaaaatatctcgtcgatgttgcaggatatccgcgcgctgcgccacactgaaatcgactatatcaatggttttctcttacgccgcgcccgcgcgcatgggattgccgtaccggaaa
acacccgcctgtttgaaatggtaaaaagaaaggagagtgaatatgagcgcatcggcactggtttgcctcgcccctggtag
Serine hydroxymethyltransferase, amino acid sequence (SEQ ID NO: 19): 
MLKREMNIADYDAELWQAMEQEKVRQEEHIELIASENYTSPRVMQAQGSQLTNKYAEGYPGKRYYGGCEYVDIVE
QLAIDRAKELFGADYANVQPHSGSQANFAVYTALLEPGDTVLGMNLAHGGHLTHGSPVNFSGKLYNIVPYGIDATG
HIDYADLEKQAKEHKPKMIIGGFSAYSGVVDWAKMREIADSIGAYLFVDMAHVAGLVAAGVYPNPVPHAHVVTTT
THKTLAGPRGGLILAKGGSEELYKKLNSAVFPGGQGGPLMHVIAGKAVALKEAMEPEFKTYQQQVAKNAKAMVEV
FLERGYKVVSGGTDNHLFLVDLVDKNLTGKEADAALGRANITVNKNSVPNDPKSPFVTSGIRVGTPAITRRGFKEAE
AKELAGWMCDVLDSINDEAVIERIKGKVLDICARYPVYA
Serine hydroxymethyltransferase, nucleotide sequence (SEQ ID NO: 20): 
atgttaaagcgtgaaatgaacattgccgattatgatgccgaactgtggcaggctatggagcaggaaaaagtacgtcaggaagagcacatcgaactgatcgcctccgaaaactacacc
agcccgcgcgtaatgcaggcgcagggttctcagctgaccaacaaatatgctgaaggttatccgggcaaacgctactacggcggttgcgagtatgttgatatcgttgaacaactggcg
atcgatcgtgcgaaagaactgttcggcgctgactacgctaacgtccagccgcactccggctcccaggctaactttgcggtctacaccgcgctgctggaaccaggtgataccgttctgg
gtatgaacctggcgcatggcggtcacctgactcacggttctccggttaacttctccggtaaactgtacaacatcgttccttacggtatcgatgctaccggtcatatcgactacgccgatct
ggaaaaacaagccaaagaacacaagccgaaaatgattatcggtggtttctctgcatattccggcgtggtggactgggcgaaaatgcgtgaaatcgctgacagcatcggtgcttacctg
ttcgttgatatggcgcacgttgcgggcctggttgctgctggcgtctacccgaacccggttcctcatgctcacgttgttactaccaccactcacaaaaccctggcgggtccgcgcggcgg
cctgatcctggcgaaaggtggtagcgaagagctgtacaaaaaactgaactctgccgttttccctggtggtcagggcggtccgttgatgcacgtaatcgccggtaaagcggttgctctg
aaagaagcgatggagcctgagttcaaaacttaccagcagcaggtcgctaaaaacgctaaagcgatggtagaagtgttcctcgagcgcggctacaaagtggtttccggcggcactga
taaccacctgttcctggttgatctggttgataaaaacctgaccggtaaagaagcagacgccgctctgggccgtgctaacatcaccgtcaacaaaaacagcgtaccgaacgatccgaa
gagcccgtttgtgacctccggtattcgtgtaggtactccggcgattacccgtcgcggctttaaagaagccgaagcgaaagaactggctggctggatgtgtgacgtgctggacagcatc
aatgatgaagccgttatcgagcgcatcaaaggtaaagttctcgacatctgcgcacgttacccggtttacgcataa
Aminomethyltransferase, amino acid sequence (SEQ ID NO: 21): 
MAQQTPLYEQHTLCGARMVDFHGWMMPLHYGSQIDEHHAVRTDAGMFDVSHMTIVDLRGSRTREFLRYLLANDV
AKLTKSGKALYSGMLNASGGVIDDLIVYYFTEDFFRLVVNSATREKDLSWITQHAEPFGIEITVRDDLSMIAVQGPNA
QAKAATLFNDAQRQAVEGMKPFFGVQAGDLFIATTGYTGEAGYEIALPNEKAADFWRALVEAGVKPCGLGARDTL
RLEAGMNLYGQEMDETISPLAANMGWTIAWEPADRDFIGREALEVQREHGTEKLVGLVMTEKGVLRNELPVRFTD
AQGNQHEGIITSGTFSPTLGYSIALARVPEGIGETAIVQIRNREMPVKVTKPVFVRNGKAVA
Aminomethyltransferase, nucleotide sequence (SEQ ID NO: 22): 
atggcacaacagactcctttgtacgaacaacacacgctttgcggcgctcgcatggtggatttccacggctggatgatgccgttgcattacggttcgcaaatcgacgaacatcatgcggt
acgtaccgatgccggaatgtttgatgtgtcacatatgaccatcgtcgatctccgcggcagccgcacccgggagtttctgcgttatctgctggcgaacgatgtggcgaagctcaccaaa
agcggcaaagccctttactcggggatgttgaatgcctctggcggtgtgatagatgatctcatcgtctactactttactgaagatttcttccgcctcgttgttaactccgccacccgcgaaaa
agacctctcctggattacccaacacgctgaacctttcggcatcgaaattaccgttcgtgatgacctttccatgattgccgtgcaagggccgaatgcgcaggcaaaagctgccacactgtt
taatgacgcccagcgtcaggcggtggaagggatgaaaccgttctttggcgtgcaggcgggcgatctgtttattgccaccactggttataccggtgaagcgggctatgaaattgcgctg
cccaatgaaaaagcggccgatttctggcgtgcgctggtggaagcgggtgttaagccatgtggcttgggcgcgcgtgacacgctgcgtctggaagcgggcatgaatctttatggtcag
gagatggacgaaactatttctcctttagccgccaacatgggctggactatcgcctgggaaccggcagatcgtgactttatcggtcgtgaagccctggaagtgcagcgtgagcatggta
cagaaaaactggttggtctggtgatgaccgaaaaaggcgtgctgcgtaatgaactgccggtacgctttaccgatgcgcagggcaaccagcatgaaggcattatcaccagcggtacttt
ctccccgacgctgggttacagcattgcgctggcgcgcgtgccggaaggtattggcgaaacggcgattgtgcaaattcgcaaccgtgaaatgccggttaaagtgacaaaacctgttttt
gtgcgtaacggcaaagccgtcgcgtga
Glycine decarboxylase, amino acid sequence (SEQ ID NO: 23): 
MTQTLSQLENSGAFIERHIGPDAAQQQEMLNAVGAQSLNALTGQIVPKDIQLATPPQVGAPATEYAALAELKAIASR
NKRFTSYIGMGYTAVQLPPVILRNMLENPGWYTAYTPYQPEVSQGRLEALLNFQQVTLDLTGLDMASASLLDEATA
AAEAMAMAKRVSKLKNANRFFVASDVHPQTLDVVRTRAETFGFEVIVDDAQKVLDHQDVFGVLLQQVGTTGEIH
DYTALISELKSRKIVVSVAADIMALVLLTAPGKQGADIVFGSAQRFGVPMGYGGPHAAFFAAKDEYKRSMPGRIIGV
SKDAAGNTALRMAMQTREQHIRREKANSNICTSQVLLANIASLYAVYHGPVGLKRIANRIHRLTDILAAGLQQKGLK
LRHAHYFDTLCVEVADKAGVLTRAEAAEINLRSDILNAVGITLDETTTRENVMQLFSVLLGDNHGLDIDTLDKDVA
HDSRSIQPAMLRDDEILTHPVFNRYHSETEMMRYMHSLERKDLALNQAMIPLGSCTMKLNAAAEMIPITWPEFAEL
HPFCPPEQAEGYQQMIAQLADWLVKLTGYDAVCMQPNSGAQGEYAGLLAIRHYHESCNEGHRDICLIPASAHGTNP
ASAHMAGMQVVVVACDKNGNIDLTDLRAKAEQAGDNLSCIMVTYPSTHGVYEETIREVCEVVHQFGGQVYLDGA
NMNAQVGITSPGFIGADVSHLNLHKTFCIPHGGGGPGMGPIGVKAHLAPFVPGHSVVQIEGMLTRQGAVSAAPFGS
ASILPISWMYIRMMGAEGLKKASQVAILNANYIASRLQDAFPVLYTGRDGRVAHECILDIRPLKEETGISELDIAKRLI
DYGFHAPTMSFPVAGTLMVEPTESESKVELDRFIDAMLAIRAEIDQVKAGVWPLEDNPLVNAPHIQSELVAEWAHPY
SREVAVFPAGVADKYWPTVKRLDDVYGDRNLFCSCVPISEYQ
Glycine decarboxylase, nucleotide sequence (SEQ ID NO: 24): 
atgacacagacgttaagccagcttgaaaacagcggcgcttttattgaacgccatatcggaccggacgccgcgcaacagcaagaaatgctgaatgccgttggtgcacaatcgttaaac
gcgctgaccggccagattgtgccgaaagatattcagcttgcgacaccaccgcaggttggcgcaccggcgaccgaatacgccgcactggcagaactcaaggctattgccagtcgca
ataaacgcttcacgtcttacatcggcatgggttacaccgccgtgcagctaccgccggttatcctgcgtaacatgctggaaaatccgggctggtataccgcgtacactccgtatcaacct
gaagtctcccagggccgccttgaagcactgctcaacttccagcaggtaacgctggatttgactggactggatatggcctctgcttctcttctggacgaggccaccgctgccgccgaag
caatggcgatggcgaaacgcgtcagcaaactgaaaaatgccaaccgcttcttcgtggcttccgatgtgcatccgcaaacgctggatgtggtccgtactcgtgccgaaacctttggtttt
gaagtgattgtcgatgacgcgcaaaaagtgctcgaccatcaggacgtcttcggcgtgctgttacagcaggtaggcactaccggtgaaattcacgactacactgcgcttattagcgaact
gaaatcacgcaaaattgtggtcagcgttgccgccgatattatggcgctggtgctgttaactgcgccgggtaaacagggcgcggatattgtttttggttcggcgcaacgcttcggcgtgc
cgatgggctacggtggcccacacgcggcattctttgcggcgaaagatgaatacaaacgctcaatgccgggccgtattatcggtgtatcgaaagatgcagctggcaacaccgctctgc
gcatggcgatgcagactcgcgagcaacatattcgtcgtgagaaagcgaactccaacatttgtacttcccaggtactgctggcaaacatcgccagcctgtatgccgtttatcacggcccg
gttggcctgaaacgtatcgctaaccgcattcaccgtctgaccgatatcctggcggcaggcctgcaacaaaaaggtctgaagctgcgccatgcgcactatttcgacactctgtgtgtgga
agtggccgataaggcgggcgtactgacgcgtgccgaagcggctgaaatcaacctgcgtagcgatatcctgaacgcggttgggatcacccttgatgaaaccaccacgcgcgaaaac
gtgatgcagcttttcagcgtactgctgggggataaccacggcctggacatcgacacgctggacaaagacgtggctcacgacagccgctctattcaacctgcgatgctgcgcgacga
cgaaatcctcacccatccggtgtttaatcgctaccacagcgaaaccgaaatgatgcgctatatgcactcgctggagcgtaaagatctggcgctgaatcaggcgatgatcccgctgggt
tcctgcaccatgaaactgaacgccgccgccgagatgatcccaatcacctggccggaatttgccgaactgcacccgttctgcccgccggagcaggccgaaggttatcagcagatgatt
gcgcagctggctgactggctggtgaaactgaccggttacgacgccgtttgtatgcagccgaactctggcgcacagggcgaatacgcgggcctgctggcgattcgtcattatcatgaa
 
agctgcaacgaagggcatcgcgatatctgcctgatcccggcttctgcgcacggaactaaccccgcttctgcacatatggcaggaatgcaggtggtggttgtggcgtgtgataaaaac
ggcaacatcgatctgactgatctgcgcgcgaaagcggaacaggcgggcgataacctctcctgtatcatggtgacttatccttctacccacggcgtgtatgaagaaacgatccgtgaag
tgtgtgaagtcgtgcatcagttcggcggtcaggtttaccttgatggcgcgaacatgaacgcccaggttggcatcacctcgccgggctttattggtgcggacgtttcacaccttaacctac
ataaaactttctgcattccgcacggcggtggtggtccgggtatgggaccgatcggcgtgaaagcgcatttggcaccgtttgtaccgggtcatagcgtggtgcaaatcgaaggcatgtt
aacccgtcagggcgcggtttctgcggcaccgttcggtagcgcctctatcctgccaatcagctggatgtacatccgcatgatgggcgcagaagggctgaaaaaagcaagccaggtgg
caatcctcaacgccaactatattgccagccgcctgcaggatgccttcccggtgctgtataccggtcgcgacggtcgcgtggcgcacgaatgtattctcgatattcgcccgctgaaaga
agaaaccggcatcagcgagctggatattgccaagcgcctgatcgactacggtttccacgcgccgacgatgtcgttcccggtggcgggtacgctgatggttgaaccgactgaatctga
aagcaaagtggaactggatcgctttatcgacgcgatgctggctatccgcgcagaaattgaccaggtgaaagccggtgtctggccgctggaagataacccgctggtgaacgcgccgc
acattcagagcgaactggtcgccgagtgggcgcatccgtacagccgtgaagttgcggtattcccggcaggtgtggcagacaaatactggccgacagtgaaacgtctggatgatgttt
acggcgaccgtaacctgttctgctcctgcgtaccgattagcgaataccagtaa
Phosphoglycerate dehydrogenase, amino acid sequence (SEQ ID NO: 25): 
MSQNGRPVVLIADKLAQSTVDALGDAVEVRWVDGPNRPELLDAVKEADALLVRSATTVDAEVIAAAPNLKIVGRA
GVGLDNVDIPAATEAGVMVANAPTSNIHSACEHAISLLLSTARQIPAADATLREGEWKRSSFNGVEIFGKTVGIVGFG
HIGQLFAQRLAAFETTIVAYDPYANPARAAQLNVELVELDELMSRSDFVTIHLPKTKETAGMFDAQLLAKSKKGQIII
NAARGGLVDEQALADAIESGHIRGAGFDVYSTEPCTDSPLFKLPQVVVTPHLGASTEEAQDRAGTDVADSVLKALA
GEFVADAVNVSGGRVGEEVAVWMDLA
Phosphoglycerate dehydrogenase, nucleotide sequence (SEQ ID NO: 26): 
atgagccagaatggccgtccggtagtcctcatcgccgataagcttgcgcagtccactgttgacgcgcttggagatgcagtagaagtccgttgggttgacggacctaaccgcccagaa
ctgcttgatgcagttaaggaagcggacgcactgctcgtgcgttctgctaccactgtcgatgctgaagtcatcgccgctgcccctaacttgaagatcgtcggtcgtgccggcgtgggctt
ggacaacgttgacatccctgctgccactgaagctggcgtcatggttgctaacgcaccgacctctaatattcactccgcttgtgagcacgcaatttctttgctgctgtctactgctcgccag
atccctgctgctgatgcgacgctgcgtgagggcgagtggaagcggtcttctttcaacggtgtggaaattttcggaaaaactgtcggtatcgtcggttttggccacattggtcagttgtttg
ctcagcgtcttgctgcgtttgagaccaccattgttgcttacgatccttacgctaaccctgctcgtgcggctcagctgaacgttgagttggttgagttggatgagctgatgagccgttctgac
tttgtcaccattcaccttcctaagaccaaggaaactgctggcatgtttgatgcgcagctccttgctaagtccaagaagggccagatcatcatcaacgctgctcgtggtggccttgttgatg
agcaggctttggctgatgcgattgagtccggtcacattcgtggcgctggtttcgatgtgtactccaccgagccttgcactgattctcctttgttcaagttgcctcaggttgttgtgactcctca
cttgggtgcttctactgaagaggctcaggatcgtgcgggtactgacgttgctgattctgtgctcaaggcgctggctggcgagttcgtggcggatgctgtgaacgtttccggtggtcgcgt
gggcgaagaggttgctgtgtggatggatctggcttaa
Phosphoserine/phosphohydroxythreonine aminotransferase, amino acid sequence (SEQ ID NO: 27): 
MAQIFNFSSGPAMLPAEVLKQAQQELRDWNGLGTSVMEVSHRGKEFIQVAEEAEKDFRDLLNVPSNYKVLFCHGG
GRGQFAAVPLNILGDKTTADYVDAGYWAASAIKEAKKYCTPNVFDAKVTVDGLRAVKPMREWQLSDNAAYMHY
CPNETIDGIAIDETPDFGADVVVAADFSSTILSRPIDVSRYGVIYAGAQKNIGPAGLTIVIVREDLLGKANIACPSILDYS
ILNDNGSMFNTPPTFAWYLSGLVFKWLKANGGVAEMDKINQQKAELLYGVIDNSDFYRNDVAKANRSRMNVPFQL
ADSALDKLFLEESFAAGLHALKGHRVVGGMRASIYNAMPLEGVKALTDFMVEFERRHG
Phosphoserine/phosphohydroxythreonine aminotransferase, nucleotide sequence (SEQ ID NO: 28): 
atggctcaaatcttcaattttagttctggtccggcaatgctaccggcagaggtgcttaaacaggctcaacaggaactgcgcgactggaacggtcttggtacgtcggtgatggaagtgag
tcaccgtggcaaagagttcattcaggttgcagaggaagccgagaaggattttcgcgatcttcttaatgtcccctccaactacaaggtattattctgccatggcggtggtcgcggtcagttt
gctgcggtaccgctgaatattctcggtgataaaaccaccgcagattatgttgatgccggttactgggcggcaagtgccattaaagaagcgaaaaaatactgcacgcctaatgtctttgac
gccaaagtgactgttgatggtctgcgcgcggttaagccaatgcgtgaatggcaactctctgataatgctgcttatatgcattattgcccgaatgaaaccatcgatggtatcgccatcgacg
aaacgccagacttcggcgcagatgtggtggtcgccgctgacttctcttcaaccattctttcccgtccgattgacgtcagccgttatggtgtaatttacgctggcgcgcagaaaaatatcgg
cccggctggcctgacaatcgtcatcgttcgtgaagatttgctgggcaaagcgaatatcgcgtgtccgtcgattctggattattccatcctcaacgataacggctccatgtttaacacgccg
ccgacatttgcctggtatctatctggtctggtctttaaatggctgaaagcgaacggcggtgtagctgaaatggataaaatcaatcagcaaaaagcagaactgctatatggggtgattgat
aacagcgatttctaccgcaatgacgtggcgaaagctaaccgttcgcggatgaacgtgccgttccagttggcggacagtgcgcttgacaaattgttccttgaagagtcttttgctgctggc
cttcatgcactgaaaggtcaccgtgtggtcggcggaatgcgcgcttctatttataacgccatgccgctggaaggcgttaaagcgctgacagacttcatggttgagttcgaacgccgtca
cggttaa
Phosphoserine phosphatase, amino acid sequence (SEQ ID NO: 29):
Phosphoserine phosphatase, nucleotide sequence (SEQ ID NO: 30): 
atgcctaacattacctggtgcgacctgcctgaagatgtctctttatggccgggtctgcctctttcattaagtggtgatgaagtgatgccactggattaccacgcaggtcgtagcggctggc
tgctgtatggtcgtgggctggataaacaacgtctgacccaataccagagcaaactgggtgcggcgatggtgattgttgccgcctggtgcgtggaagattatcaggtgattcgtctggca
ggttcactcaccgcacgggctacacgcctggcccacgaagcgcagctggatgtcgccccgctggggaaaatcccgcacctgcgcacgccgggtttgctggtgatggatatggact
ccaccgccatccagattgaatgtattgatgaaattgccaaactggccggaacgggcgagatggtggcggaagtaaccgaacgggcgatgcgcggcgaactcgattttaccgccag
cctgcgcagccgtgtggcgacgctgaaaggcgctgacgccaatattctgcaacaggtgcgtgaaaatctgccgctgatgccaggcttaacgcaactggtgctcaagctggaaacgc
tgggctggaaagtggcgattgcctccggcggctttactttctttgctgaatacctgcgcgacaagctgcgcctgaccgccgtggtagccaatgaactggagatcatggacggtaaattt
accggcaatgtgatcggcgacatcgtagacgcgcagtacaaagcgaaaactctgactcgcctcgcgcaggagtatgaaatcccgctggcgcagaccgtggcgattggcgatggag
ccaatgacctgccgatgatcaaagcggcagggctggggattgcctaccatgccaagccaaaagtgaatgaaaaggcggaagtcaccatccgtcacgctgacctgatggggggtattc
tgcatcctctcaggcagcctgaatcagaagtaa
Branched-chain amino acid aminotransferase, amino acid sequence (SEQ ID NO: 31): 
VTTKKADYIWFNGEMVRWEDAKVHVMSHALHYGTSVFEGIRCYDSHKGPVVFRHREHMQRLHDSAKIYRFPVSQ
SIDELMEACRDVIRKNNLTSAYIRPLIFVGDVGMGVNPPAGYSTDVIIAAFPWGAYLGAEALEQGIDAMVSSWNRAA
PNTIPTAAKAGGNYLSSLLVGSEARRHGYQEGIALDVNGYISEGAGENLFEVKDGVLFTPPFTSSALPGITRDAIIKLA
KELGIEVREQVLSRESLYLADEVFMSGTAAEITPVRSVDGIQVGEGRCGPVTKRIQQAFFGLFTGETEDKWGWLDQ
VNQ
Branched-chain amino acid aminotransferase, nucleotide sequence (SEQ ID NO: 32): 
gtgaccacgaagaaagctgattacatttggttcaatggggagatggttcgctgggaagacgcgaaggtgcatgtgatgtcgcacgcgctgcactatggcacctcggtttttgaaggca
tccgttgctacgactcgcacaaaggaccggttgtattccgccatcgtgagcatatgcagcgtctgcatgactccgccaaaatctatcgctttccggtttcgcagagcattgatgagctgat
ggaagcttgtcgtgacgtgatccgcaaaaacaatctcaccagcgcctatatccgtccgctgatcttcgtcggtgatgttggcatgggcgttaacccgccagcgggatactcaaccgatg
tgattatcgccgctttcccgtggggagcgtatctgggcgcagaagcgctggagcaggggatcgatgcgatggtttcctcctggaaccgcgcagcaccaaacaccatcccaaccgcg
gcaaaagccggtggtaactacctctcttccctgctggtgggtagtgaagcacgccgccacggttatcaggaaggtatcgcgctggatgtgaatggttacatctctgaaggtgcaggcg
aaaacctgtttgaagtgaaagacggcgtgctgttcaccccaccgttcacctcctccgcgctgccgggtattacccgtgatgccatcatcaaactggcaaaagagctgggaattgaagtc
cgtgagcaggtgctgtcgcgcgaatccctgtacctggcggatgaagtgtttatgtccggtactgcggcagaaatcacgccagtgcgcagcgtagatggtattcaggttggtgaaggc
cgttgcggcccggttaccaaacgcatccagcaagccttcttcggcctcttcactggcgaaaccgaagataaatggggctggttagatcaagttaatcaataa
Acetohydroxy acid reductoisomerase from *Escherichia coli,* amino acid sequence (SEQ ID NO: 167): 
MANYFNTLNLRQQLAQLGKCRFMGRDEFADGASYLQGKKVVIVGCGAQGLNQGLNMRDSGLDISYALRKEAIAE
KRASWRKATENGFKVGTYEELIPQADLVINLTPDKQHSDVVRTVQPLMKDGAALGYSHGFNIVEVGEQIRKDITVV
MVAPKCPGTEVREEYKRGFGVPTLIAVHPENDPKGEGMAIAKAWAAATGGHRAGVLESSFVAEVKSDLMGEQTILC
GMLQAGSLLCFDKLVEEGTDPAYAEKLIQFGWETITEALKQGGITLMMDRLSNPAKLRAYALSEQLKEIMAPLFQKH
MDDIISGEFSSGMMADWANDDKKLLTWREETGKTAFETAPQYEGKIGEQEYFDKGVLMIAMVKAGVELAFETMV
DSGIIEESAYYESLHELPLIANTIARKRLYEMNVVISDTAEYGNYLFSYACVPLLKPFMAELQPGDLGKAIPEGAVDN
GQLRDVNEAIRSHAIEQVGKKLRGYMTDMKRIAVAG
Acetohydroxy acid reductoisomerase from *Escherichia coli,* nucleotide sequence (SEQ ID NO: 168): 
atggctaactacttcaatacactgaatctgcgccagcagctggcacagctgggcaaatgtcgctttatgggccgcgatgaattcgccgatggcgcgagctaccttcagggtaaaaag
tagtcatcgtcggctgtggcgcacagggtctgaaccagggcctgaacatgcgtgattctggtctcgatatctcctacgctctgcgtaaagaagcgattgccgagaagcgcgcgtcctg
gcgtaaagcgaccgaaaatggttttaaagtgggtacttacgaagaactgatcccacaggcggatctggtgattaacctgacgccggacaagcagcactctgatgtagtgcgcaccgt
acagccactgatgaaagacggcgcggcgctgggctactcgcacggtttcaacatcgtcgaagtgggcgagcagatccgtaaagatatcaccgtagtgatggttgcgccgaaatgcc
caggcaccgaagtgcgtgaagagtacaaacgtgggttcggcgtaccgacgctgattgccgttcacccggaaaacgatccgaaaggcgaaggcatggcgattgccaaagcctggg
 
cggctgcaaccggtggtcaccgtgcgggtgtgctggaatcgtccttcgttgcggaagtgaaatctgacctgatgggcgagcaaaccatcctgtgcggtatgttgcaggctggctctct
gctgtgcttcgacaagctggtggaagaaggtaccgatccagcatacgcagaaaaactgattcagttcggttgggaaaccatcaccgaagcactgaaacagggcggcatcaccctga
tgatggaccgtctctctaacccggcgaaactgcgtgcttatgcgctttctgaacagctgaaagagatcatggcacccctgttccagaaacatatggacgacatcatctccggcgaattct
cttccggtatgatggcggactgggccaacgatgataagaaactgctgacctggcgtgaagagaccggcaaaaccgcgtttgaaaccgcgccgcagtatgaaggcaaaatcggcga
gcaggagtacttcgataaaggcgtactgatgatcgcgatggtgaaagcgggcgttgaactggcgttcgaaaccatggtcgattccggcatcattgaagagtctgcatattatgaatcac
tgcacgagctgccgctgattgccaacaccatcgcccgtaagcgtctgtacgaaatgaacgtggttatctctgataccgctgagtacggtaactatctgttctcttacgcttgtgtgccgttg
ctgaaaccgtttatggcagagctgcaaccgggcgacctgggtaaagctattccggaaggcgcggtagataacgggcaactgcgtgatgtgaacgaagcgattcgcagccatgcgat
tgagcaggtaggtaagaaactgcgcggctatatgacagatatgaaacgtattgctgttgcgggttaa
M1-93 promoter (SEQ ID NO: 169):
   ttatctctggcggtgttgacaagagataacaacgttgatataattgagcccgtattgttagcatgtacgtttaaaccaggaaacagct
RBS5 artificial regulatory element (SEQ ID NO: 170):
   ttatctctggcggtgttgacaagagataacaacgttgatataattgagcccgtattgttagcatgtacgtttaaaccaggaggactacg
RBSL1 (SEQ ID NO: 171):
   ttatctctggcggtgttgacaagagataacaacgttgatataattgagcccgtattgttagcatgtacgtttaaaccaggaggtcagca
RBSL2 (SEQ ID NO: 172):
   ttatctctggcggtgttgacaagagataacaacgttgatataattgagcccgtattgttagcatgtacgtttaaaccaggagggttcga
M1-46 (SEQ ID NO: 173):
   ttatctctggcggtgttgacaagagataacaacgttgatataattgagcctctcgccccaccaattcggtttaaaccaggaaacagct
serCB gene cluster (SEQ ID NO: 174):

ttatctctggcggtgttgacaagagataacaacgttgatataattgagcccgtattgttagcatgtacgtttaaaccaggaaacagctatggctcaaatcttcaattttagttctggtccggca
atgctaccggcagaggtgcttaaacaggctcaacaggaactgcgcgactggaacggtcttggtacgtcggtgatggaagtgagtcaccgtggcaaagagttcattcaggttgcagag
gaagccgagaaggattttcgcgatcttcttaatgtcccctccaactacaaggtattattctgccatggcggtggtcgcggtcagtttgctgcggtaccgctgaatattctcggtgataaaac
caccgcagattatgttgatgccggttactgggcggcaagtgccattaaagaagcgaaaaaatactgcacgcctaatgtctttgacgccaaagtgactgttgatggtctgcgcgcggtta
agccaatgcgtgaatggcaactctctgataatgctgcttatatgcattattgcccgaatgaaaccatcgatggtatcgccatcgacgaaacgccagacttcggcgcagatgtggtggtc
gccgctgacttctcttcaaccattctttcccgtccgattgacgtcagccgttatggtgtaatttacgctggcgcgcagaaaaatatcggcccggctggcctgacaatcgtcatcgttcgtga
agatttgctgggcaaagcgaatatcgcgtgtccgtcgattctggattattccatcctcaacgataacggctccatgtttaacacgccgccgacatttgcctggtatctatctggtctggtcttt
aaatggctgaaagcgaacggcggtgtagctgaaatggataaaatcaatcagcaaaaagcagaactgctatatggggtgattgataacagcgatttctaccgcaatgacgtggcgaaa
gctaaccgttcgcggatgaacgtgccgttccagttggcggacagtgcgcttgacaaattgttccttgaagagtcttttgctgctggccttcatgcactgaaaggtcaccgtgtggtcggc
ggaatgcgcgcttctatttataacgccatgccgctggaaggcgttaaagcgctgacagacttcatggttgagttcgaacgccgtcacggttaagtcgacaaaaggagaacaaacatgc
ctaacattacctggtgcgacctgcctgaagatgtctctttatggccgggtctgcctctttcattaagtggtgatgaagtgatgccactggattaccacgcaggtcgtagcggctggctgct
gtatggtcgtgggctggataaacaacgtctgacccaataccagagcaaactgggtgcggcgatggtgattgttgccgcctggtgcgtggaagattatcaggtgattcgtctggcaggtt
cactcaccgcacgggctacacgcctggcccacgaagcgcagctggatgtcgccccgctggggaaaatcccgcacctgcgcacgccgggtttgctggtgatggatatggactccac
cgccatccagattgaatgtattgatgaaattgccaaactggccggaacgggcgagatggtggcggaagtaaccgaacgggcgatgcgcggcgaactcgattttaccgccagcctgc
gcagccgtgtggcgacgctgaaaggcgctgacgccaatattctgcaacaggtgcgtgaaaatctgccgctgatgccaggcttaacgcaactggtgctcaagctggaaacgctgggc
tggaaagtggcgattgcctccggcggctttactttctttgctgaatacctgcgcgacaagctgcgcctgaccgccgtggtagccaatgaactggagatcatggacggtaaatttaccgg
caatgtgatcggcgacatcgtagacgcgcagtacaaagcgaaaactctgactcgcctcgcgcaggagtatgaaatcccgctggcgcagaccgtggcgattggcgatggagccaat
gacctgccgatgatcaaagcggcagggctggggattgcctaccatgccaagccaaaagtgaatgaaaaggcggaagtcaccatccgtcacgctgacctgatgggggtattctgcat
cctctcaggcagcctgaatcagaagtaa

## Claims

1. A genetically engineered pantoic acid-producing strain, having or having an enhanced NADH-dependent acetohydroxy acid reductoisomerase, wherein preferably, the NADH-dependent acetohydroxy acid reductoisomerase is derived from *Thermacetogenium phaeum,* and more preferably comprises an amino acid sequence shown in SEQ ID NO: 9 or an amino acid sequence having at least 75%, 80%, 85%, 90%, 91%, 92%, 93%, 94%, 95%, 96%, 97%, 98%, 99% or higher identity thereto and having NADH-dependent acetohydroxy acid reductoisomerase activity.

2. The genetically engineered pantoic acid-producing strain according to claim 1,
further having or having enhanced activities of: an acetolactate synthase, a dihydroxy acid dehydratase, a 3-methyl-2-oxobutanoate hydroxymethyltransferase, a 2-dehydropantoate-2-reductase, a serine hydroxymethyltransferase, a glycine cleavage enzyme system (e.g., an aminomethyltransferase and/or a glycine decarboxylase), a phosphoglycerate dehydrogenase, a phosphoserine/phosphohydroxythreonine aminotransferase, and a phosphoserine phosphatase, and
optionally, having reduced or inactivated activities of: an L-serine deaminase I, a propionate kinase, a formate acetyltransferase, an alcohol dehydrogenase, a pyruvate formate lyase, a fumarate reductase, a lactate dehydrogenase, a methylglyoxal synthase, an acetate kinase, a ribokinase, a valine-pyruvate transaminase, a phosphotransacetylase, and/or a branched-chain amino acid aminotransferase; preferably wherein the branched-chain amino acid aminotransferase is attenuated,
preferably, the 3-methyl-2-oxobutanoate hydroxymethyltransferase comprises a 3-methyl-2-oxobutanoate hydroxymethyltransferase derived from *Corynebacterium glutamicum* and/or *Escherichia coli,*
preferably, the phosphoglycerate dehydrogenase is derived from *Corynebacterium glutamicum,* and/or the dihydroxy acid dehydratase, the 2-dehydropantoate-2-reductase, the glycine cleavage enzyme system (e.g., the aminomethyltransferase and/or the glycine decarboxylase), the phosphoserine/phosphohydroxythreonine aminotransferase, and the phosphoserine phosphatase are derived from *Escherichia coli,* and
preferably, the acetolactate synthase comprises an acetolactate synthase derived from *Bacillus subtilis,* and/or an acetolactate synthase I, an acetolactate synthase II, and/or an L-valine feedback-resistant acetolactate synthase III derived from *Escherichia coli.*

3. The genetically engineered pantoic acid-producing strain according to claim 2, wherein:
the acetolactate synthase derived from *Bacillus subtilis* comprises an amino acid sequence shown in SEQ ID NO: 1 or an amino acid sequence having at least 75%, 80%, 85%, 90%, 91%, 92%, 93%, 94%, 95%, 96%, 97%, 98%, 99% or higher identity thereto and having acetolactate synthase activity; and/or
the acetolactate synthase I comprises an amino acid sequence shown in SEQ ID NO: 3 or an amino acid sequence having at least 75%, 80%, 85%, 90%, 91%, 92%, 93%, 94%, 95%, 96%, 97%, 98%, 99% or higher identity thereto and having acetolactate synthase I activity; and/or
the acetolactate synthase II comprises an amino acid sequence shown in SEQ ID NO: 5 or an amino acid sequence having at least 75%, 80%, 85%, 90%, 91%, 92%, 93%, 94%, 95%, 96%, 97%, 98%, 99% or higher identity thereto and having acetolactate synthase II activity; and/or
the L-valine feedback-resistant acetolactate synthase III comprises an amino acid sequence shown in SEQ ID NO: 7 or an amino acid sequence having at least 75%, 80%, 85%, 90%, 91%, 92%, 93%, 94%, 95%, 96%, 97%, 98%, 99% or higher identity thereto and having L-valine feedback-resistant acetolactate synthase III activity; and/or
the dihydroxy acid dehydratase comprises an amino acid sequence shown in SEQ ID NO: 11 or an amino acid sequence having at least 75%, 80%, 85%, 90%, 91%, 92%, 93%, 94%, 95%, 96%, 97%, 98%, 99% or higher identity thereto and having dihydroxy acid dehydratase activity; and/or
the 3-methyl-2-oxobutanoate hydroxymethyltransferase derived from *Corynebacterium glutamicum* comprises an amino acid sequence shown in SEQ ID NO: 13 or an amino acid sequence having at least 75%, 80%, 85%, 90%, 91%, 92%, 93%, 94%, 95%, 96%, 97%, 98%, 99% or higher identity thereto and having 3-methyl-2-oxobutanoate hydroxymethyltransferase activity; and/or
the 3-methyl-2-oxobutanoate hydroxymethyltransferase derived from *Escherichia coli* comprises an amino acid sequence shown in SEQ ID NO: 15 or an amino acid sequence having at least 75%, 80%, 85%, 90%, 91%, 92%, 93%, 94%, 95%, 96%, 97%, 98%, 99% or higher identity thereto and having 3-methyl-2-oxobutanoate hydroxymethyltransferase activity; and/or
the 2-dehydropantoate-2-reductase comprises an amino acid sequence shown in SEQ ID NO: 17 or an amino acid sequence having at least 75%, 80%, 85%, 90%, 91%, 92%, 93%, 94%, 95%, 96%, 97%, 98%, 99% or higher identity thereto and having 2-dehydropantoate-2-reductase activity; and/or
the serine hydroxymethyltransferase comprises an amino acid sequence shown in SEQ ID NO: 19 or an amino acid sequence having at least 75%, 80%, 85%, 90%, 91%, 92%, 93%, 94%, 95%, 96%, 97%, 98%, 99% or higher identity thereto and having serine hydroxymethyltransferase activity; and/or
the aminomethyltransferase comprises an amino acid sequence shown in SEQ ID NO: 21 or an amino acid sequence having at least 75%, 80%, 85%, 90%, 91%, 92%, 93%, 94%, 95%, 96%, 97%, 98%, 99% or higher identity thereto and having aminomethyltransferase activity; and/or
the glycine decarboxylase comprises an amino acid sequence shown in SEQ ID NO: 23 or an amino acid sequence having at least 75%, 80%, 85%, 90%, 91%, 92%, 93%, 94%, 95%, 96%, 97%, 98%, 99% or higher identity thereto and having glycine decarboxylase activity; and/or
the phosphoglycerate dehydrogenase comprises an amino acid sequence shown in SEQ ID NO: 25 or an amino acid sequence having at least 75%, 80%, 85%, 90%, 91%, 92%, 93%, 94%, 95%, 96%, 97%, 98%, 99% or higher identity thereto and having phosphoglycerate dehydrogenase activity; and/or
the phosphoserine/phosphohydroxythreonine aminotransferase comprises an amino acid sequence shown in SEQ ID NO: 27 or an amino acid sequence having at least 75%, 80%, 85%, 90%, 91%, 92%, 93%, 94%, 95%, 96%, 97%, 98%, 99% or higher identity thereto and having phosphoserine/phosphohydroxythreonine aminotransferase activity; and/or
the phosphoserine phosphatase comprises an amino acid sequence shown in SEQ ID NO: 29 or an amino acid sequence having at least 75%, 80%, 85%, 90%, 91%, 92%, 93%, 94%, 95%, 96%, 97%, 98%, 99% or higher identity thereto and having phosphoserine phosphatase activity; and/or
the attenuated branched-chain amino acid aminotransferase comprises an amino acid sequence shown in SEQ ID NO: 31 or an amino acid sequence having at least 75%, 80%, 85%, 90%, 91%, 92%, 93%, 94%, 95%, 96%, 97%, 98%, 99% or higher identity thereto and having attenuated branched-chain amino acid aminotransferase activity.

4. The genetically engineered pantoic acid-producing strain according to any one of claims 1-3,
expressing a gene encoding the attenuated branched-chain amino acid aminotransferase; and/or
having overexpressed genes encoding: the acetolactate synthase, the NADH-dependent acetohydroxy acid reductoisomerase derived from a *Thermacetogenium phaeum* strain, the dihydroxy acid dehydratase, the 3-methyl-2-oxobutanoate hydroxymethyltransferase derived from *Corynebacterium glutamicum,* the 2-dehydropantoate-2-reductase, the serine hydroxymethyltransferase, the glycine cleavage enzyme system (e.g., the aminomethyltransferase and/or the glycine decarboxylase), the 3-methyl-2-oxobutanoate hydroxymethyltransferase derived from *Escherichia coli,* the phosphoglycerate dehydrogenase, the phosphoserine/phosphohydroxythreonine aminotransferase, and the phosphoserine phosphatase, and/or
optionally, not having a gene encoding one or more, and preferably all of the following enzymes, or having an endogenous gene encoding one or more, and preferably all of the following enzymes knocked out: the L-serine deaminase I, the propionate kinase, the formate acetyltransferase, the alcohol dehydrogenase, the pyruvate formate lyase, the fumarate reductase, the lactate dehydrogenase, the methylglyoxal synthase, the a cetate kinase, the ribokinase, the valine-pyruvate transaminase, and the phosphotransacetylase,
wherein preferably, the gene encoding the acetolactate synthase comprises a gene encoding the acetolactate synthase derived from *Bacillus subtilis,* and/or genes encoding the acetolactate synthase I, the acetolactate synthase II, and/or the L-valine feedback-resistant acetolactate synthase III derived from *Escherichia coli.*

5. The genetically engineered pantoic acid-producing strain according to claim 4, wherein:
the gene encoding the acetolactate synthase derived from *Bacillus subtilis* comprises a nucleotide sequence shown in SEQ ID NO: 2 or a degenerate sequence thereof, or a nucleotide sequence having at least 75%, 80%, 85%, 90%, 91%, 92%, 93%, 94%, 95%, 96%, 97%, 98%, 99% or higher identity thereto; and/or
the gene encoding the acetolactate synthase I comprises a nucleotide sequence shown in SEQ ID NO: 4 or a degenerate sequence thereof, or a nucleotide sequence having at least 75%, 80%, 85%, 90%, 91%, 92%, 93%, 94%, 95%, 96%, 97%, 98%, 99% or higher identity thereto; and/or
the gene encoding the acetolactate synthase II comprises a nucleotide sequence shown in SEQ ID NO: 6 or a degenerate sequence thereof, or a nucleotide sequence having at least 75%, 80%, 85%, 90%, 91%, 92%, 93%, 94%, 95%, 96%, 97%, 98%, 99% or higher identity thereto; and/or
the gene encoding the L-valine feedback-resistant acetolactate synthase III comprises a nucleotide sequence shown in SEQ ID NO: 8 or a degenerate sequence thereof, or a nucleotide sequence having at least 75%, 80%, 85%, 90%, 91%, 92%, 93%, 94%, 95%, 96%, 97%, 98%, 99% or higher identity thereto; and/or
the gene encoding the NADH-dependent acetohydroxy acid reductoisomerase comprises a nucleotide sequence shown in SEQ ID NO: 10 or a degenerate sequence thereof, or a nucleotide sequence having at least 75%, 80%, 85%, 90%, 91%, 92%, 93%, 94%, 95%, 96%, 97%, 98%, 99% or higher identity thereto; and/or
the gene encoding the dihydroxy acid dehydratase comprises a nucleotide sequence shown in SEQ ID NO: 12 or a degenerate sequence thereof, or a nucleotide sequence having at least 75%, 80%, 85%, 90%, 91%, 92%, 93%, 94%, 95%, 96%, 97%, 98%, 99% or higher identity thereto; and/or
the gene encoding the 3-methyl-2-oxobutanoate hydroxymethyltransferase derived from *Corynebacterium glutamicum* comprises a nucleotide sequence shown in SEQ ID NO: 14 or a degenerate sequence thereof, or a nucleotide sequence having at least 75%, 80%, 85%, 90%, 91%, 92%, 93%, 94%, 95%, 96%, 97%, 98%, 99% or higher identity thereto; and/or
the gene encoding the 3-methyl-2-oxobutanoate hydroxymethyltransferase derived from *Escherichia coli* comprises a nucleotide sequence shown in SEQ ID NO: 16 or a degenerate sequence thereof, or a nucleotide sequence having at least 75%, 80%, 85%, 90%, 91%, 92%, 93%, 94%, 95%, 96%, 97%, 98%, 99% or higher identity thereto; and/or
the gene encoding the 2-dehydropantoate-2-reductase comprises a nucleotide sequence shown in SEQ ID NO: 18 or a degenerate sequence thereof, or a nucleotide sequence having at least 75%, 80%, 85%, 90%, 91%, 92%, 93%, 94%, 95%, 96%, 97%, 98%, 99% or higher identity thereto; and/or
the gene encoding the serine hydroxymethyltransferase comprises a nucleotide sequence shown in SEQ ID NO: 20 or a degenerate sequence thereof, or a nucleotide sequence having at least 75%, 80%, 85%, 90%, 91%, 92%, 93%, 94%, 95%, 96%, 97%, 98%, 99% or higher identity thereto; and/or
the gene encoding the aminomethyltransferase comprises a nucleotide sequence shown in SEQ ID NO: 22 or a degenerate sequence thereof, or a nucleotide sequence having at least 75%, 80%, 85%, 90%, 91%, 92%, 93%, 94%, 95%, 96%, 97%, 98%, 99% or higher identity thereto; and/or
the gene encoding the glycine decarboxylase comprises a nucleotide sequence shown in SEQ ID NO: 24 or a degenerate sequence thereof, or a nucleotide sequence having at least 75%, 80%, 85%, 90%, 91%, 92%, 93%, 94%, 95%, 96%, 97%, 98%, 99% or higher identity thereto; and/or
the gene encoding the phosphoglycerate dehydrogenase comprises a nucleotide sequence shown in SEQ ID NO: 26 or a degenerate sequence thereof, or a nucleotide sequence having at least 75%, 80%, 85%, 90%, 91%, 92%, 93%, 94%, 95%, 96%, 97%, 98%, 99% or higher identity thereto; and/or
the gene encoding the phosphoserine/phosphohydroxythreonine aminotransferase comprises a nucleotide sequence shown in SEQ ID NO: 28 or a degenerate sequence thereof, or a nucleotide sequence having at least 75%, 80%, 85%, 90%, 91%, 92%, 93%, 94%, 95%, 96%, 97%, 98%, 99% or higher identity thereto; and/or
the gene encoding the phosphoserine phosphatase comprises a nucleotide sequence shown in SEQ ID NO: 30 or a degenerate sequence thereof, or a nucleotide sequence having at least 75%, 80%, 85%, 90%, 91%, 92%, 93%, 94%, 95%, 96%, 97%, 98%, 99% or higher identity thereto; and/or
the gene encoding the attenuated branched-chain amino acid aminotransferase comprises a nucleotide sequence shown in SEQ ID NO: 32 or a degenerate sequence thereof, or a nucleotide sequence having at least 75%, 80%, 85%, 90%, 91%, 92%, 93%, 94%, 95%, 96%, 97%, 98%, 99% or higher identity thereto.

6. The genetically engineered pantoic acid-producing strain according to any one of claims 1-5, wherein the genetically engineered pantoic acid-producing strain belongs to *Escherichia, Enterobacter, Corynebacterium glutamicum, Bacillus subtilis,* or yeast, preferably *Escherichia coli,* and more preferably *Escherichia coli* deposited in the China General Microbiological Culture Collection Center (CGMCC) in Beijing, China, with a deposit number CGMCC No. 26276.

7. A method for producing the genetically engineered pantoic acid-producing strain according to any one of claims 1-6, comprising conferring or enhancing activity of an NADH-dependent acetohydroxy acid reductoisomerase in a pantoic acid-producing strain, wherein preferably, the NADH-dependent acetohydroxy acid reductoisomerase is derived from *Thermacetogenium phaeum,* and more preferably comprises an amino acid sequence shown in SEQ ID NO: 9 or an amino acid sequence having at least 75%, 80%, 85%, 90%, 91%, 92%, 93%, 94%, 95%, 96%, 97%, 98%, 99% or higher identity thereto and having NADH-dependent acetohydroxy acid reductoisomerase activity.

8. The method according to claim 7, further comprising, in the strain:
conferring or enhancing activities of: an acetolactate synthase, a dihydroxy acid dehydratase, a 3-methyl-2-oxobutanoate hydroxymethyltransferase, a 2-dehydropantoate-2-reductase, a serine hydroxymethyltransferase, a glycine cleavage enzyme system (e.g., an aminomethyltransferase and/or a glycine decarboxylase), a phosphoglycerate dehydrogenase, a phosphoserine/phosphohydroxythreonine aminotransferase, and a phosphoserine phosphatase, and
optionally, attenuating or inactivating, if present, activities of: an L-serine deaminase I, a propionate kinase, a formate acetyltransferase, an alcohol dehydrogenase, a pyruvate formate lyase, a fumarate reductase, a lactate dehydrogenase, a methylglyoxal synthase, an acetate kinase, a ribokinase, a valine-pyruvate transaminase, a phosphotransacetylase, and/or a branched-chain amino acid aminotransferase; and preferably attenuating activity of a branched-chain amino acid aminotransferase,
wherein preferably, the 3-methyl-2-oxobutanoate hydroxymethyltransferase comprises a 3-methyl-2-oxobutanoate hydroxymethyltransferase derived from *Corynebacterium glutamicum* and/or *Escherichia coli,*
preferably, the phosphoglycerate dehydrogenase is derived from *Corynebacterium glutamicum,* and/or the dihydroxy acid dehydratase, the 2-dehydropantoate-2-reductase, the glycine cleavage enzyme system (e.g., the aminomethyltransferase and/or the glycine decarboxylase), the phosphoserine/phosphohydroxythreonine aminotransferase, and the phosphoserine phosphatase are derived from *Escherichia coli,* and
preferably, the acetolactate synthase comprises an acetolactate synthase derived from *Bacillus subtilis,* and/or an acetolactate synthase I, an acetolactate synthase II, and/or an L-valine feedback-resistant acetolactate synthase III derived from *Escherichia coli.*

9. The method according to claim 8, wherein:
the acetolactate synthase derived from *Bacillus subtilis* comprises an amino acid sequence shown in SEQ ID NO: 1 or an amino acid sequence having at least 75%, 80%, 85%, 90%, 91%, 92%, 93%, 94%, 95%, 96%, 97%, 98%, 99% or higher identity thereto and having acetolactate synthase activity; and/or
the acetolactate synthase I comprises an amino acid sequence shown in SEQ ID NO: 3 or an amino acid sequence having at least 75%, 80%, 85%, 90%, 91%, 92%, 93%, 94%, 95%, 96%, 97%, 98%, 99% or higher identity thereto and having acetolactate synthase I activity; and/or
the acetolactate synthase II comprises an amino acid sequence shown in SEQ ID NO: 5 or an amino acid sequence having at least 75%, 80%, 85%, 90%, 91%, 92%, 93%, 94%, 95%, 96%, 97%, 98%, 99% or higher identity thereto and having acetolactate synthase II activity; and/or
the L-valine feedback-resistant acetolactate synthase III comprises an amino acid sequence shown in SEQ ID NO: 7 or an amino acid sequence having at least 75%, 80%, 85%, 90%, 91%, 92%, 93%, 94%, 95%, 96%, 97%, 98%, 99% or higher identity thereto and having L-valine feedback-resistant acetolactate synthase III activity; and/or
the dihydroxy acid dehydratase comprises an amino acid sequence shown in SEQ ID NO: 11 or an amino acid sequence having at least 75%, 80%, 85%, 90%, 91%, 92%, 93%, 94%, 95%, 96%, 97%, 98%, 99% or higher identity thereto and having dihydroxy acid dehydratase activity; and/or
the 3-methyl-2-oxobutanoate hydroxymethyltransferase derived from *Corynebacterium glutamicum* comprises an amino acid sequence shown in SEQ ID NO: 13 or an amino acid sequence having at least 75%, 80%, 85%, 90%, 91%, 92%, 93%, 94%, 95%, 96%, 97%, 98%, 99% or higher identity thereto and having 3-methyl-2-oxobutanoate hydroxymethyltransferase activity; and/or
the 3-methyl-2-oxobutanoate hydroxymethyltransferase derived from *Escherichia coli* comprises an amino acid sequence shown in SEQ ID NO: 15 or an amino acid sequence having at least 75%, 80%, 85%, 90%, 91%, 92%, 93%, 94%, 95%, 96%, 97%, 98%, 99% or higher identity thereto and having 3-methyl-2-oxobutanoate hydroxymethyltransferase activity; and/or
the 2-dehydropantoate-2-reductase comprises an amino acid sequence shown in SEQ ID NO: 17 or an amino acid sequence having at least 75%, 80%, 85%, 90%, 91%, 92%, 93%, 94%, 95%, 96%, 97%, 98%, 99% or higher identity thereto and having 2-dehydropantoate-2-reductase activity; and/or
the serine hydroxymethyltransferase comprises an amino acid sequence shown in SEQ ID NO: 19 or an amino acid sequence having at least 75%, 80%, 85%, 90%, 91%, 92%, 93%, 94%, 95%, 96%, 97%, 98%, 99% or higher identity thereto and having serine hydroxymethyltransferase activity; and/or
the aminomethyltransferase comprises an amino acid sequence shown in SEQ ID NO: 21 or an amino acid sequence having at least 75%, 80%, 85%, 90%, 91%, 92%, 93%, 94%, 95%, 96%, 97%, 98%, 99% or higher identity thereto and having aminomethyltransferase activity; and/or
the glycine decarboxylase comprises an amino acid sequence shown in SEQ ID NO: 23 or an amino acid sequence having at least 75%, 80%, 85%, 90%, 91%, 92%, 93%, 94%, 95%, 96%, 97%, 98%, 99% or higher identity thereto and having glycine decarboxylase activity; and/or
the phosphoglycerate dehydrogenase comprises an amino acid sequence shown in SEQ ID NO: 25 or an amino acid sequence having at least 75%, 80%, 85%, 90%, 91%, 92%, 93%, 94%, 95%, 96%, 97%, 98%, 99% or higher identity thereto and having phosphoglycerate dehydrogenase activity; and/or
the phosphoserine/phosphohydroxythreonine aminotransferase comprises an amino acid sequence shown in SEQ ID NO: 27 or an amino acid sequence having at least 75%, 80%, 85%, 90%, 91%, 92%, 93%, 94%, 95%, 96%, 97%, 98%, 99% or higher identity thereto and having phosphoserine/phosphohydroxythreonine aminotransferase activity; and/or
the phosphoserine phosphatase comprises an amino acid sequence shown in SEQ ID NO: 29 or an amino acid sequence having at least 75%, 80%, 85%, 90%, 91%, 92%, 93%, 94%, 95%, 96%, 97%, 98%, 99% or higher identity thereto and having phosphoserine phosphatase activity; and/or
the attenuated branched-chain amino acid aminotransferase comprises an amino acid sequence shown in SEQ ID NO: 31 or an amino acid sequence having at least 75%, 80%, 85%, 90%, 91%, 92%, 93%, 94%, 95%, 96%, 97%, 98%, 99% or higher identity thereto and having attenuated branched-chain amino acid aminotransferase activity.

10. The method according to any one of claims 7-9, comprising, in the strain:
expressing a gene encoding the attenuated branched-chain amino acid aminotransferase; and/or
overexpressing: a gene encoding the acetolactate synthase derived from *Bacillus subtilis,* a gene encoding the acetolactate synthase I, a gene encoding the acetolactate synthase II, a gene encoding the L-valine feedback-resistant acetolactate synthase III, a gene encoding the NADH-dependent acetohydroxy acid reductoisomerase derived from a *Thermacetogenium phaeum* strain, a gene encoding the dihydroxy acid dehydratase, a gene encoding the 3-methyl-2-oxobutanoate hydroxymethyltransferase derived from *Corynebacterium glutamicum,* a gene encoding the 2-dehydropantoate-2-reductase, a gene encoding the serine hydroxymethyltransferase, a gene encoding the glycine cleavage enzyme system (e.g., a gene encoding the aminomethyltransferase and/or a gene encoding the glycine decarboxylase), a gene encoding the 3-methyl-2-oxobutanoate hydroxymethyltransferase derived from *Escherichia coli,* a gene encoding the phosphoglycerate dehydrogenase, a gene encoding the phosphoserine/phosphohydroxythreonine aminotransferase, and a gene encoding the phosphoserine phosphatase; and/or
optionally, knocking out endogenous genes, if present, encoding one or more, and preferably all of the following enzymes: the L-serine deaminase I, the propionate kinase, the formate acetyltransferase, the alcohol dehydrogenase, the pyruvate formate lyase, the fumarate reductase, the lactate dehydrogenase, the methylglyoxal synthase, the acetate kinase, the ribokinase, the valine-pyruvate transaminase, and/or the phosphotransacetylase.

11. The method according to claim 10, wherein:
the gene encoding the acetolactate synthase derived from *Bacillus subtilis* comprises a nucleotide sequence shown in SEQ ID NO: 2 or a degenerate sequence thereof, or a nucleotide sequence having at least 75%, 80%, 85%, 90%, 91%, 92%, 93%, 94%, 95%, 96%, 97%, 98%, 99% or higher identity thereto; and/or
the gene encoding the acetolactate synthase I comprises a nucleotide sequence shown in SEQ ID NO: 4 or a degenerate sequence thereof, or a nucleotide sequence having at least 75%, 80%, 85%, 90%, 91%, 92%, 93%, 94%, 95%, 96%, 97%, 98%, 99% or higher identity thereto; and/or
the gene encoding the acetolactate synthase II comprises a nucleotide sequence shown in SEQ ID NO: 6 or a degenerate sequence thereof, or a nucleotide sequence having at least 75%, 80%, 85%, 90%, 91%, 92%, 93%, 94%, 95%, 96%, 97%, 98%, 99% or higher identity thereto; and/or
the gene encoding the L-valine feedback-resistant acetolactate synthase III comprises a nucleotide sequence shown in SEQ ID NO: 8 or a degenerate sequence thereof, or a nucleotide sequence having at least 75%, 80%, 85%, 90%, 91%, 92%, 93%, 94%, 95%, 96%, 97%, 98%, 99% or higher identity thereto; and/or
the gene encoding the NADH-dependent acetohydroxy acid reductoisomerase comprises a nucleotide sequence shown in SEQ ID NO: 10 or a degenerate sequence thereof, or a nucleotide sequence having at least 75%, 80%, 85%, 90%, 91%, 92%, 93%, 94%, 95%, 96%, 97%, 98%, 99% or higher identity thereto; and/or
the gene encoding the dihydroxy acid dehydratase comprises a nucleotide sequence shown in SEQ ID NO: 12 or a degenerate sequence thereof, or a nucleotide sequence having at least 75%, 80%, 85%, 90%, 91%, 92%, 93%, 94%, 95%, 96%, 97%, 98%, 99% or higher identity thereto; and/or
the gene encoding the 3-methyl-2-oxobutanoate hydroxymethyltransferase derived from *Corynebacterium glutamicum* comprises a nucleotide sequence shown in SEQ ID NO: 14 or a degenerate sequence thereof, or a nucleotide sequence having at least 75%, 80%, 85%, 90%, 91%, 92%, 93%, 94%, 95%, 96%, 97%, 98%, 99% or higher identity thereto; and/or
the gene encoding the 3-methyl-2-oxobutanoate hydroxymethyltransferase derived from *Escherichia coli* comprises a nucleotide sequence shown in SEQ ID NO: 16 or a degenerate sequence thereof, or a nucleotide sequence having at least 75%, 80%, 85%, 90%, 91%, 92%, 93%, 94%, 95%, 96%, 97%, 98%, 99% or higher identity thereto; and/or
the gene encoding the 2-dehydropantoate-2-reductase comprises a nucleotide sequence shown in SEQ ID NO: 18 or a degenerate sequence thereof, or a nucleotide sequence having at least 75%, 80%, 85%, 90%, 91%, 92%, 93%, 94%, 95%, 96%, 97%, 98%, 99% or higher identity thereto; and/or
the gene encoding the serine hydroxymethyltransferase comprises a nucleotide sequence shown in SEQ ID NO: 20 or a degenerate sequence thereof, or a nucleotide sequence having at least 75%, 80%, 85%, 90%, 91%, 92%, 93%, 94%, 95%, 96%, 97%, 98%, 99% or higher identity thereto; and/or
the gene encoding the aminomethyltransferase comprises a nucleotide sequence shown in SEQ ID NO: 22 or a degenerate sequence thereof, or a nucleotide sequence having at least 75%, 80%, 85%, 90%, 91%, 92%, 93%, 94%, 95%, 96%, 97%, 98%, 99% or higher identity thereto; and/or
the gene encoding the glycine decarboxylase comprises a nucleotide sequence shown in SEQ ID NO: 24 or a degenerate sequence thereof, or a nucleotide sequence having at least 75%, 80%, 85%, 90%, 91%, 92%, 93%, 94%, 95%, 96%, 97%, 98%, 99% or higher identity thereto; and/or
the gene encoding the phosphoglycerate dehydrogenase comprises a nucleotide sequence shown in SEQ ID NO: 26 or a degenerate sequence thereof, or a nucleotide sequence having at least 75%, 80%, 85%, 90%, 91%, 92%, 93%, 94%, 95%, 96%, 97%, 98%, 99% or higher identity thereto; and/or
the gene encoding the phosphoserine/phosphohydroxythreonine aminotransferase comprises a nucleotide sequence shown in SEQ ID NO: 28 or a degenerate sequence thereof, or a nucleotide sequence having at least 75%, 80%, 85%, 90%, 91%, 92%, 93%, 94%, 95%, 96%, 97%, 98%, 99% or higher identity thereto; and/or
the gene encoding the phosphoserine phosphatase comprises a nucleotide sequence shown in SEQ ID NO: 30 or a degenerate sequence thereof, or a nucleotide sequence having at least 75%, 80%, 85%, 90%, 91%, 92%, 93%, 94%, 95%, 96%, 97%, 98%, 99% or higher identity thereto; and/or
the gene encoding the attenuated branched-chain amino acid aminotransferase comprises a nucleotide sequence shown in SEQ ID NO: 32 or a degenerate sequence thereof, or a nucleotide sequence having at least 75%, 80%, 85%, 90%, 91%, 92%, 93%, 94%, 95%, 96%, 97%, 98%, 99% or higher identity thereto.

12. The method according to any one of claims 7-11, wherein the strain is selected from *Escherichia, Enterobacter, Corynebacterium glutamicum, Bacillus subtilis,* and yeast, and preferably *Escherichia coli.*

13. A method for producing a genetically engineered pantoic acid-producing strain, comprising: in *Escherichia coli* deposited in the China General Microbiological Culture Collection Center (CGMCC) in Beijing, China, with a deposit number CGMCC No. 21699, replacing an NADPH-dependent acetohydroxy acid reductoisomerase-encoding gene with a gene encoding an NADH-dependent acetohydroxy acid reductoisomerase,
wherein preferably, the NADH-dependent acetohydroxy acid reductoisomerase is derived from a *Thermacetogenium phaeum* strain, more preferably, the NADH-dependent acetohydroxy acid reductoisomerase comprises an amino acid sequence shown in SEQ ID NO: 9 or an amino acid sequence having at least 75%, 80%, 85%, 90%, 91%, 92%, 93%, 94%, 95%, 96%, 97%, 98%, 99% or higher identity thereto and having NADH-dependent acetohydroxy acid reductoisomerase activity, and more preferably, the gene encoding the NADH-dependent acetohydroxy acid reductoisomerase comprises a nucleotide sequence shown in SEQ ID NO: 10 or a degenerate sequence thereof, or a nucleotide sequence having at least 75%, 80%, 85%, 90%, 91%, 92%, 93%, 94%, 95%, 96%, 97%, 98%, 99% or higher identity thereto.

14. The method according to any one of claims 7-13, wherein the genetically engineered pantoic acid-producing strain is *Escherichia coli* deposited in the China General Microbiological Culture Collection Center (CGMCC) in Beijing, China, with a deposit number CGMCC No. 26276.

15. A method for improving D-pantoic acid production of a pantoic acid-producing strain, comprising: conferring or enhancing activity of an NADH-dependent acetohydroxy acid reductoisomerase in the pantoic acid-producing strain, wherein preferably, the NADH-dependent acetohydroxy acid reductoisomerase is derived from *Thermacetogenium phaeum,* and more preferably comprises an amino acid sequence shown in SEQ ID NO: 9 or an amino acid sequence having at least 75%, 80%, 85%, 90%, 91%, 92%, 93%, 94%, 95%, 96%, 97%, 98%, 99% or higher identity thereto and having NADH-dependent acetohydroxy acid reductoisomerase activity.

16. A method for producing D-pantoic acid, comprising culturing the genetically engineered pantoic acid-producing strain according to any one of claims 1-6 or a genetically engineered pantoic acid-producing strain obtained by the method according to any one of claims 7-15 under conditions suitable for fermentative production of D-pantoic acid, and optionally comprising isolating and purifying produced D-pantoic acid.

17. Use of the genetically engineered pantoic acid-producing strain according to any one of claims 1-6 or a genetically engineered pantoic acid-producing strain obtained by the method according to any one of claims 7-15 in production of D-pantoic acid.
